(19) Europäisches Patentamt

European Patent Office

Office européen des brevets

(11) **EP 1 950 303 A1**

(12) **EUROPÄISCHE PATENTANMELDUNG**

(43) Veröffentlichungstag:
 **30.07.2008 Patentblatt 2008/31**

(51) Int Cl.:
 *C12N 15/82* (2006.01)  *A01H 5/10* (2006.01)
 *C08B 30/00* (2006.01)  *A23L 1/00* (2006.01)

(21) Anmeldenummer: 07090009.7

(22) Anmeldetag: **26.01.2007**

(84) Benannte Vertragsstaaten:
 **AT BE BG CH CY CZ DE DK EE ES FI FR GB GR HU IE IS IT LI LT LU LV MC NL PL PT RO SE SI SK TR**
 Benannte Erstreckungsstaaten:
 **AL BA HR MK RS**

(71) Anmelder: **Bayer CropScience AG**
 **40789 Monheim (DE)**

(72) Erfinder:
 • **Frohberg, Claus**
  **14532 Kleinmachnow (DE)**
 • **Schmidt, Ralf-Christian**
  **14532 Stahnsdorf (DE)**

(74) Vertreter: **Beyer, Andreas et al**
 **Bayer BioScience GmbH**
 **Intellectual Property Department**
 **Hermannswerder 20a**
 **14473 Potsdam (DE)**

(54) **Genetisch modifizierte Pflanzen, die eine Stärke mit geringem Amylosegehalt und erhöhtem Quellvermögen synthetisieren**

(57) Die vorliegende Erfindung betrifft genetisch modifizierte monokotyle Pflanzenzellen und Pflanzen, deren Stärke einen apparenten Amylosegehalt von weniger als 5 Gew.-% sowie eine erhöhte Aktivität eines Proteins mit der Aktivität einer Stärkesynthase II und eine erhöhte Aktivität eines Proteins mit der Aktivität einer Glucan- Wasser-Dikinase aufweisen. Solche Pflanzen synthetisieren Stärke mit erhöhtem Heißwasser Quellvermögen. Verfahren zur Herstellung dieser Pflanzenzellen, Pflanzen, Stärken und Mehle sind ebenfalls Gegenstand der vorliegenden Erfindung.

**EP 1 950 303 A1**

**Beschreibung**

Die vorliegende Erfindung betrifft genetisch modifizierte monokotyle Pflanzenzellen und Pflanzen, deren Stärke einen apparenten Amylosegehalt von weniger als 5 Gew.-% sowie eine erhöhte Aktivität eines Proteins mit der Aktivität einer Stärkesynthase II und eine erhöhte Aktivität eines Proteins mit der Aktivität einer Glucan-Wasser-Dikinase aufweisen. Solche Pflanzen synthetisieren Stärke mit erhöhtem Heißwasser Quellvermögen. Verfahren zur Herstellung dieser Pflanzenzellen, Pflanzen, Stärken und Mehle sind ebenfalls Gegenstand der vorliegenden Erfindung.

Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in Höheren Pflanzen ist.

Weiterhin stellt Stärke einen ernährungsphysiologisch wesentlichen Bestandteil der menschlichen und tierischen Nahrung dar. Die strukturellen Merkmale der in Nahrungsmitteln enthaltenden Stärke können die funktionellen (z.B. Wasserbindungsvermögen, Quellvermögen), ernährungsphysiologischen (z.B. Verdaubarkeit, Einfluss des Nahrungsmittels auf den glykämischen Index) oder strukturgebenden (z.B. Schnittfestigkeit, Textur, Klebrigkeit, Verarbeitbarkeit) Eigenschaften von verschiedensten Nahrungsmitteln beeinflussen. Nahrungsmittelzusammensetzungen enthalten daher häufig eine Stärke mit bestimmten strukturellen Merkmalen, die die gewünschten Eigenschaften des betreffenden Nahrungsmittels bedingen. Auch die Eigenschaften von Nahrungsmitteln, die Stärke speichernde Pflanzengewebe (z.B. Körner, Früchte, Mehle) enthalten, können von der in den Pflanzengeweben enthaltenden Stärke beeinflusst werden.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades, des Auftretens von Verzweigungen der Glucoseketten und deren Kettenlängen unterscheiden, die darüber hinaus modifiziert, z.B. phosphoryliert sein können. Daher stellt Stärke keinen einheitlichen Rohstoff dar. Man unterscheidet insbesondere die Amylose vom Amylopektin. In typischen, für die industrielle Stärkeproduktion oder als Nahrungsmittel verwendeten Pflanzen, wie z.B. Mais, Reis, Weizen oder Kartoffel, besteht die synthetisierte Stärke zu ca. 20% - 25% aus Amylose und zu ca. 70% - 80% aus Amylopektin.

Die funktionellen, ernährungsphysiologischen oder strukturgebenden Eigenschaften der Stärke, wie z.B. die Löslichkeit, das Retrogradationsverhalten, das Wasserbindevermögen, die Filmbildungseigenschaften, die Viskosität, die Verkleisterungseigenschaften, die Gefrier-Tau-Stabilität, die Säurestabilität, die Gelfestigkeit, das Quellvermögen, die Verdaubarkeit, die Stärkekorngröße von Stärken werden u.a. durch die strukturellen Merkmale der Stärke wie das Amylose/Amylopektin-Verhältnis, das Molekulargewicht der Glucosepolymere, das Muster der Seitenkettenverteilung, den Gehalt an Ionen, den Lipid- und Proteingehalt und/oder die Stärkekornmorphologie etc. beeinflusst.

Durch auf Züchtung basierende Verfahren können ausgewählte strukturelle Merkmale der Stärke und damit auch funktionelle, ernährungsphysiologische oder strukturgebende Eigenschaften von Stärke in pflanzlichen Zellen verändert werden. Jedoch ist dieses heute nur für ausgewählte strukturelle Merkmale von Stärke (z.B. Amylopektin-/Amylosegehalt, US 5,300,145) möglich. Derzeit ist es z.B. nicht möglich, den Gehalt an Stärkephosphat in Pflanzen allein durch züchterische Maßnahmen zu beeinflussen.

Eine Alternative zu züchterischen Verfahren besteht in der gezielten Modifikation Stärke produzierender Pflanzen durch gentechnische Methoden. Voraussetzung hierfür ist jedoch die Identifizierung und Charakterisierung der an der Stärkesynthese und/oder Stärkemodifikation beteiligten Enzyme und deren anschließende funktionelle Analyse in transgenen Pflanzen.

An der Stärkesynthese in pflanzlichen Zellen sind verschiedene Enzyme, die unterschiedliche Reaktionen katalysieren, beteiligt. Stärkesynthasen (EC2.4.1.21, ADP-glucose:1,4-alpha-D-glucan 4-alpha-D-glucosyltransferase) katalysieren eine Polymerisierungsreaktion durch Übertragung eines Glucosylrestes von ADP-Glucose auf alpha-1,4-Glucane, wobei der übertragene Glucosylrest mit dem alpha-1,4-Glucan durch Erzeugung einer alpha-1,4-Bindung verknüpft wird. In allen bisher untersuchten Pflanzen konnten jeweils mehrere Isoformen von Stärkesynthasen nachgewiesen werden. Stärkesynthasen können in zwei unterschiedliche Gruppen eingeteilt werden: Stärkekorn gebundene Stärkesynthasen ("granule-bound starch synthases"; GBSS) und lösliche Stärkesynthasen ("soluble starch synthases"; im Zusammenhang mit der vorliegenden Erfindung auch als "SS" abgekürzt). Stärkekorn gebundene Stärkesynthasen katalysieren die Synthese von Amylose, wohingegen lösliche Stärkesynthasen an der Synthese von Amylopektin beteiligt sind (Ball und Morell, 2003, Annu. Rev, Plant Biol. 54, 207-233; Teltow et al., 2004, J. Expt. Bot. 55(406), 2131-2145). Die Gruppe der löslichen Stärkesynthasen weist mehrere Isoformen auf, die in der Fachliteratur als SSI, SSII, SSIII, SSIV und SSV bezeichnet werden. Die Zuordnung von Stärkesynthasen zu den einzelnen Isoformen (SSI, SSII, SSIII, SSIV, SSV) erfolgt anhand von Sequenzhomologien der betreffenden Proteinsequenzen der jeweiligen Enzyme (Ball und Morell, 2003, Annu. Rev, Plant Biol. 54, 207-233). Jeder einzelnen Isoform der löslichen Stärkesynthasen wird nach momentaner Lehrmeinung eine spezifische Funktion bei der Stärkesynthese zugewiesen. In dicotylen Pflanzen konnte bisher nur eine Isoform von SSII Proteinen nachgewiesen werden, während in manchen monokotylen Pflanzen (z.B. Mais) zwei unterschiedliche Klassen von SSII Proteinen nachgewiesen wurden, die mit SSIIa bzw. SSIIb bezeichnet werden. In monokotylen Pflanzen wird SSIIa präferentiell im Endosperm und SSIIb präferentiell in den Blattgewebe exprimiert (Teltow et al., 2004, J. Expt. Bot. 55(406): 2131-2145). Die spezifische Funktion insbesondere der einzelnen löslichen Stärkesynthasen bei der Synthese der Stärke ist zurzeit noch nicht abschließend geklärt (Ball und Morell, 2003, Annu. Rev, Plant Biol. 54: 207-233).

Die funktionellen, ernährungsphysiologischen oder strukturgebenden Eigenschaften von Stärke werden auch vom Phosphatgehalt, einer nicht-Kohlenstoffkomponente, beeinflusst. Dabei ist zwischen Phosphat, welches in Form von Monoestern kovalent an die Glucosemoleküle der Stärke gebunden ist (im Zusammenhang mit der vorliegenden Erfindung als Stärkephosphat bezeichnet) und Phosphat in Form von mit der Stärke assoziierten Phospholipiden zu unterscheiden.

Der Gehalt an Stärkephosphat variiert je nach Pflanzensorte. So synthetisieren z.B. bestimmte Maismutanten eine Stärke mit erhöhtem Gehalt an Stärkephosphat (waxy-Mais 0,002% und Hoch-Amylose-Mais 0,013%), während herkömmliche Maissorten nur Spuren von Stärkephosphat aufweisen. Ebenfalls geringe Mengen an Stärkephosphat findet man in Weizen (0,001 %) während in Hafer und *Sorghum* kein Stärkephosphat nachgewiesen werden konnte. In waxy Reis-Mutanten wurde weniger Stärkephosphat (0,003%) gefunden als in herkömmlichen Reissorten (0,013%). Signifikante Mengen von Stärkephosphat wurden in Knollen- oder Wurzelspeicherstärke synthetisierenden Pflanzen wie z.B. Tapioca (0,008%), Süßkartoffel (0,011 %), Pfeilwurz (0,021%) oder Kartoffel (0,089%) nachgewiesen. Die im vorangegangenen zitierten prozentualen Werte für den Stärkephosphatgehalt beziehen sich jeweils auf das Trockengewicht der Stärke und sind von Jane et al. (1996, Cereal Foods World 41 (11): 827-832) ermittelt worden.

Stärkephosphat kann in Form von Monoestern an der C2, C3 oder C6 Position der polymerisierten Glucosemonomere vorliegen (Takeda und Hizukuri, 1971, Starch/Stärke 23: 267-272). Die Phosphatverteilung des Phosphates in von Pflanzen synthetisierter Stärke zeichnet sich im Allgemeinen dadurch aus, dass etwa 30% bis 40% der Phosphatreste in C3-Position und etwa 60% bis 70% der Phosphatreste in C6-Position der Glucosemoleküle kovalent gebunden sind (Blennow et al., 2000, Int. J. of Biological Macromolecules 27: 211-218). Blennow et al. (2000, Carbohydrate Polymers 41: 163-174) ermittelten einen Gehalt an Stärkephosphat, der in C6-Position der Glukosemoleküle gebunden ist, für verschiedene Stärken, wie z.B. Kartoffelstärke (zwischen 7,8 und 33,5 nmol pro mg Stärke, je nach Sorte), Stärke aus verschiedenen *Curcuma* Spezies (zwischen 1,8 und 63 nmol pro mg Stärke), Tapiocastärke (2,5 nmol pro mg Stärke), Reisstärke (1,0 nmol pro mg Stärke), Mungbohnenstärke (3,5 nmol pro mg Stärke) und Sorghumstärke (0,9 nmol pro mg Stärke). In Gerstenstärke und Stärke aus verschiedenen waxy-Mutanten von Mais konnten diese Autoren kein an der C6-Position gebundenes Stärkephosphat nachweisen. Bisher konnte kein Zusammenhang zwischen dem Genotyp einer Pflanze und dem Gehalt von Stärkephosphat hergestellt werden (Jane et al., 1996, Cereal Foods World 41 (11): 827-832).

Bisher sind zwei Proteine beschrieben, welche die Einführung von kovalenten Bindungen von Phosphatresten an die Glucosemoleküle der Stärke vermitteln. Das erste Protein besitzt die enzymatische Aktivität einer alpha-Glucan-Wasser-Dikinase (GWD, E.C.: 2.7.9.4) (Ritte et al., 2002, PNAS 99: 7166-7171), wird insbesondere in der älteren wissenschaftlichen Literatur häufig als R1 bezeichnet und ist an die Stärkekörner der Speicherstärke in Kartoffelknollen gebunden (Lorberth et al., 1998, Nature Biotechnology 16: 473-477). Das zweite, in der Literatur beschriebene Protein, das die Einführung von Stärkephosphat in Stärke katalysiert, besitzt die enzymatische Aktivität einer Phospho-Glucan-Wasser-Dikinase (PWD, E.C.: 2.7.9.5) (Kötting et al., 2005, Plant Physiol. 137: 2424-252, Baunsgaard et al., 2005, Plant Journal 41: 595-605).

Ein wesentlicher Unterschied zwischen GWD und PWD besteht darin, dass GWD nicht-phosphorylierte Stärke als Substrat verwenden kann, d.h. eine *de novo* Phosphorylierung von nicht-phosphorylierter Stärke durch GWD katalysiert werden kann, während PWD bereits phosphorylierte Stärke als Substrat benötigt, d.h. zusätzlich Phosphat in bereits phosphorylierte Stärke einführt (Kötting et al., 2005, Plant Physiol. 137: 2424-252, Baunsgaard et al., 2005, Plant Journal 41: 595-605). Ein weiterer wesentlicher Unterschied zwischen GWD und PWD besteht darin, dass GWD Phosphatgruppen ausschließlich in C6-Position der Glucosemoleküle von Stärke einführt, während PWD ausschließlich die C3-Position der Glucosemoleküle von Stärke phosphoryliert (Ritte et al., 2006, FEBS Letters 580: 4872-4876).

In der von GWD bzw. PWD katalysierten Reaktion werden die Edukte alpha-1,4-Glucan (für GWD) bzw. phosphoryliertes alpha-1,4-Glucan (für PWD), Adenosintriphosphat (ATP) und Wasser zu den Produkten Glucan-Phosphat (Stärkephosphat), unorganisches Phosphat und Adenosinmonophosphat umgesetzt (Kötting et al., 2005, Plant Physiol. 137: 2424-252; Ritte et al., 2002, PNAS 99: 7166-7171).

Weizenpflanzen, welche durch Expression eines GWD kodierenden Gens aus Kartoffel eine erhöhte Aktivität von GWD Proteinen aufweisen, sind in WO 02/34923 beschrieben. Diese Pflanzen synthetisieren im Vergleich zu entsprechenden Wildtyp-Pflanzen, in welchen kein Stärkephosphat detektiert werden konnte, eine Stärke mit signifikanten Mengen an Stärkephosphat in der C6-Position der Glucosemoleküle.

WO 05/002359 beschreibt die Expression einer bezüglich von Maispflanzen verwendeten Codon optimierten GWD aus Kartoffel in Maispflanzen. Mittels $^{31}$P NMR wurde ein Gesamtsphosphatgehalt (gebunden in C6-, C3- und C2-Position der Glucosemoleküle) der betreffenden Maisstärke von 0,0736% Phosphat bezogen auf die Menge Glucose ermittelt. Legt man für Phosphat ($H_3PO_4$) ein Molekulargewicht von 98 zu Grunde, so ergibt sich für den in WO 05/002359 ermittelten Gesamtphosphatgehalt von 0,0736% für Stärke, isoliert aus transgenen Maispflanzen, ein Gesamtphosphatgehalt von ca. 7,5 nmol Phosphat pro mg Stärke. Pflanzen, welche durch Expression eines PWD kodierenden Gens aus *Arabidopsis thaliana* eine erhöhte Aktivität eines PWD Proteins aufweisen, sind in WO 05/095617 beschrieben. Diese Pflanzen weisen im Vergleich zu entsprechenden nicht transformierten Wildtyp-Pflanzen einen erhöhten Gehalt an Stärkephosphat auf.

Eine wichtige funktionelle Eigenschaft, z.B. bei der Verarbeitung von Stärken in der Nahrungsmittelindustrie, stellt das Quellvermögen dar. Verschiedene strukturelle Eigenschaften von Stärken, wie das Amylose-/Amylopektinverhältnis, die Seitenkettenlänge, die Molekulargewichtsverteilung der Stärkepolymere, die Anzahl der Verzweigungen als auch die Menge an Stärkephosphat haben einen Einfluss auf funktionelle Eigenschaften, insbesondere auf das Quellvermögen der betreffenden Stärken (Narayana und Moorthy, 2002, Starch/Stärke 54: 559-592).

Amylose wurde lange als lineares Polymer, bestehend aus $\alpha$-1,4-glycosidisch verknüpften $\alpha$-D-Glukosemonomeren, angesehen. In neueren Studien wurde jedoch die Anwesenheit von $\alpha$-1,6-glycosidischen Verzweigungspunkten (ca. 0,1%) nachgewiesen (Hizukuri und Takagi, 1984, Carbohydr. Res. 134: 1-10; Takeda et al., 1984, Carbohydr. Res. 132: 83-92).

Amylopektin stellt ein komplexes Gemisch aus unterschiedlich verzweigten Glukoseketten dar. Im Gegensatz zur Amylose ist das Amylopektin stärker verzweigt. Mit der aus $\alpha$-1,4-glycosidisch verknüpften $\alpha$-D-Glukosemonomeren bestehenden Hauptkette sind Seitenketten über $\alpha$-1,6-glykosidische Bindungen verknüpft. Nach Lehrbuchangaben (Voet and Voet, 1990. Biochemistry, John Wiley & Sons) treten die $\alpha$-1,6-Verzweigungen durchschnittlich alle 24 bis 30 Glukosereste auf. Dies entspricht einem Verzweigungsgrad von ca. 3% - 4%. Die Angaben zum Verzweigungsgrad sind variabel und abhängig von der Herkunft (z.B. Pflanzenspezies, Pflanzensorte usw.) der jeweiligen Stärke. In typischen für die industrielle Stärkeproduktion verwendeten Pflanzen, wie z.B. Mais, Weizen oder Kartoffel, besteht die synthetisierte Stärke zu ca. 20% - 30% aus Amylose-Stärke und zu ca. 70% - 80% aus Amylopektin-Stärke.

Ein weiterer wesentlicher Unterschied zwischen Amylose und Amylopektin liegt im Molekulargewicht. Während Amylose, je nach Herkunft der Stärke, ein Molekulargewicht von $5\times10^5$ - $10^6$ Da besitzt, liegt das Molekulargewicht des Amylopektins zwischen $10^7$ und $10^8$ Da. Die beiden Makromoleküle lassen sich durch ihr Molekulargewicht und ihre unterschiedlichen physikalisch-chemischen Eigenschaften differenzieren, was am einfachsten durch ihre unterschiedlichen Jodbindungseigenschaften sichtbar gemacht werden kann.

In vielen technischen Anwendungen wird nur Amylopektin benötigt, da Amylopektin verdickend wirkt. Amylose wirkt gelierend und ist daher für zahlreiche Anwendungen eher unerwünscht. Reine Amylopektin-Stärke ermöglicht eine sehr einheitliche Oberflächenstruktur und gleichzeitig eine hohe Viskosität, Stabilität, und Transparenz. Anwendungsmöglichkeiten dieser Stärken gibt es in der Papier-, Klebstoff-, Textil-, Bau- und Kosmetikindustrie. Weiterhin ist Amylopektin-Stärke das bevorzugte Ausgangsmaterial zur Herstellung von Maltodextrinen aufgrund deren erhöhter Wasserlöslichkeit, Löslichkeitsstabilität und Klarheit gegenüber Maltodextrinen, die aus amylosehaltigen Stärken hergestellt werden.

In der Lebensmittelindustrie werden Amylopektin-Stärken häufig als Stabilisatoren, Bindemittel und zur Texturverbesserung eingesetzt. Insbesondere sind Amylopektinstärken bei solchen Verarbeitungsprozessen von Vorteil, bei denen große Temperaturschwankungen bei Prozessierung und Fertigung auftreten (z.B. Gefrier-Tau-Stabilität). Der Einsatz von Amylopektinstärken in der Lebensmittelindustrie wächst insbesondere vor dem Hintergrund des steigenden Bedarfs an (Halb-) Fertigprodukten.

Die GBSSI ("Granule-Bound Starch Synthase I") ist an der Bildung von Amylose beteiligt. Bisher sind Pflanzen beschrieben, bei welchen die Aktivität der Stärkekorn-gebundenen Stärkesynthase GBSSI reduziert ist (Shure et al., 1983, Cell 35: 225-233; Hovenkamp-Hermelink et al., 1987, Theoretical and Applied Genetics 75: 217-221; Visser et al., 1991, Mol. Gen. Genet. 225: 289-296; Hergersberg, 1988, Dissertation, Universität Köln; WO 92/11376). Weiterhin sind Mutanten bekannt, denen ein funktionales GBSSI-Gen fehlt und welche daher eine Amylose-freie (= Amylopektin-) Stärke synthetisieren (Kossmann und Lloyd 2000, Critical Reviews in Plant Sciences, 19(3): 171-226). Das Endosperm einer entsprechenden GBSSI-Mutante aus Mais weist ein wachsartiges Erscheinungsbild auf, worauf die Einführung der Bezeichnung "waxy"-Endosperm als Synonym für Amylose-freie Stärken zurückzuführen ist.

Bei der Beschreibung des Quellvermögens von Stärke ist zwischen Quellvermögen in kaltem Wasser (z.B. Raumtemperatur) und Quellvermögen in warmem bzw. heißem Wasser zu unterscheiden. Native Stärken weisen, wenn überhaupt, in kaltem Wasser ein vernachlässigbares Quellvermögen auf, während physikalisch modifizierte (vorverkleisterte, getrocknete) Stärken bereits in kaltem Wasser quellen können. Herstellungsverfahren für in kaltem Wasser quellende Stärken sind z.B. beschrieben in US 4,280,851. Im Zusammenhang mit der vorliegenden Erfindung bezieht sich der Begriff "Quellvermögen" auf das Verhalten von Stärke in warmen/heißen wässrigen Suspensionen. Das Quellvermögen wird standardmäßig ermittelt, indem Stärkekörner in Anwesenheit eines Wasserüberschusses erwärmt, nicht gebundenes Wasser nach Zentrifugation der Suspension entfernt und der Quotient aus dem Gewicht des erhaltenen Rückstandes und dem der eingewogenen Menge an Stärke gebildet wird. Bei der Durchführung dieses Verfahrens werden bei Erwärmung der Stärkesuspension kristalline Bereiche der Stärkekörner aufgelöst und Wassermoleküle in die Stärkekörner eingelagert, jedoch ohne dass dabei die Struktur des Stärkekorns selbst aufgelöst wird, d.h. es findet lediglich eine Quellung der einzelnen Stärkekörner statt.

Im Vergleich zu Cerealienstärken weisen Stärken, die aus Knollen oder knollenartigen Geweben isoliert wurden, ein wesentlich höheres Heißwasser Quellvermögen auf.

Für Kartoffelstärken, isoliert aus verschiedenen Varietäten, wurde nach der Methode von Leach et al. (1959, Cereal Chemistry 36: 534-544) bei 85°C ein maximales Quellvermögen von 74,15 g/g (Varietät Kufri Jyoti) ermittelt (Singh et al., 2002, Journal of the Science of Food and Agriculture 82: 1376-1383). Takizawa et al. (2004, Brazilian Archives of Biology and Technology 47(6): 921-931) ermittelten für Kartoffelstärke ein Quellvermögen von 100 g/g (90°C, nach der Methode von Leach et al., supra). Weizenstärke, isoliert aus verschiedenen Kultivaren, weist ein Quellvermögen von 16,6 g/g bis 26,0 g/g (Temperatur: kochende wässrige 0,1% $AgNO_3$ Suspension) auf (Yamamori und Quynh, 2000, Theor Appl Genet 100: 23-38). Stärke, isoliert aus verschiedenen Kultivaren spelzenfreier (hull-less) Gerste, weist ein Quellvermögen von 16,5 g/g bzw. 19,3 g/g und waxy bzw. Amylose freie Stärke verschiedener Kultivare besagter Gerste weist ein Quellvermögen von 36,0 g/g bis 55,7 g/g auf (Temperatur: 70°C wässrige 0,1% $AgNO_3$, Yasui et al., 2002, Starch/Stärke 54: 179-184). Für Maisstärke wurde ein Quellvermögen von 22,3 g/g und für Hoch-Amylose Maisstärken ein Quellvermögen von 9,6 g/g (Hylon V), 6,1 g/g (Hylon VII) bzw. 3,9 g/g (LAPS = Low AmyloPectin Starch) ermittelt (90°C, Shi et al., 1998, J. Cereal Sci. 27: 289-299). In US 6,299,907 wurde für waxy Maisstärke ein Quellvermögen von 35,4 g/g angegeben. Für Stärke, isoliert aus verschiedenen Reiskultivaren, wurde nach der Methode von Leach et al. (supra) ein Quellvermögen von 26,0 g/g bis 33,2 g/g ermittelt (Sodhi und Singh, 2003, Food Chemistry 80: 99-108). Chen et al. (2003, Starch/Stärke 55: 203-212) ermittelten für verschiedene Mischungen von waxy Reisstärken mit Hoch-Amylose Reisstärken ein Quellvermögen von etwa 25 g/g bis etwa 49 g/g (95°C, wässrige Suspension). Yasui et al. (2002, Starch/Stärke 54: 179-184) ermittelten für eine Amylose freie Reisstärke ein Quellvermögen von 55,7 g/g (gemessen in kochendem Wasser in 0,1% wässriger Silbernitrat Lösung). Durch die Herstellung von Derivaten nativer Stärken können funktionelle Eigenschaften der Stärken verändert werden. Kreuzvernetzte ("cross-linked") Weizenstärken weisen je nach Grad der Kreuzvernetzung ein Quellvermögen von 6,8 g/g bis 8,9 g/g, acetylierte Weizenstärken ein Quellvermögen von maximal 10,3 g/g und gleichzeitig kreuzvernetzte und acetylierte Weizenstärken ein Quellvermögen von 9,4 g/g auf, während die entsprechenden nicht derivatisierten Stärken ein Quellvermögen von 8,8 g/g aufwiesen (gemessen bei 90°C; Van Hung und Morita, 2005, Starch/Stärke 57: 413-420).

Für acetylierte Waxy-Reisstärken wurde ein Quellvermögen von ca. 30 g/g und für kreuzvernetzte Waxy-Reisstärke ein Quellvermögen von ca. 15 g/g ermittelt, während entsprechende nicht-derivatisierte Waxy-Reisstärke ein Quellvermögen von ca. 41 g/g aufwies. Acetylierte Reisstärke wies ein Quellvermögen von ca. 20 g/g und kreuzvernetzte Reisstärke ein Quellvermögen von ca. 13 g/g auf, während entsprechende nicht derivatisierte Reisstärke ein Quellvermögen von ca. 14 g/g aufwies (gemessen bei 90°C, Liu et al., 1999, Starch/Stärke 52: 249-252). US 6,299,907 beschreibt kreuzvernetzte Stärken, wobei die Kreuzvernetzungsreaktion nach Vorquellung der betreffenden Stärken in einer Natriumhydroxid/Sulfat Lösung durchgeführt wurde. Je nach Grad der Kreuzvernetzung wurde für Weizenstärke ein Quellvermögen von 6,8 g/g bis 7,3 g/g (entsprechende nicht derivatisierte Weizenstärke 14,7 g/g), für Hydroxypropylweizenstärke ein Quellvermögen von 9,7 g/g (entsprechende nicht derivatisierte Weizenstärke 22,9 g/g), für kreuzvernetzte Maisstärke ein Quellvermögen von 5,9 g/g (entsprechende nicht derivatisierte Maisstärke 16,7 g/g), für kreuzvernetzte Waxy-Maisstärke ein Quellvermögen von 8,3 g/g (entsprechende nicht derivatisierte Waxy-Maisstärke 35,4 g/g) und für kreuzvernetzte Kartoffelstärke ein Quellvermögen von 6,7 g/g (entsprechende nicht derivatisierte Kartoffelstärke wurde nicht genau spezifiziert) ermittelt (gemessen bei 95°C). Daraus ergibt sich, dass das Quellvermögen von Stärke durch heutzutage gängige Methoden der Derivatisierung, wenn überhaupt, nicht wesentlich gesteigert werden kann.

Der vorliegenden Erfindung liegt die Aufgabe zu Grunde, modifizierte waxy Stärken mit veränderten funktionellen Eigenschaften sowie neue Pflanzenzellen und Pflanzen, die eine waxy Stärke mit veränderten funktionellen Eigenschaften synthetisieren, als auch Verfahren und Mittel zur Erzeugung besagter Pflanzen und/oder Pflanzenzellen zur Verfügung zu stellen. Insbesondere bestehen die veränderten funktionellen Eigenschaften darin, dass die modifizierten Stärken ein erhöhtes Heisswasser Quellvermögen aufweisen.

Somit betrifft die vorliegende Erfindung genetisch modifizierte monokotyle Pflanzenzellen oder genetisch modifizierte monokotyle Pflanzen, deren Stärke einen apparenten Amylosegehalt von weniger als 5 Gew.-% aufweist und welche zusätzlich eine erhöhte Aktivität eines Proteins mit der enzymatischen Aktivität einer Stärkesynthase II und zusätzlich eine erhöhte Aktivität eines Proteins mit der enzymatischen Aktivität einer Glucan-Wasser-Dikinase aufweisen, im Vergleich zu entsprechenden genetisch nicht modifizierten Wildtyp-Pflanzenzellen bzw. zu entsprechenden genetisch nicht modifizierten Wildtyp-Pflanzen.

Die genetische Modifikation kann dabei jede genetische Modifikation sein, die zur Synthese einer Stärke von weniger als 5 Gew.-% Amylose und gleichzeitig zu einer Erhöhung der Aktivität mindestens eines Proteins mit der Aktivität einer Stärkesynthase II und (gleichzeitig) mindestens eines Proteins mit der Aktivität einer Glucan-Wasser-Dikinase in genetisch modifizierten Pflanzenzellen oder genetisch modifizierten Pflanzen führt, im Vergleich zu entsprechenden nicht genetisch modifizierten Wildtyp-Pflanzenzellen oder Wildtyp-Pflanzen.

Der Begriff "Wildtyp-Pflanzenzelle" bedeutet im Zusammenhang mit der vorliegenden Erfindung, dass es sich um Pflanzenzellen handelt, die als Ausgangsmaterial für die Herstellung der erfindungsgemäßen Pflanzenzellen dienen, d.h. deren genetische Information, abgesehen von der eingeführten genetischen Modifikation, der einer erfindungsgemäßen Pflanzenzelle entspricht.

Der Begriff "Wildtyp-Pflanze" bedeutet im Zusammenhang mit der vorliegenden Erfindung, dass es sich um Pflanzen handelt, die als Ausgangsmaterial für die Herstellung der erfindungsgemäßen Pflanzen dienten, d.h. deren genetische Information, abgesehen von der eingeführten genetischen Modifikation, der einer erfindungsgemäßen Pflanze entspricht.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten wurden. Im Zusammenhang mit der vorliegenden Erfindung bedeutet der Begriff "entsprechend" im Zusammenhang mit Wildtyp-Pflanzenzelle oder Wildtyp-Pflanze, dass die Pflanzenzellen oder Pflanzen, die miteinander verglichen werden, unter gleichen Kulturbedingungen aufgezogen wurden und dass sie ein gleiches (Kultur-) Alter aufweisen.

Der Begriff "monokotyle Pflanzen" bezeichnet die Einkeimblättrigen Pflanzen. Sie gehören botanisch zu einer der drei Klassen der Bedecktsamer (Magnoliophyta). Monokotyle Pflanzen zeichnen sich im Gegensatz zu Dikotylen dadurch aus, dass im Embryo typischerweise nur ein Keimblatt angelegt wird (griech: *monos* = "einzig" und *kotyledon* = "Keimblatt"). Weiterhin haben sie geschlossene Leitbündel, d.h., Phloem und Xylem sind nicht durch ein Meristem getrennt, daher ist kein sekundäres Dickenwachstum möglich. Zu dieser Pflanzenklasse gehören u.a. die Gräser mit den Ordnungen Cyperales und Poales und zahlreiche andere Familien.

Der Begriff "erhöhte Aktivität mindestens eines Proteins mit der (enzymatischen) Aktivität einer Stärkesynthase II" bedeutet dabei im Rahmen der vorliegenden Erfindung eine Erhöhung der Expression endogener Gene, die Proteine mit der Aktivität einer Stärkesynthase 11 kodieren und/oder eine Erhöhung der Menge an Proteinen mit der Aktivität einer Stärkesynthase 11 in den Zellen und/oder eine Erhöhung der Aktivität von Proteinen mit der Aktivität einer Stärkesynthase 11 in den Zellen.

Der Begriff "erhöhte Aktivität eines Proteins mit der (enzymatischen) Aktivität einer Glucan-Wasser-Dikinase" bedeutet dabei im Rahmen der vorliegenden Erfindung eine Erhöhung der Expression endogener Gene, die Proteine mit der Aktivität einer Glucan-Wasser-Dikinase kodieren und/oder eine Erhöhung der Menge an Proteinen mit der Aktivität einer Glucan-Wasser-Dikinase in den Zellen und/oder eine Erhöhung der Aktivität von Proteinen mit der Aktivität einer Glucan-Wasser-Dikinase in den Zellen.

Die Erhöhung der Expression kann beispielsweise bestimmt werden durch Messung der Menge an Transkripten, die Proteine mit der Aktivität einer Stärkesynthase II oder Proteine mit der Aktivität einer Glucan-Wasser-Dikinase kodieren. Dieses kann z.B. durch Northern-Blot-Analyse oder Q-PCR (Quantitative Transcription Polymerase Chain Reaction) erfolgen.

Eine Erhöhung der Menge eines Proteins mit der Aktivität einer Glucan-Wasser-Dikinase bedeutet dabei vorzugsweise eine Erhöhung der Menge an betreffendem Protein im Vergleich zu entsprechenden nicht genetisch modifizierten Zellen um mindestens 50%, insbesondere um mindestens 70%, bevorzugt um mindestens 85% und besonders bevorzugt um mindestens 100%.

Eine Erhöhung der Menge an Protein mit der Aktivität einer Glucan-Wasser-Dikinase bedeutet auch, dass Pflanzen oder Pflanzenzellen, die keine nachweisbaren Mengen an Proteinen mit der Aktivität einer Glucan-Wasser-Dikinase aufweisen, nach erfindungsgemäßer genetischer Modifikation eine nachweisbare Menge an Protein mit der Aktivität einer Glucan-Wasser-Dikinase aufweisen.

Methoden zur Herstellung von Antikörpern, die spezifisch mit einem bestimmten Protein reagieren, d.h. die spezifisch an besagtes Protein binden, sind dem Fachmann bekannt (siehe z.B. Lottspeich und Zorbas (Eds.), 1998, Bioanalytik, Spektrum akad, Verlag, Heidelberg, Berlin, ISBN 3-8274-0041-4). Die Herstellung solcher Antikörper wird von einigen Firmen (z.B. Eurogentec, Belgien) als Auftragsservice angeboten. Antikörper, mit welchem eine Erhöhung der Menge an Protein mit der Aktivität einer Glucan-Wasser-Dikinase mittels immunologischer Methoden festgestellt werden kann, sind bei Lorberth et al. (1998, Nature Biotechnology 16: 473-477) und Ritte et al. (2000, Plant Journal 21: 387-391) beschrieben. Antikörper, mit welchem eine Erhöhung der Menge an Protein mit der Aktivität einer Stärkesynthase II mittels immunologischer Methoden festgestellt werden kann, sind bei Walter ("Untersuchungen der Expression und Funktion der Stärkesynthase II (SSII) aus Weizen (Triticum aestivum)", Dissertation am Fachbereich Biologie der Universität Hamburg, ISBN 3-8265-8212-8) beschrieben. Die Menge der Aktivität eines Proteins mit der Aktivität einer Glucan-Wasser-Dikinase kann z.B. nachgewiesen werden wie in der Literatur beschrieben (Mikkelsen et al., 2004, Biochemical Journal 377: 525-532; Ritte et al., 2002, PNAS 99: 7166-7171).

Die Menge der Aktivität eines Proteins mit der Aktivität einer Stärkesynthase II kann z.B. wie in der Literatur beschrieben (Nishi et al., 2001, Plant Physiology 127: 459-472) bestimmt werden. Eine bevorzugte Methode zur Bestimmung der Menge an Aktivität eines Proteins mit der Aktivität einer Stärkesynthase II ist unter "Allgemeine Methoden" beschrieben.

Vorzugsweise weisen erfindungsgemäße Pflanzenzellen oder erfindungsgemäße Pflanzen eine Aktivität eines Proteins mit der Aktivität einer Stärkesynthase II auf, die mindestens 2 fach, bevorzugt mindestens 6 fach erhöht ist, im Vergleich zu entsprechenden genetisch nicht modifizierten Wildtyp-Pflanzenzellen bzw. Wildtyp-Pflanzen.

Proteine mit der Aktivität einer Stärkesynthase II (ADP-Glukose-1,4-alpha-D-glucan-4-alpha-D-glucosyltransferase; EC 2.4.1.21) weisen in ihrem Aufbau eine Abfolge bestimmter Domänen auf. Am N-Terminus weisen sie ein Signalpeptid für den Transport in Plastiden auf. In Richtung vom N-Terminus nach C-Terminus folgen eine N-terminale Region und eine katalytische Domäne. (Li et al., 2003, Funct Integr Genomics 3, 76-85). Weitere Analysen, basierend auf Aminosäure Sequenzvergleichen (http://hits.isb-sib.ch/cgi-bin/PFSCAN) verschiedener Proteine mit der Aktivität einer Stärkesynthase II ergaben, dass diese Proteine drei spezifische Domänen aufweisen. In der unter SEQ ID NO 4 dargestellten Aminosäuresequenz stellen die Aminosäuren 322 bis 351 Domäne 1, die Aminosäuren 423 bis 462 Domäne 2 und die Aminosäuren 641 bis 705 Domäne 3 dar. Domäne 1 wird von den Nukleotiden 1190 bis 1279, Domäne 2 von den Nukleotiden 1493 bis 1612 und Domäne 3 von den Nukleotiden 2147 bis 2350 der unter SEQ ID NO 3 dargestellten Nukleinsäuresequenz codiert.

Im Zusammenhang mit der vorliegenden Erfindung soll unter dem Begriff "Protein mit der Aktivität einer Stärkesynthase II" ein Protein verstanden werden, dass eine Glucosylierungsreaktion katalysiert, bei der Glucosereste des Substrates ADP-Glucose unter Bildung einer alpha-1,4-Verknüpfung auf alpha-1,4-verknüpfte Glucanketten übertragen werden (ADP-Glukose + {(1,4)- alpha-D-glucosyl}(N) <=> ADP + {(1,4)- alpha-D-glucosyl}(N+1)), wobei die Aminosäuresequenz des Proteins mit der Aktivität eines Proteins einer Stärkesynthase II mit den Aminosäuren 322 bis 351 (Domäne 1) der unter SEQ ID NO 4 dargestellten Aminosäuresequenz eine Identität von mindestens 86%, bevorzugt mindestens 93%, besonders bevorzugt mindestens 95%, insbesondere bevorzugt von mindestens 98% und mit den Aminosäuren 423 bis 462 (Domäne 2) der unter SEQ ID NO 4 dargestellten Aminosäuresequenz eine Identität von mindestens 83%, bevorzugt mindestens 86%, besonders bevorzugt mindestens 95%, insbesondere bevorzugt von mindestens 98% und mit den Aminosäuren 641 bis 705 (Domäne 3) der unter SEQ ID NO 4 dargestellten Aminosäuresequenz eine Identität von mindestens 70%, vorzugsweise mindestens 82%, bevorzugt 86%, besonders bevorzugt 95%, insbesondere bevorzugt von mindestens 98% aufweist. Nukleinsäuresequenzen und die dazu korrespondierenden Aminosäuresequenzen, die eine besagte Identität mit den Domänen 1, 2 und 3 aufweisen und ein Protein mit der Aktivität einer Stärkesynthase II kodieren, sind dem Fachmann bekannt und z.B. veröffentlicht unter Accession No AY133249 (Hordeum vulgare), Accession No AY133248 (Aegilops tauschii), Accession Nos XP467757, AAK64284 (Oryza sativa), Accession No AAK81729 (Oryza sativa) Accession Nos AAD13341, AAS77569, No AAD13342 (Zea mays), Accession No AAF13168 (Manihut esculenta), Accession No BAD18846 (Phaseolus vulgaris), Accession No CAA61241 (Solanum tuberosum), Accession No CAA61269 (Pisum sativum), Accession No AAC19119 (Ipomea batatas), Accession No AAF 26156 (Arabidopsis thaliana), Accession No AAP41030 (Colocasia esculenta), Accession No AAS88880 (Ostraeococcus tauri), oder Accession No AAC17970 (Chlamydomonas reinhardii). Die genannten Nukleinsäuresequenzen und Aminosäuresequenzen kodierend ein Protein mit der Aktivität einer Stärkesynthase II sind zugänglich über NCBI (http://www.ncbi.nlm.nih.gov/entrez/) und sind durch Nennung der Referenzen ausdrücklich in die Beschreibung der vorliegenden Anmeldung aufgenommen.

Im Rahmen der vorliegenden Erfindung soll unter dem Begriff "Protein mit der Aktivität einer Glucan-Wasser-Dikinase" ein Protein verstanden werden, welches einen beta-Phosphatrest von ATP auf Stärke überträgt. Stärken, isoliert aus Blättern einer Arabidopsis thaliana sex1-3 Mutante weisen keine nachweisbaren Mengen an kovalent gebundenen Phosphatresten auf, werden jedoch von einem Protein mit der Aktivität einer Glucan-Wasser-Dikinase in vitro phosphoryliert. D.h. nicht-phosphorylierte-Stärke, z.B. isoliert aus Blättern einer Arabidopsis thaliana sex1-3 Mutante, wird in einer durch ein Protein mit der Aktivität einer Glucan-Wasser-Dikinase katalysierten Phosphorylierungsreaktion als Substrat verwendet.

Von einem Protein mit der Aktivität einer Glucan-Wasser-Dikinase wird der beta-Phosphatrest des ATP auf die Stärke in C6-Position der Glucose und der gamma-Phosphatrest des ATP auf Wasser übertragen. Als weiteres Reaktionsprodukt entsteht AMP (Adenosinmonophosphat). Ein Protein mit der Aktivität einer Glucan-Wasser-Dikinase wird daher auch als [alpha-1,4-Glucan]-Wasser-Dikinase bzw. als Stärke-Wasser-Dikinase bezeichnet (E.C.: 2.7.9.4; Ritte et al., 2002, PNAS 99: 7166-7171).

Bei der durch ein Protein mit der Aktivität einer Glucan-Wasser-Dikinase katalysierten Phosphorylierung von Stärke entstehen zusätzliche Phosphatmonoesterbindungen ausschließlich in C6-Position der Glucosemoleküle (Ritte et al., 2006, FEBS Letters 580: 4872-4876). Bei der Katalyse der Phosphorylierungsreaktion einer Stärke durch ein Protein mit der Aktivität einer Glucan-Wasser-Dikinase entsteht als Zwischenprodukt ein phosphoryliertes Protein, bei welchem der beta-Phosphatrest des ATP kovalent an eine Aminosäure des Proteins mit der Aktivität einer Glucan-Wasser-Dikinase gebunden ist (Ritte et al., 2002, PNAS 99, 7166-7171). Das Zwischenprodukt entsteht durch Autophosphorylierung des Proteins mit der Aktivität einer Glucan-Wasser-Dikinase, d.h. das Protein mit der Aktivität einer Glucan-Wasser-Dikinase selbst katalysiert die Reaktion, die zu dem Zwischenprodukt führt. Aminosäuresequenzen, die Proteine mit der Aktivität einer Glucan-Wasser-Dikinase kodieren, enthalten eine Phosphohistidin-Domäne. Phosphohistidin-Domänen sind z.B. beschrieben bei Tien-Shin Yu et al. (2001, Plant Cell 13, 1907-1918). Bei der Autophosphorylierung eines Proteins mit der Aktivität einer Glucan-Wasser-Dikinase wird ein Histidinrest in der Phosphohistidin-Domäne der Aminosäuresequenz, kodierend ein Protein mit der Aktivität einer Glucan-Wasser-Dikinase, phosphoryliert (Mikkelsen et al., 2004, Biochemical Journal 377: 525-532). In der beispielsweise unter SEQ ID NO 2 dargestellten Proteinsequenz eines Proteins mit der Aktivität einer Glucan-Wasser-Dikinase aus *Solanum tuberosum* stellen die Aminosäuren 1064 bis 1075 die Phosphohistidin-Domäne dar. Wird der konservierte Histidinrest (in der beispielsweise unter SEQ ID NO 2 dargestellten Proteinsequenz Aminosäure 1069) der Phosphohistidin-Domäne durch eine andere Aminosäure ersetzt, findet keine Autophosphorylierung und damit auch keine Phosphorylierung von Glucanen durch das mutagenisierte Protein mehr statt (Mikkelsen et al., 2004, Biochemical Journal 377: 525-532). Weiterhin zeichnet sich ein Protein mit der Aktivität einer Glucan-Wasser-Dikinase dadurch aus, dass es eine C-terminale Nukleotidbindedomäne aufweist, die in der beispielsweise unter SEQ ID NO 2 dargestellten Aminosäuresequenz von den Aminosäuren 1121 bis 1464 umfasst wird. Eine Deletion der Nukleotidbindedomäne führt zur Inaktivierung eines Proteins mit der Aktivität einer Glucan-Wasser-Dikinase (Mikkelsen und Blennow, 2005, Biochemical Journal 385, 355-361). Am N-Terminus weisen Proteine mit der Aktivität einer Glucan-Wasser-Dikinase eine Kohlenhydratbindedomäne (CBM, Carbohydrate Binding Domain) auf, die in der unter SEQ ID NO 2 dargestellten Aminosäuresequenz von den Aminosäuren 78 bis 362 umfasst wird. Kohlenhydratbindedomänen zeichnen sich u.a. dadurch aus, dass ihre Aminosäuresequenz konservierte Tryptophanreste aufweisen. Werden diese konservierten Aminosäurereste gegen andere Aminosäuren ausgetauscht, so verlieren die Kohlenhydratbindedomänen ihre Fähigkeit, Glucane zu binden. So führt z.B. ein Austausch der Aminosäuren W139 oder W194 in der unter SEQ ID NO 2 dargestellten Aminosäuresequenz zu einem Verlust der Funktion dieser Kohlenhydratbindedomäne. Wird die Kohlenhydratbindedomäne einer Glucan-Wasser-Dikinase deletiert (beispielsweise eine Deletion der Aminosäuren 1 bis 362, wobei die Aminosäuren 1 bis 77 in der unter SEQ ID NO 2 dargestellten Aminosäuresequenz ein plastidäres Signalpeptid darstellen), führt dies jedoch nicht zur Inaktivierung der phosphorylierenden Aktivität des Enzyms (Mikkelsen et al., 2006, Biochemistry 45: 4674-4682).

Nukleinsäuresequenzen und zu diesen korrespondierende Aminosäuresequenzen, kodierend ein Protein mit der Aktivität einer Glucan-Wasser-Dikinase sind aus unterschiedlichen Spezies, wie z.B. Kartoffel (WO 97/11188, Gen-Bank Acc.: AY027522, Y09533), Weizen (WO 00/77229, US 6,462,256, GenBank Acc.: AAN93923, GenBank Acc.: AR236165), Reis (GenBank Acc.: AAR61445, GenBank Acc.: AR400814), Mais (GenBank Acc.: AAR61444, GenBank Acc.: AR400813), Soyabohne (GenBank Acc.: AAR61446, GenBank Acc.: AR400815; Citrus (GenBank Acc.: AY094062), *Arabidopsis* (GenBank Acc.: AF312027) und der Grünalge *Ostreococcus tauri* (GenBank Acc.: AY570720.1) beschrieben. Die genannten Nukleinsäuresequenzen und Aminosäuresequenzen kodierend ein Protein mit der Aktivität einer Glucan-Wasser-Dikinase sind u.a. veröffentlicht vom NCBI (http://www.ncbi.nlm.nih.gov/entrez/) und sind durch Nennung der Referenzen ausdrücklich in die Beschreibung der vorliegenden Anmeldung aufgenommen.

Unter dem Begriff "GBSS I" ist im Zusammenhang mit der vorliegenden Erfindung jedes Enzym zu verstehen, das zur Gruppe der Stärkekorn-gebundenen Stärkesynthasen der Isoform I (EC 2.4.1.21) gehört.

Unter dem Begriff "GBSSI-Gen" im Zusammenhang mit der vorliegenden Erfindung ein Nukleinsäuremolekül oder Polynukleotid (cDNA, DNA) zu verstehen, das für eine Granule-Bound-Starch Synthase I (GBSS I) codiert. Seq ID No 7 - 12 umfassen Nukleinsäuresequenzen bzw. Aminosäuresequenzen, die jeweils für ein Protein mit der Aktivität einer GBSS I aus Reis, Weizen sowie Mais codieren.

Polynukleotide kodierend für GBSS I sind für verschiedene monokotyle Pflanzenspezies, wie z.B. für Mais (Genbank Acc. Nos. AF079260, AF079261), Weizen (Genbank Acc. Nos. AB019622, AB019623, AB019624), Reis (Genbank Acc. Nos. AF092443, AF092444, AF031162), Gerste (Genbank Acc. Nos. X07931, X07932), Sorghum bicolor (Genbank Acc. No U23945) und Hartweizen (Genbank Acc. No AB029063 beschrieben. Die genannten Nukleinsäuresequenzen und Aminosäuresequenzen kodierend ein Protein mit der Aktivität einer GBSS I sind u.a. veröffentlicht vom NCBI (http://www.ncbi.nlm.nih.gov/entrez/) und sind durch Nennung der Referenzen ausdrücklich in die Beschreibung der vorliegenden Anmeldung aufgenommen.

Mutanten, denen ein funktionales GBSS 1 -Gen fehlt, synthetisieren eine Amylose-freie Stärke (= "waxy"-Stärke). Solche sind für eine Reihe von Fruchtarten beschrieben, wie z.B. für Mais (z.B. bei Sprague et al, 1943, J. Am. Soc. Agron. 35:817-822; Shure et al. 1983, Cell 35: 225-233), Reis (Sano 1984, Theor. Appl. Genet. 68: 467-473; Villareal and Juliano 1986, Starch/Staerke 38:118-119), Gerste (Rohde et al 1988, Nucleic Acids Res 16: 7185-7186), Weizen (Nakamura et al 1995, Mol. Gen. Genet. 248: 253-259), Kartoffel (Hovenkamp-Hermelink et al. 1987, Theor. Appl. Genet. 75: 217-221) und Hirse (Okuno und Sakaguchi 1982, J. Hered 73: 467). Synonym wird der Begriff "waxy Mutante" verwendet, auf Grund des wachsfarben scheinenden Endosperms bei Mais. Auch das GBSS I-Protein wird häufig als "waxy Protein" bezeichnet (Kossmann und Lloyd 2000 "Understanding and Influencing Starch Biochemistry", Critical Reviews in Plant Sciences, 19(3): 171-226).

Geeignete Pflanzenzellen bzw. Pflanzen zur Herstellung der erfindungsgemäßen Pflanzenzellen und Pflanzen sind solche, die eine Reduktion des apparenten Amylosegehaltes in der von ihnen synthetisierten Stärke auf weniger als 5 Gew.-% aufweisen.

In einer Ausführungsform der vorliegenden Erfindung wird eine genetische Modifikation der erfindungsgemäßen Pflanzenzellen oder der erfindungsgemäßen Pflanzen durch Mutagenese eines oder mehrerer GBSS I Gene hervorgerufen. Die Art der Mutation ist dafür unerheblich, solange sie zu einer Verringerung oder vollständigen Reduktion der GBSSI-Aktivität und dadurch zu einer Reduktion des apparenten Amylosegehaltes der in den erfindungsgemäßen Pflanzen enthaltenen Stärke auf weniger als 5 Gew.-% führt.

Eine Mutation, die zur Verringerung der GBSSI-Aktivität und zu Reduktion des apparenten Amylosegehaltes der Stärke auf weniger als 5 Gew.-% in den erfindungsgemäßen Pflanzenzellen und Pflanzen führt, kann spontan auftreten und die entsprechenden Pflanzen können mit Hilfe der unten beschriebenen Methoden selektiert und vermehrt werden.

Im Rahmen der vorliegenden Erfindung ist mit "waxy-Mutante" eine Pflanze bezeichnet, deren Stärke einen Gehalt an apparenter Amylose von unter Gew.-5% enthält. Gleichermaßen bezeichnet "waxy-Stärke" eine Stärke mit einem Gehalt an apparenter Amylose von unter Gew.-5%.

Unter dem Begriff "Mutagenese" ist im Zusammenhang mit der vorliegenden Erfindung jegliche Art von eingeführten Mutationen zu verstehen, wie z.B. Deletionen, Punktmutationen (Nukleotidaustausche), Insertionen, Inversionen, Genkonversionen oder Chromosomentranslokationen.

Agenzien, die zur Erzeugung chemisch induzierter Mutationen eingesetzt werden können und die durch Einwirkung der entsprechenden Mutagene dabei entstehenden Mutationsarten sind z.B. beschrieben von Ehrenberg und Husain (1981, Mutation Research 86: 1-113) und Müller (1972, Biologisches Zentralblatt 91 (1): 31-48). Die Erzeugung von Reis-Mutanten unter Verwendung von Gamma-Strahlen, Ethyl-Methan-Sulfonat (EMS), N-Methyl-N-nitrosoharnstoff oder Natriumazid (NaN$_3$) ist z.B. beschrieben von Jauhar und Siddiq (1999, Indian Journal of Genetics, 59 (1): 23-28), Rao (1977, Cytologica 42: 443-450), Gupta und Sharma (1990, Oryza 27: 217-219) und Satoh und Omura (1981, Japanese Journal of Breeding 31 (3): 316-326). Die Erzeugung von Weizen-Mutanten unter Verwendung von NaN$_3$ bzw. Maleinsäurehydrazid ist von Arora et al. (1992, Annals of Biology 8 (1): 65-69) beschrieben. Eine Übersicht zur Erzeugung von Weizen-Mutanten unter Verwendung von verschiedenen Arten energiereicher Strahlung und chemischer Agenzien ist von Scarascia-Mugnozza et al. (1993, Mutation Breeding Review 10: 1-28) dargestellt. Svec et al. (1998, Cereal Research Communications 26 (4): 391-396) beschreiben die Anwendung von N-Ethyl-N-nitrosoharnstoff zur Erzeugung von Mutanten in Triticale. Die Verwendung von MMS (Methylmethansulfonsäure) und GammaStrahlung zur Erzeugung von Hirse-Mutanten ist von Shashidhara et al. (1990, Journal of Maharashtra Agricultural Universities 15 (1): 20-23) beschrieben.

Monokotyle Pflanzenzellen und Pflanzen, die eine Stärke mit einem apparenten Amylosegehalt von weniger als 5 Gew.-% synthetisieren (= waxy-Pflanzen bzw. - zellen), können auch durch die Verwendung der so genannten Insertionsmutagenese (Übersichtsartikel: Thorneycroft et al., 2001, Journal of Experimental Botany 52 (361): 1593-1601) hergestellt werden. Unter "Insertionsmutagenese" ist insbesondere das Inserieren von Transposons oder so genannter Transfer-DNA (T-DNA) in ein Gen zu verstehen.

Bei den Transposons kann es sich dabei sowohl um solche handeln, die in einer (Wildtyp-) Pflanzenzelle natürlicherweise vorkommen (endogene Transposons), als auch um solche, die natürlicherweise nicht in besagter Zelle vorkommen, sondern mittels gentechnischer Methoden, wie z.B. Transformation, in die Zelle eingeführt werden (heterologe Transposons). Die Veränderung der Expression von Genen mittels Transposons ist dem Fachmann bekannt. Eine Übersicht über die Nutzung von endogenen und heterologen Transposons als Werkzeuge in der Pflanzenbiotechnologie ist von Ramachandran und Sundaresan (2001, Plant Physiology and Biochemistry 39: 234-252) dargestellt. Die Möglichkeit, Mutanten zu identifizieren, bei welchen spezifische Gene durch Transposoninsertionsmutagenese inaktiviert wurden, ist in einer Übersicht von Maes et al. (1999, Trends in Plant Science 4 (3): 90-96) dargestellt. Die Erzeugung von Reis-Mutanten mit Hilfe endogener Transposons ist von Hirochika (2001, Current Opinion in Plant Biology 4: 118-122) beschrieben. Die Identifizierung von Mais-Genen mit Hilfe endogener Retrotransposons wird z.B. von Hanley et al. (2000, The Plant Journal 22 (4): 557-566) dargestellt. Die Möglichkeit, Mutanten mit Hilfe von Retrotransposons herzustellen und Methoden, Mutanten zu identifizieren, sind von Kumar und Hirochika (2001, Trends in Plant Science 6 (3): 127-134) beschrieben. Die Aktivität von heterologen Transposons in unterschiedlichen Spezies ist sowohl für dikotelydone als auch für monokotyledone Pflanzen beschrieben worden: z.B. für Reis (Greco et al., 2001, Plant Physiology 125: 1175-1177; Liu et al., 1999, Molecular and General Genetics 262: 413-420; Hiroyuki et al., 1999, The Plant Journal 19 (5): 605-613; Jeon und Gynheung, 2001, Plant Science 161: 211-219), Gerste (Koprek et al., 2000, The Plant Journal 24 (2): 253-263), Arabidopsis thaliana (Aarts et al., 1993, Nature 363: 715-717; Schmidt und Willmitzer, 1989, Molecular and General Genetics 220: 17-24; Altmann et al., 1992, Theoretical and Applied Genetics 84: 371-383; Tissier et al., 1999, The Plant Cell 11: 1841-1852), Tomate (Belzile und Yoder, 1992, The Plant Journal 2 (2): 173-179) und Kartoffel (Frey et al., 1989, Molecular and General Genetics 217: 172-177; Knapp et al., 1988, Molecular and General Genetics 213: 285-290).

Grundsätzlich können monokotyle "waxy"-Pflanzenzellen und -Pflanzen mit Hilfe homologer als auch heterologer Transposons hergestellt werden, wobei unter Verwendung von homologen Transposons solche zu verstehen sind, die bereits natürlicherweise im Pflanzengenom vorhanden sind.

Ebenfalls ist die T-DNA-Mutagenese grundsätzlich zur Erzeugung von "waxy"-Pflanzenzellen und -Pflanzen geeignet.

Die T-DNA-Insertionsmutagenese beruht darauf, dass bestimmte Abschnitte (T-DNA) von Ti-Plasmiden aus Agrobacterium in das Genom von pflanzlichen Zellen integriert werden können. Der Ort der Integration in das pflanzliche Chromosom ist dabei nicht festgelegt, sondern kann an jeder beliebigen Stelle erfolgen. Integriert die T-DNA in einen Abschnitt des Chromosoms, der eine Genfunktion darstellt, so kann dieses zur Veränderung der Genexpression und damit auch zur Änderung der Aktivität eines durch das betreffende Gen codierten Proteins führen. Insbesondere führt die Integration einer T-DNA in den kodierenden Bereich eines Gens häufig dazu, dass das entsprechende Protein von der betreffenden Zelle gar nicht mehr oder nicht mehr in aktiver Form synthetisiert werden kann. Die Verwendung von T-DNA-Insertionen zur Erzeugung von Mutanten ist z.B. für Arabidopsis thaliana (Krysan et al., 1999, The Plant Cell 11: 2283-2290; Atipiroz-Leehan und Feldmann, 1997, Trends in Genetics 13 (4): 152-156; Parinov und Sundaresan, 2000, Current Opinion in Biotechnology 11: 157-161) und Reis (Jeon und An, 2001, Plant Science 161: 211-219; Jeon et al., 2000, The Plant Journal 22 (6): 561-570) beschrieben. Methoden zur Identifizierung von Mutanten, die mit Hilfe der T-DNA-Insertionsmutagenese erzeugt wurden, sind u.a. beschrieben von Young et al. (2001, Plamt Physiology 125: 513-518), Parinov et al. (1999, The Plant Cell 11: 2263-2270), Thorneycroft et al. (2001, Journal of Experimental Botany 52: 1593-1601) und McKinney et al. (1995, The Plant Journal 8 (4): 613-622).

Das Auffinden von Mutationen in den entsprechenden Genen, kann mit Hilfe von dem Fachmann bekannten Methoden geschehen. Es können molekulare Analysen, basierend auf Hybridisierungen mit Sonden ("Southern Blot"), der Amplifikation mittels Polymerasekettenreaktion (PCR), der Sequenzierung betreffender genomischer Nukleinsäure-Fragmente und die Suche nach einzelnen Nukleotidaustauschen angewandt werden. Eine Methode, um Mutationen anhand von Hybridisierungsmustern zu identifizieren, ist z.B. die Suche nach Restriktionsfragment-Längenunterschieden ("Restriction Fragment Length Polymorphism", RFLP) (Nam et al., 1989, The Plant Cell 1: 699-705; Leister and Dean, 1993, The Plant Journal 4 (4): 745-750). Eine auf PCR basierende Methode ist z.B. die Analyse von amplifizierten Fragment-Längenunterschieden ("Amplified Fragment Length Polymorphism", AFLP) (Castiglioni et al., 1998, Genetics 149: 2039-2056; Meksem et al., 2001, Molecular Genetics and Genomics 265: 207-214; Meyer et al. 1998, Molecular and General Genetics 259: 150-160). Auch die Verwendung von mit Restriktionsendonukleasen geschnittenen amplifizierten Fragmenten ("Cleaved Amplified Polymorphic Sequences", CAPS) kann zur Identifizierung von Mutationen herangezogen werden (Konieczny und Ausubel, 1993, The Plant Journal 4: 403-410; Jarvis et al., 1994, Plant Mol. Biol. 24: 685-687; Bachem et al., 1996, The Plant Journal 9 (5): 745-753). Methoden zur Ermittlung von SNPs sind u.a. von Qi et al. (2001, Nucleic Acids Research 29 (22): 116), Drenkard et al. (2000, Plant Physiology 124: 1483-1492) und Cho et al. (1999, Nature Genetics 23: 203-207) beschrieben worden. Insbesondere sind Methoden, die es erlauben, viele Pflanzen innerhalb kurzer Zeit auf Mutationen in bestimmten Genen hin zu untersuchen, geeignet. Solch eine Methode, das sogenannte TILLING ("Targetting Induced Local Lesions In Genomes") ist von McCallum et al. (2000, Plant Physiology 123: 439-442) beschrieben worden.

Dem Fachmann ist bekannt, dass es sich bei oben beschriebenen Mutationen in der Regel um rezessive Mutationen handelt. Zur Ausprägung des waxy Phänotyps ist es daher notwendig, reinerbige (homozygote) Pflanzenzellen bzw. Pflanzen zu erzeugen. Verfahren zur Erzeugung von reinerbigen Pflanzen sind dem Fachmann bekannt.

Homozygote "waxy" Mutanten können mittels Jodfärbung der Stärke identifiziert werden. Dazu werden stärkehaltige Gewebeproben (z.B. Endosperm, Pollen) mit Jod-Lösung angefärbt und z.B. im Mikroskop untersucht. Waxy-Stärken färben braun (im Vergleich zur blau-Färbung des Wildtyps).

In einer weiteren Ausführungsform der vorliegenden Erfindung führt die Einführung eines oder mehrerer fremder Nukleinsäuremoleküle/Polynukleotide, deren Vorhandensein und/oder die Expression eines oder mehrerer fremder Nukleinsäuremoleküle/Polynukleotide zur Inhibierung der Expression von endogenen Genen, die für das GBSS I-Protein kodieren sowie zu einer Reduktion des apparenten Amylosegehaltes der in der erfindungsgemäßen Pflanzenzelle bzw. erfindungsgemäßen Pflanze enthaltenen Stärke auf weniger als 5 Gew.-%.

Dies kann durch verschiedene, dem Fachmann bekannte Verfahren erzielt werden. Hierzu zählen beispielsweise die Expression einer entsprechenden Antisense-RNA, oder einer doppelsträngigen RNA, die Bereitstellung von Molekülen oder Vektoren, die einen Cosuppressions-Effekt vermitteln, die Expression eines entsprechend konstruierten Ribozyms, das spezifisch Transkripte spaltet, die für GBSSI kodieren, oder die so genannte "in vivo-Mutagenese". Ferner kann die Verringerung der GBSSI-Aktivität(en) und/oder die Verringerung der Genexpression des GBSSI-Gens in den Pflanzenzellen auch durch die simultane Expression von Sense- und Antisense-RNA-Molekülen des jeweiligen zu reprimierenden Zielgens, vorzugsweise des GBSSI-Gens, hervorgerufen werden. Diese Methoden sind dem Fachmann geläufig.

Darüber hinaus ist bekannt, dass in planta die Bildung von doppelsträngiger RNA von Promotorsequenzen in trans zu einer Methylierung und einer transkriptionellen Inaktivierung homologer Kopien dieses Promotors führen kann (Mette et al., 2000, EMBO J. 19: 5194-5201).

Zur Inhibierung der Genexpression mittels Antisense- oder Cosuppressions-Technologie kann beispielsweise ein DNA-Molekül verwendet werden, das die gesamte für GBSSI kodierende Sequenz einschließlich eventuell vorhandener flankierender Sequenzen umfasst, als auch DNA-Moleküle, die nur Teile der kodierenden Sequenz umfassen, wobei diese Teile lang genug sein müssen, um in den Zellen einen Antisense-Effekt bzw. Cosuppressions-Effekt zu bewirken. Geeignet sind im Allgemeinen Sequenzen mit einer Mindestlänge von 15 bp, bevorzugt mit einer Mindestlänge von 20-30 bp, besonders bevorzugt mit einer Länge von 100-500 bp, für eine sehr effiziente Antisense- bzw. Cosuppressions-Inhibition insbesondere Sequenzen mit einer Länge über 500 bp.

Für Antisense- oder Cosuppressions-Ansätze geeignet, ist auch die Verwendung von Polynukleotidsequenzen, die einen hohen Grad an Identität zu den endogen in der Pflanzenzelle vorkommenden Sequenzen haben, die für GBSSI kodieren. Die minimale Identität sollte größer als ca. 65% sein. Die Verwendung von Sequenzen mit Identitäten von mindestens 90%, insbesondere zwischen 95% und 100% ist zu bevorzugen.

Ferner ist zur Erzielung eines Antisense- oder eines Cosuppressions-Effekts auch die Verwendung von Introns denkbar, d.h. von nicht kodierenden Bereichen von Genen, die für GBSSI kodieren.

Die Verwendung von Intron-Sequenzen zur Inhibierung der Expression von Genen, die für Proteine der Stärkebiosynthese kodieren, wurde beschrieben in WO 97/04112, WO 97/04113, WO 98/37213, WO 98/37214.

Dem Fachmann ist bekannt, wie er einen Antisense- und einen Cosuppressions-Effekt erzielen kann. Das Verfahren der Cosuppressions-Inhibierung wurde beispielsweise beschrieben von Jorgensen (1990, Trends Biotechnol. 8: 340-344), Niebel et al. (1995, Top. Microbiol. Immunol. 197: 91-103), Flavell et al. (1995, Curr. Top. Microbiol. Immunol. 197: 43-46), Palauqui und Vaucheret (1995, Plant Mol. Biol. 29: 149-159), Vaucheret et al. (1995, Mol. Gen. Genet. 248: 311-317), de Borne et al. (1994, Mol. Gen. Genet. 243: 613-621).

Ferner kann die Verringerung der GBSSI-Aktivität in den Pflanzenzellen auch durch die simultane Expression von Sense- und Antisense-RNA-Molekülen des jeweiligen zu reprimierenden Zielgens, vorzugsweise des GBSSI-Gens, hervorgerufen werden.

Dies kann beispielsweise durch die Verwendung von chimären Konstrukten erreicht werden, die "inverted repeats" des jeweiligen Zielgens oder Teilen des Zielgens enthalten. Hierbei kodieren die chimären Konstrukte für Sense- und Antisense-RNA-Moleküle des jeweiligen Zielgens. Sense- und Antisense-RNA werden in planta gleichzeitig als ein RNA-Molekül synthetisiert, wobei Sense- und Antisense-RNA durch einen Spacer voneinander getrennt sein und ein doppelsträngiges RNA-Molekül bilden können (RNAi-Technologie).

Es konnte gezeigt werden, dass die Einführung von inverted-repeat-DNA-Konstrukten in das Genom von Pflanzen eine sehr effiziente Methode ist, um die zu den inverted-repeat-DNA-Konstrukten korrespondierenden Gene zu reprimieren (Waterhouse et al., 1998, Proc. Natl. Acad. Sci. USA 95, 13959-13964; Wang und Waterhouse, 2000, Plant Mol. Biol. 43, 67-82; Singh et al., 2000, Biochemical Society Transactions 28 (6), 925- 927; Liu et al., 2000, Biochemical Society Transactions 28 (6), 927-929; Smith et al., 2000, Nature 407, 319-320; WO 99/53050). Sense- und Antisense-Sequenzen des Zielgens bzw. der Zielgene können auch getrennt voneinander mittels gleicher oder unterschiedlicher Promotoren exprimiert werden (Nap et al, 6th International Congress of Plant Molecular Biology, 18.-24. Juni 2000, Quebec, Poster S7-27, Vortrag Session S7).

Auch die Expression von Ribozymen zur Verringerung der Aktivität von bestimmten Enzymen in Zellen ist dem Fachmann bekannt und ist beispielsweise beschrieben in EP-B1 0321201. Die Expression von Ribozymen in pflanzlichen Zellen wurde z.B. beschrieben von Feyter et al. (1996, Mol. Gen. Genet. 250: 329-338).

Ferner kann die Verringerung der GBSSI-Aktivität und/oder die Reduktion des apparenten Amylose-Gehaltes der in den Pflanzenzellen enthaltenen Stärke auf weniger als 5 Gew.-% auch durch die so genannte "in vivo-Mutagenese" erreicht werden, bei der durch Transformation von Zellen ein hybrides RNA-DNA-Oligonukleotid ("Chimeroplast") in Zellen eingeführt wird (Kipp et al., Poster Session beim 5th International Congress of Plant Molecular Biology, 21.-27. September 1997, Singapur; R. A. Dixon und C. J. Arntzen, Meeting report zu Metabolic Engineering in Transgenic Plants, Keystone Symposia, Copper Mountain, CO, USA, 1997, TIBTECH 15: 441-447; WO 95/15972; Kren et al., 1997, Hepatology 25: 1462-1468; Cole-Strauss et al., 1996, Science 273: 1386-1389; Beetham et al., 1999, PNAS 96: 8774-8778).

Ein Teil der DNA-Komponente des RNA-DNA-Oligonukleotids ist homolog zu einer Polynukleotidsequenz eines endogenen GBSSI-Gens, weist jedoch im Vergleich zur Polynukleotidsequenz eines endogenen GBSSI-Gens eine Mutation auf oder enthält eine heterologe Region, die von den homologen Regionen umschlossen ist. Durch Basenpaarung der homologen Regionen des RNA-DNA-Oligonukleotids und des endogenen Polynukleotids, gefolgt von homologer Rekombination, kann die in der DNA-Komponente des RNA-DNA-Oligonukleotids enthaltene Mutation oder heterologe Region in das Genom einer Pflanzenzelle übertragen werden.

Die Verringerung der GBSSI-Aktivität in den Pflanzenzellen kann somit auch durch die Erzeugung doppelsträngiger RNA-Moleküle von GBSSI-Genen erreicht werden. Vorzugsweise werden hierzu "inverted repeats" von DNA-Molekülen, die von GBSSI-Genen dargestellten Nukleotidsequenz oder -cDNAs abgeleitet sind, in das Genom von Pflanzen eingeführt, wobei die zu transkribierenden DNA-Moleküle unter Kontrolle eines Promotors stehen, der die Expression besagter RNA-Moleküle steuert.

Eine weitere Möglichkeit, die Aktivität von Proteinen in Pflanzenzellen oder Pflanzen zu verringern, ist die Methode der sogenannten Immunomodulation. Es ist bekannt, dass eine *in planta* Expression von Antikörpern, die ein pflanzliches Protein spezifisch erkennen, durch Ausbildung eines Protein-Antikörper-Komplexes eine Verringerung der Aktivität betreffender Proteine in entsprechenden Pflanzenzellen oder Pflanzen zur Folge hat (Conrad und Manteufel, 2001, Trends in Plant Science 6: 399-402; De Jaeger et al., 2000, Plant Molecular Biology 43: 419-428; Jobling et al., 2003, Nature Biotechnology 21: 77-80).

Alle genannten Verfahren basieren auf der Einführung eines oder mehrerer fremden Nukleinsäuremoleküle in das Genom von Pflanzenzellen oder Pflanzen und sind daher grundsätzlich geeignet, erfindungsgemäße Pflanzenzellen und erfindungsgemäße Pflanzen herzustellen.

Die Verringerung der Expression kann beispielsweise bestimmt werden durch Messung der Menge an Transkripten, die für die betreffenden Enzyme kodieren, z.B. durch Northern-Blot-Analyse oder quantitative RT-PCR. Die Verringerung der Menge an GBSSI-Protein kann beispielsweise bestimmt werden durch immunologische Methoden, wie Western-Blot-Analyse, ELISA ("Enzyme Linked Immuno Sorbent Assay") oder RIA ("Radio Immune Assay").

Eine Verringerung der GBSSI-Aktivität in den erfindungsgemäßen Pflanzenzellen bzw. Pflanzen kann auch mittelbar durch die Quantifizierung des Reaktionsproduktes des GBSSI-Proteins, der Amylose nachgewiesen werden. Für die Bestimmung des Anteiles an Amylose in pflanzlichen Stärken sind dem Fachmann eine Vielzahl von Methoden bekannt. Für Cerealien insbesondere Reis erfolgt die Bestimmung des Gehaltes apparenter Amylose bevorzugt in Anlehnung an die Methode von Juliano (1971, Cereal Science Today 16 (10): 334-340), wie weiter unten unter "Material und Methoden" beschrieben.

In einer weiteren Ausführungsform kann zur Erzeugung der erfindungsgemäßen Pflanzenzellen oder der erfindungsgemäßen Pflanzen anstelle einer Wildtyp-Pflanzenzelle bzw. -Pflanze eine Mutante verwendet werden, die sich dadurch auszeichnet, dass sie bereits eine Stärke mit einem apparenten Amylosegehalt von weniger als 5 Gew.-% synthetisiert und/oder eine erhöhte Aktivität eines Proteins mit der Aktivität einer Glucan-Wasser-Dikinase und/oder eine erhöhte Aktivität eines Proteins mit der Aktivität einer Stärkesynthase II aufweist. Bei diesen Mutanten kann es sich sowohl um spontan auftretende Mutanten als auch um solche handeln, die durch den gezielten Einsatz von Mutagenen erzeugt wurden. Möglichkeiten zur Erzeugung von solchen Mutanten sind vorstehend beschrieben worden.

Weiterhin umfasst die vorliegende Erfindung eine erfindungsgemäße genetisch modifizierte monokotyle Pflanzenzelle bzw. Pflanze, deren genetische Modifikation in der Einführung mindestens eines fremden Nukleinsäuremoleküls in das Genom der zur Transformation verwendeten Pflanze besteht.

Durch Einführung eines fremden Nukleinsäuremoleküls sind die erfindungsgemäßen Pflanzenzellen oder erfindungsgemäßen Pflanzen in ihrer genetischen Information verändert. Das Vorhandensein mindestens eines fremden Nukleinsäuremoleküls führt zu einer phänotypischen Veränderung. "Phänotypische" Veränderung bedeutet dabei vorzugsweise eine messbare Veränderung einer oder mehrerer Funktionen der Zellen. Beispielsweise zeigen die genetisch modifizierten erfindungsgemäßen Pflanzenzellen und die genetisch modifizierten erfindungsgemäßen Pflanzen aufgrund des Vorhandenseins oder bei Expression eingeführter fremder Nukleinsäuremoleküle eine Erhöhung der Aktivität eines Proteins mit der Aktivität einer Glucan-Wasser-Dikinase und eine Erhöhung der Aktivität eines Proteins mit der Aktivität einer Stärkesynthase II und/oder eine Reduktion der Aktivität eines Proteins mit der Aktivität einer GBSS I.

Unter dem Begriff "fremdes Nukleinsäuremolekül" versteht man im Zusammenhang mit der vorliegenden Erfindung ein solches Molekül, das entweder natürlicherweise in den zur Transformation verwendeten Pflanzenzellen nicht vorkommt, oder das in der konkreten räumlichen Anordnung nicht natürlicherweise in den zur Transformation verwendeten Pflanzenzellen vorkommt oder das an einem Ort im Genom der zur Transformation verwendeten Pflanzenzelle lokalisiert ist, an dem es natürlicherweise nicht vorkommt. Bevorzugt ist das fremde Nukleinsäuremolekül ein rekombinantes Molekül, das aus verschiedenen Elementen besteht, deren Kombination oder spezifische räumliche Anordnung natürlicherweise in pflanzlichen Zellen nicht auftritt.

So können rekombinante Nukleinsäuremoleküle z.B. neben Nukleinsäuremolekülen, die ein Protein mit der Aktivität einer Glucan-Wasser-Dikinase und/oder ein Protein mit der Aktivität einer Stärkesynthase II kodieren und/oder eine Nukleinsäure, die eine Reduktion der Aktivität einer GBSSI bewirkt, zusätzliche Nukleinsäuresequenzen aufweisen, welche natürlicherweise nicht in Kombination mit den genannten Nukleinsäuremolekülen vorliegen. Die genannten zusätzlichen Nukleinsäuresequenzen, die auf einem rekombinanten Nukleinsäuremolekül in Kombination mit einem Protein mit der Aktivität einer Glucan-Wasser-Dikinase und/oder einem Protein mit der Aktivität einer Stärkesynthase II kodierenden Nukleinsäuremolekül vorliegen und/oder einer Nukleinsäure, die geeignet ist, eine Reduktion der Aktivität eines Proteins mit der Aktivität einer GBSSI zu vermitteln, können dabei beliebige Sequenzen sein. Sie können z.B. genomische und/oder pflanzliche Nukleinsäuresequenzen darstellen. Bevorzugt handelt es sich bei diesen zusätzlichen Nukleinsäuresequenzen um regulatorische Sequenzen (Promotoren, Terminationssignale, Enhancer), besonders bevorzugt um regulatorische Sequenzen, die in pflanzlichem Gewebe aktiv sind, insbesondere bevorzugt um gewebespezifische regulatorische Sequenzen.

Methoden zur Erzeugung rekombinanter Nukleinsäuremoleküle sind dem Fachmann bekannt und umfassen gentechnische Methoden, wie z.B. die Verbindung von Nukleinsäuremolekülen durch Ligation, genetische Rekombination oder die Neusynthese von Nukleinsäuremolekülen (siehe z.B. Sambrok et al., Molecular Cloning, A Laboratory Manual, 3rd edition (2001) Cold Spring Harbour Laboratory Press, Cold Spring Harbour, NY, ISBN: 0879695773; Ausubel et al., Short Protocols in Molecular Biology, John Wiley & Sons; 5th edition (2002), ISBN: 0471250929).

Unter dem Begriff "Genom" soll im Zusammenhang mit der vorliegenden Erfindung die Gesamtheit des in einer pflanzlichen Zelle vorliegenden Erbmaterials verstanden werden. Dem Fachmann ist bekannt, dass neben dem Zellkern auch andere Kompartimente (z.B. Plastiden, Mitochondrien) Erbmaterial enthalten.

Prinzipiell kann ein fremdes Nukleinsäuremolekül jedes beliebige Nukleinsäuremolekül sein, das in der Pflanzenzelle oder Pflanze eine Erhöhung der Aktivität eines Proteins mit der Aktivität einer Glucan-Wasser-Dikinase und eines Proteins mit der Aktivität einer Stärkesynthase II sowie eine Reduktion der Aktivität eines Proteins mit der Aktivität einer GBSSI bewirkt.

In einer bevorzugten Ausführungsform handelt es sich bei den fremden Nukleinsäuremolekülen kodierend ein Protein mit der Aktivität einer Glucan-Wasser-Dikinase um die bereits genannten, dem Fachmann bekannten Nukleinsäuremoleküle aus den verschiedenen Pflanzenspezies. Besonders bevorzugt sind dabei Nukleinsäuremoleküle, kodierend ein Protein mit der Aktivität einer Glucan-Wasser-Dikinase aus Kartoffel, insbesondere bevorzugt ein Protein mit der Aktivität einer Glucan-Wasser-Dikinase, welches die unter SEQ ID NO 2 dargestellte Aminosäuresequenz aufweist oder von der in SEQ ID NO 1 dargestellten Nukleinsäuresequenz codiert wird.

In einer weiteren bevorzugten Ausführungsform handelt es sich bei den fremden Nukleinsäuremolekülen, kodierend ein Protein mit der Aktivität einer Stärkesynthase II, um die bereits genannten, dem Fachmann bekannten Nukleinsäuremoleküle aus den verschiedenen Pflanzenspezies. Besonders bevorzugt sind dabei Nukleinsäuremoleküle, kodierend ein Protein mit der Aktivität einer Stärkesynthase II aus Weizen, insbesondere bevorzugt ein Protein mit der Aktivität einer Stärkesynthase 11, welches die unter SEQ ID NO 4 dargestellte Aminosäuresequenz aufweist oder von der in SEQ ID NO 3 dargestellten Nukleinsäuresequenz codiert wird.

In einer weiteren bevorzugten Ausführungsform handelt es sich um Nukleinsäuremoleküle, kodierend ein Protein mit der Aktivität einer Stärkesynthase II aus Reis, insbesondere bevorzugt ein Protein mit der Aktivität einer Stärkesynthase II, welches die unter SEQ ID NO 6 dargestellte Aminosäuresequenz aufweist oder von der in SEQ ID NO 5 dargestellten Nukleinsäuresequenz codiert wird.

In einer weiteren bevorzugten Ausführungsform handelt es sich bei den fremden Nukleinsäuremolekülen, kodierend ein Protein mit der Aktivität einer GBSSI, um die bereits genannten, dem Fachmann bekannten Nukleinsäuremoleküle aus den verschiedenen Pflanzenspezies. Besonders bevorzugt sind dabei Nukleinsäuremoleküle, kodierend ein Protein mit der Aktivität einer GBSSI aus Reis, insbesondere bevorzugt ein Protein mit der Aktivität einer GBSSI, welches die unter SEQ ID NO 8 dargestellte Aminosäuresequenz aufweist oder von der in SEQ ID NO 7 dargestellten Nukleinsäuresequenz codiert wird.

In einer weiteren bevorzugten Ausführungsform handelt es sich um Nukleinsäuremoleküle, kodierend ein Protein mit der Aktivität einer GBSSI aus Weizen, insbesondere bevorzugt ein Protein mit der Aktivität einer GBSSI, welches die unter SEQ ID NO 10 dargestellte Aminosäuresequenz aufweist oder von der in SEQ ID NO 9 dargestellten Nukleinsäuresequenz codiert wird.

In einer weiteren bevorzugten Ausführungsform handelt es sich Nukleinsäuremoleküle, kodierend ein Protein mit der Aktivität einer GBSSI aus Mais, insbesondere bevorzugt ein Protein mit der Aktivität einer GBSSI, welches die unter SEQ ID NO 12 dargestellte Aminosäuresequenz aufweist oder von der in SEQ ID NO 11 dargestellten Nukleinsäuresequenz codiert wird.

In einer weiteren Ausführungsform sind die erfindungsgemäßen Pflanzenzellen und Pflanzen dadurch gekennzeichnet, dass sie homozygot für die waxy Mutation(en) sind und damit eine Stärke synthetisieren, deren Gehalt an apparenter Amylose unter 5 Gew-% liegt.

Unter dem Begriff "homozygot für die waxy-Mutation(en)" soll im Zusammenhang mit der vorliegenden Erfindung die Reinerbigkeit für die nicht funktionellen GBSSI-Gene verstanden werden. Der Fachmann versteht unter Homozygotie, dass im Erbgut einer Zelle alle Allele zu einem bestimmten Merkmal identisch sind, also zwei oder mehr identische Kopien eines bestimmten Gens auf den beiden Chromatiden eines Chromosoms vorliegen, die das Gen enthalten. Sie sind bezüglich dieses Gens homozygot (= reinerbig) und geben bei Selbstung das entsprechende Merkmal an alle Nachkommen weiter. Dem Fachmann ist bekannt, dass bei polyploiden Pflanzen wie z.B. Weizen, für die Ausprägung des waxy Phänotyps unter Umständen notwendig ist, drei nicht funktionale GBSSI Allele (auf dem A, B und D Sub-Genom) homozygot vorliegen zu haben.

Bei den zur genetischen Modifikation in die den waxy-Phänotyp ausprägenden Pflanzenzelle oder Pflanze eingebrachten fremden Nukleinsäuremolekülen kann es sich um ein einzelnes Nukleinsäuremolekül oder um mehrere Nukleinsäuremoleküle handeln. Es kann sich dabei sowohl um Nukleinsäuremoleküle handeln, die für ein Protein mit der Aktivität einer Glucan-Wasser-Dikinase kodierende Nukleinsäuresequenzen und für ein Protein mit der Aktivität einer Stärkesynthase II kodierende Nukleinsäuresequenzen enthalten, als auch um Nukleinsäuremoleküle, bei denen die für ein Protein mit der Aktivität einer Glucan-Wasser-Dikinase kodierenden Nukleinsäuresequenzen und die für ein Protein mit der Aktivität einer Stärkesynthase II kodierenden Nukleinsäuresequenzen auf verschiedenen Nukleinsäuremolekülen vorliegen. Die für ein Protein mit der Aktivität einer Glucan-Wasser-Dikinase kodierenden Nukleinsäuresequenzen und die für ein Protein mit der Aktivität einer Stärkesynthase II kodierenden Nukleinsäuresequenzen können beispielsweise in einem Vektor, Plasmid oder in linear vorliegenden Nukleinsäuremolekülen gleichzeitig enthalten sein ("Doppelkonstrukt") oder aber Bestandteile von zwei jeweils voneinander getrennten Vektoren, Plasmiden oder linearen Nukleinsäuremolekülen sein.

Liegen die für ein Protein mit der Aktivität einer Glucan-Wasser-Dikinase kodierenden Nukleinsäuresequenzen und die für ein Protein mit der Aktivität einer Stärkesynthase II kodierenden Nukleinsäuresequenzen in zwei voneinander getrennten Nukleinsäuremolekülen vor, so können sie entweder zeitgleich ("Cotransformation") oder auch nacheinander, d.h. zeitlich aufeinander folgend ("Supertransformation") in das Genom der Pflanzenzelle oder Pflanze eingeführt werden. Die voneinander getrennten Nukleinsäuremoleküle können auch in verschiedene individuelle Pflanzenzellen oder Pflanzen einer Spezies eingeführt werden. Es können dadurch Pflanzenzellen oder Pflanzen erzeugt werden, bei welchen die Aktivität von entweder mindestens einem Protein mit der Aktivität einer Glucan-Wasser-Dikinase oder aber mindestens einem Protein mit der Aktivität einer Stärkesynthase II erhöht ist. Erfindungsgemäße Pflanzen können dann durch anschließendes Kreuzen derjenigen Pflanzen, bei denen die Aktivität eines Proteins mit der Aktivität einer Glucan-Wasser-Dikinase erhöht ist, mit solchen, bei denen die Aktivität eines Proteins mit der Aktivität einer Stärkesynthase II erhöht ist, hergestellt werden. Die Parameter zur Auswahl der Pflanzen, die für die jeweiligen Verfahrensschritte verwendet werden, sind weiter unten definiert.

In einer weiteren Ausführungsform wird der waxy-Phänotyp der erfindungsgemäßen Pflanzenzellen bzw. Pflanzen durch das Einbringen eines oder mehrerer rekombinanter Nukleinsäuremoleküle bewirkt, welche für die Reduktion der GBSSI Aktivität geeignet ist.

Bei den zur genetischen Modifikation in die Wildtyp-Pflanzenzelle bzw. -Pflanze eingebrachten fremden Nukleinsäuremolekülen kann es sich um ein einzelnes Nukleinsäuremolekül oder um mehrere Nukleinsäuremoleküle handeln. Es kann sich daher sowohl um Nukleinsäuremoleküle handeln, die für ein Protein mit der Aktivität einer Glucan-Wasser-Dikinase und für ein Protein mit der Aktivität einer Stärkesynthase II kodierende Nukleinsäuresequenzen enthalten und zusätzlich für Nukleinsäuresequenzen, die zur Inhibition der Aktivität der GBSSI Aktivität geeignet sind (Dreifachkonstrukt). Gleichermaßen kann es sich auch um Nukleinsäuremoleküle handeln, bei denen die für ein Protein mit der Aktivität einer Glucan-Wasser-Dikinase kodierenden Nukleinsäuresequenzen und die für ein Protein mit der Aktivität einer Stärkesynthase II kodierenden Nukleinsäuresequenzen auf verschiedenen Nukleinsäuremolekülen vorliegen, wobei das eine oder das andere dieser beiden Nukleinsäuremoleküle zusätzlich Nukleinsäuresequenzen beinhaltet, welche zur Inhibition der Aktivität der GBSSI Aktivität geeignet sind. Alternativ kann es sich auch um Nukleinsäuremoleküle handeln, bei denen die für ein Protein mit der Aktivität einer Glucan-Wasser-Dikinase kodierenden Nukleinsäuresequenzen und die für ein Protein mit der Aktivität einer Stärkesynthase II kodierenden Nukleinsäuresequenzen auf einem Nukleinsäuremolekül vorliegen und die Nukleinsäuremoleküle, die geeignet sind zur Inhibition der GBSSI Aktivität auf einem anderen Nukleinsäuremolekül vorliegen (3 Varianten von einem Doppelkonstrukt und einem Einfachkonstrukt).

In einer weiteren Ausführungsform kann es sich auch um drei verschiedene Nukleinsäuremoleküle handeln, wobei eines für eine Glucan-Wasser-Dikinase Protein kodierende Nukleinsäuresequenzen enthält, ein anderes für ein Stärkesynthase II kodierende Nukleinsäuresequenzen enthält und ein weiteres für die Inhibition der GBSSI Aktivität geeignete Nukleinsäuresequenzen enthält (3 Einfachkonstrukte).

Die zur Herstellung der erfindungsgemäßen Pflanzenzellen bzw. Pflanzen geeigneten Nukleinsäuremoleküle können beispielsweise in einem Vektor, Plasmid oder in linear vorliegenden Nukleinsäuremolekülen enthalten sein.

Liegen die zur Herstellung von erfindungsgemäßen Pflanzenzellen bzw. Pflanzen zu verwendenden Konstrukte in zwei oder drei voneinander getrennten Nukleinsäuremolekülen vor, so können sie entweder zeitgleich ("Cotransformation") oder auch nacheinander, d.h. zeitlich aufeinander folgend ("Supertransformation") in das Genom der Pflanzenzelle oder Pflanze eingeführt werden. Die voneinander getrennten Nukleinsäuremoleküle können auch in verschiedene individuelle Pflanzenzellen oder Pflanzen einer Spezies eingeführt werden. Es können dadurch Pflanzenzellen oder Pflanzen erzeugt werden, bei welchen die Aktivität von entweder mindestens einem Protein mit der Aktivität einer Glucan-Wasser-Dikinase und/oder mindestens einem Protein mit der Aktivität einer Stärkesynthase II erhöht ist und/oder mindestens einem Protein mit der Aktivität einer GBSSI Aktivität so weit verringert ist, dass die von den Pflanzenzellen bzw. Pflanzen synthetisierte Stärke einen apparenten Amylosegehalt von weniger als 5 Gew.-% aufweisen. Erfindungsgemäße Pflanzen können dann durch anschließendes Kreuzen der Pflanzen hergestellt werden.

Weiterhin können auch Pflanzen erzeugt werden, bei welchen die Aktivität von mindestens einem Protein mit der (enzymatischen) Aktivität einer GBSSI so weit verringert ist, dass die von den Pflanzenzellen bzw. Pflanzen synthetisierte Stärke einen apparenten Amylosegehalt von weniger als 5 Gew.-% aufweisen, und die in einem weiteren Schritt durch Kreuzung mit Pflanzen, bei welchen die Aktivität von mindestens einem Protein mit der Aktivität einer Stärkesynthase II erhöht ist, zu erfindungsgemäßen Pflanzenzellen, bzw. Pflanzen führen.

Für den Fall, dass ein oder mehrere Nukleinsäuremoleküle, welche Nukleinsäuresequenzen enthalten, die geeignet sind, die Aktivität mindestens eines Proteins mit der Aktivität einer Glucan-Wasser-Dikinase bzw. einer Stärkesynthase II in den Pflanzenzellen zu erhöhen und die Aktivität einer GBSSI in den Pflanzenzellen so weit zu verringern, dass die von den Zellen synthetisierte Stärke einen apparenten Amylosegehalt von weniger als 5 Gew.-% aufweisen, in einem Verfahrensschritt/zeitgleich in das Genom der Pflanzenzellen eingeführt werden, können die erfindungsgemäßen Pflanzen direkt aus den Pflanzen ausgewählt werden, die aus der Transformation hervorgehen.

In einer weiteren Ausführungsform sind die erfindungsgemäßen Pflanzenzellen und die erfindungsgemäßen Pflanzen dadurch gekennzeichnet, dass mindestens ein fremdes Nukleinsäuremolekül ein Protein mit der Aktivität einer Stärkesynthase II und ein zweites fremdes Nukleinsäuremolekül ein Protein mit der Aktivität einer Glucan-Wasser-Dikinase codiert. In einer weiteren Ausführungsform sind die erfindungsgemäßen Pflanzenzellen und die erfindungsgemäßen Pflanzen dadurch gekennzeichnet, dass ein erstes fremdes Nukleinsäuremolekül ein Protein mit der Aktivität einer Glucan-Wasser-Dikinase codiert und ein zweites fremdes Nukleinsäuremolekül ein Protein mit der Aktivität einer Stärkesynthase II codiert.

Für die Einführung von DNA in eine pflanzliche Wirtszelle steht eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten. Die Verwendung der Agrobakterien-vermittelten Transformation von Pflanzenzellen ist intensiv untersucht und u.a. in EP 120516; Hoekema (In: The Binary Plant Vector System, Offsetdrukkerij Kanters B.V. Alblasserdam (1985), Chapter V); Fraley et al., Crit. Rev. Plant Sci. 4: 1-46) und bei An et al. (1985, EMBO J. 4: 277-287) beschrieben worden.

Auch die Transformation monokotyler Pflanzen mittels auf *Agrobacterium* Transformation basierender Vektoren wurde beschrieben (Chan et al. 1993, Plant Mol. Biol. 22: 491-506; Hiei et al., 1994, Plant J. 6, 271-282; Deng et al, 1990, Science in China 33: 28-34; Wilmink et al., 1992, Plant Cell Reports 11: 76-80; May et al., 1995, Bio/Technology 13: 486-492; Conner und Domisse, 1992, Int. J. Plant Sci. 153: 550-555; Ritchie et al, 1993, Transgenic Res. 2: 252-265). Alternative Methoden zur Transformation von monokotylen Pflanzen sind die Transformation mittels des biolistischen Ansatzes (Wan und Lemaux, 1994, Plant Physiol. 104 : 37-48; Vasil et al., 1993, Bio/Technology 11 : 1553-1558; Ritala et al., 1994, Plant Mol. Biol. 24: 317-325; Spencer et al., 1990, Theor. Appl. Genet. 79: 625-631), die Protoplastentransformation, die Elektroporation von partiell permeabilisierten Zellen oder die Einbringung von DNA mittels Glasfasern. Insbesondere die Transformation von Mais wird in der Literatur mehrfach beschrieben (vgl. z. B. WO95/06128, EP0513849, EP0465875, EP0292435; Fromm et al., 1990, Biotechnology 8: 833-844; Gordon-Kamm et al., 1990, Plant Cell 2: 603-618; Koziel et al., 1993, Biotechnology 11: 194-200; Moroc et al., 1990, Theor. Appl. Genet. 80: 721-726). Auch die erfolgreiche Transformation anderer Getreidearten wurde bereits beschrieben, z.B. für Gerste (Wan und Lemaux, s.o.; Ritala et al., s.o.; Krens et al., 1982, Nature 296: 72-74) und für Weizen (Nehra et al., 1994, Plant J. 5: 285-297; Becker et al., 1994, Plant Journal 5: 299-307). Alle vorstehenden Methoden sind im Rahmen der vorliegenden Erfindung geeignet.

Pflanzenzellen und Pflanzen, deren Stärke einen Amylosegehalt von weniger als 5 Gew.-% aufweist und die durch Einführung eines Genes, kodierend ein Protein mit der Aktivität einer Glucan-Wasser-Dikinase und/oder eines Genes, kodierend ein Protein mit der Aktivität einer Stärkesynthase II genetisch modifiziert sind, lassen sich von Wildtyp-Pflanzenzellen bzw. Wildtyp-Pflanzen unter anderem dadurch unterscheiden, dass sie mindestens ein fremdes Nukleinsäuremolekül aufweisen, das natürlicherweise in Wildtyp-Pflanzenzellen bzw. Wildtyp-Pflanzen nicht vorkommt oder dadurch, dass ein solches Molekül an einem Ort im Genom der erfindungsgemäßen Pflanzenzelle oder im Genom der erfindungsgemäßen Pflanze integriert vorliegt, an dem es bei Wildtyp-Pflanzenzellen bzw. Wildtyp-Pflanzen nicht vorkommt, d.h. in einer anderen genomischen Umgebung. Ferner lassen sich derartige erfindungsgemäße Pflanzenzellen und erfindungsgemäße Pflanzen von Wildtyp-Pflanzenzellen bzw. Wildtyp-Pflanzen dadurch unterscheiden, dass sie mindestens eine Kopie des fremden Nukleinsäuremoleküls stabil integriert in ihr Genom enthalten, gegebenenfalls zusätzlich zu natürlicherweise in den Wildtyp-Pflanzenzellen bzw. Wildtyp-Pflanzen vorkommenden Kopien eines solchen Moleküls. Handelt es sich bei dem (den) in die erfindungsgemäßen Pflanzenzellen oder erfindungsgemäßen Pflanzen eingeführten fremden Nukleinsäuremolekül(en) um zusätzliche Kopien zu bereits natürlicherweise in den Wildtyp-Pflanzenzellen bzw. Wildtyp-Pflanzen vorkommenden Molekülen, so lassen sich die erfindungsgemäßen Pflanzenzellen und die erfindungsgemäßen Pflanzen von Wildtyp-Pflanzenzellen bzw. Wildtyp-Pflanzen insbesondere dadurch unterscheiden, dass diese zusätzliche(n) Kopie(n) an Orten im Genom lokalisiert ist (sind), an denen sie bei Wildtyp-Pflanzenzellen bzw. Wildtyp-Pflanzen nicht vorkommt (vorkommen). Dies lässt sich beispielsweise mit Hilfe einer Southern Blot-Analyse nachprüfen.

Weiterhin lassen sich die erfindungsgemäßen Pflanzenzellen und die erfindungsgemäßen Pflanzen von Wildtyp-Pflanzenzellen bzw. Wildtyp-Pflanzen vorzugsweise durch mindestens eines der folgenden Merkmale unterscheiden: Ist ein eingeführtes fremdes Nukleinsäuremolekül heterolog in Bezug auf die Pflanzenzelle oder Pflanze, so weisen die erfindungsgemäßen Pflanzenzellen oder erfindungsgemäßen Pflanzen Transkripte der eingeführten Nukleinsäuremoleküle auf. Diese lassen sich z. B. durch Northern-Blot-Analyse oder durch RT-PCR (Reverse Transcription Polymerase Chain Reaction) nachweisen.

Erfindungsgemäße Pflanzenzellen und erfindungsgemäße Pflanzen, die ein Antisense- und/oder ein RNAi-Transkript exprimieren, können z.B. mit Hilfe von spezifischen Nucleinsäure-Sonden, die komplementär zur der für das Protein codierenden (natürlich in der Pflanzenzelle vorkommenden) RNA sind, nachgewiesen werden. Vorzugsweise enthalten die erfindungsgemäßen Pflanzenzellen und die erfindungsgemäßen Pflanzen ein Protein, das durch ein eingeführtes Nucleinsäuremolekül codiert wird. Dies kann z. B. durch immunologische Methoden, insbesondere durch eine Western-Blot-Analyse nachgewiesen werden.

Vorzugsweise enthalten die erfindungsgemäßen Pflanzenzellen und die erfindungsgemäßen Pflanzen ein Protein, das durch ein eingeführtes Nukleinsäuremolekül codiert wird. Dies kann z. B. durch immunologische Methoden, insbesondere durch eine Western-Blot-Analyse nachgewiesen werden.

Ist ein eingeführtes fremdes Nukleinsäuremolekül homolog in Bezug auf die Pflanzenzelle oder Pflanze, können die erfindungsgemäßen Pflanzenzellen und die erfindungsgemäßen Pflanzen von Wildtyp-Pflanzenzellen bzw. Wildtyp-Pflanzen beispielsweise aufgrund der zusätzlichen Expression der eingeführten fremden Nukleinsäuremoleküle unterschieden werden. Die erfindungsgemäßen Pflanzenzellen und die erfindungsgemäßen Pflanzen enthalten vorzugsweise Transkripte der fremden Nukleinsäuremoleküle. Dies kann z. B. durch Northern-Blot-Analyse oder mit Hilfe der so genannten quantitativen PCR nachgewiesen werden.

Ein weiterer Gegenstand der vorliegenden Erfindung betrifft genetisch modifizierte monokotyle Pflanzenzellen oder genetisch modifizierte monokotyle Pflanzen, dadurch charakterisiert, dass sie eine modifizierte Stärke synthetisieren im Vergleich zu Stärke, isoliert aus entsprechenden nicht genetisch modifizierten Wildtyp-Pflanzenzellen bzw. isoliert aus entsprechenden nicht genetisch modifizierten Wildtyp-Pflanzen.

Ferner sind Gegenstand der Erfindung genetisch modifizierte monokotyle Pflanzen, die erfindungsgemäße Pflanzenzellen enthalten. Derartige Pflanzen können durch Regeneration aus erfindungsgemäßen Pflanzenzellen erzeugt werden.

Bei den erfindungsgemäßen Pflanzen kann es sich prinzipiell um jede monokotyle Pflanze handeln. Bevorzugt handelt es sich um monokotyle Nutzpflanzen, d.h. Pflanzen, die vom Menschen kultiviert werden für Zwecke der Ernährung oder für technische, insbesondere industrielle Zwecke.

In einer weiteren Ausführungsform handelt es sich bei den erfindungsgemäßen Pflanzen um stärkespeichernde monokotyle Pflanzen bzw. es stammen die erfindungsgemäßen Pflanzenzellen aus einer stärkespeichernden Pflanze.

Der Begriff "stärkespeichernde Pflanze" meint im Zusammenhang mit der vorliegenden Erfindung alle Pflanzen mit Pflanzenteilen, die eine Speicherstärke enthalten wie z.B., Mais, Reis, Weizen, Roggen, Hafer, Gerste, Sago, Taro, Hirse.

In einer bevorzugten Ausführungsform betrifft die vorliegende Erfindung monokotyle Pflanzen der (systematischen) Familie Poaceae. Besonders bevorzugt handelt es sich dabei um Reis-, Mais- oder Weizenpflanzen. Ganz besonders bevorzugt handelt es sich um Reispflanzen.

Der Begriff "Weizenpflanze" meint im Zusammenhang mit der vorliegenden Erfindung Pflanzenspezies der Gattung *Triticum* oder Pflanzen, die aus Kreuzungen mit Pflanzen der Gattung *Triticum* hervorgegangen sind, besonders in der Agrarwirtschaft zu kommerziellen Zwecken angebaute Pflanzenspezies der Gattung *Triticum* bzw. Pflanzen, die aus Kreuzungen mit Pflanzen der Gattung *Triticum* hervorgegangen sind, insbesondere bevorzugt ist *Triticum aestivum.*

Der Begriff "Maispflanze" meint im Zusammenhang mit der vorliegenden Erfindung Pflanzenspezies der Gattung Zea, besonders in der Agrarwirtschaft zu kommerziellen Zwecken angebaute Pflanzenspezies der Gattung Zea, besonders bevorzugt Zea *mays.*

Der Begriff "Reispflanze" meint im Zusammenhang mit der vorliegenden Erfindung Pflanzenspezies der Gattung *Oryza,* besonders in der Agrarwirtschaft zu kommerziellen Zwecken angebaute Pflanzenspezies der Gattung *Oryza,* besonders bevorzugt *Oryza sativa.*

Die vorliegende Erfindung betrifft auch Vermehrungsmaterial monokotyler Pflanzen, enthaltend genetisch modifizierte Pflanzenzellen.

Der Begriff "Vermehrungsmaterial" umfasst dabei jene Bestandteile der Pflanze, die geeignet sind zur Erzeugung von Nachkommen auf vegetativem oder sexuellem Weg. Für die vegetative Vermehrung eignen sich beispielsweise Stecklinge, Calluskulturen, Rhizome oder Knollen. Anderes Vermehrungsmaterial umfasst beispielsweise Früchte, Samen, Sämlinge, Protoplasten, Zellkulturen, etc.

In einer weiteren Ausführungsform betrifft die vorliegende Erfindung erntebare Pflanzenteile erfindungsgemäßer Pflanzen, wie Früchte, Speicherwurzeln, Wurzeln, Blüten, Knospen, Sprosse oder Stämme, vorzugsweise Samen oder Körner, wobei diese erntebaren Teile erfindungsgemäße Pflanzenzellen enthalten.

In einer weiteren Ausführungsform zeichnen sich die erfindungsgemäßen genetisch modifizierten monokotylen Pflanzenzellen dadurch aus, dass sie eine (waxy-) Stärke mit erhöhtem Heisswasser Quellvermögen sowie einem Amylosegehalt von weniger als 5 Gew.-% synthetisieren.

In einer bevorzugten Ausführungsform zeichnet sich die genetisch modifizierte monokotyle Pflanzenzelle dadurch aus, dass sie eine waxy-Stärke mit einem erhöhten Heißwasser Quellvermögen zwischen 60 bis 100 g/g aufweist. Besonders bevorzugt ist dabei ein Heißwasser Quellvermögen zwischen 70 und 95 g/g, ganz besonders bevorzugt zwischen 80 und 95 g/g und außerordentlich bevorzugt zwischen 80 und 90 g/g.

Ein weiterer Gegenstand der vorliegenden Erfindung betrifft ein Verfahren zur Herstellung einer genetisch modifizierten monokotylen Pflanze, worin

a) eine Pflanzenzelle genetisch modifiziert wird, wobei die genetische Modifikation die folgenden Schritte i bis iii umfasst

i) Einführung einer genetischen Modifikation in die Pflanzenzelle, wobei die genetische Modifikation zur Erhöhung der Aktivität eines Proteins mit der Aktivität einer Stärkesynthase II im Vergleich zu entsprechenden nicht genetisch modifizierten Wildtyp-Pflanzenzellen führt,

ii) Einführung einer genetischen Modifikation in die Pflanzenzelle, wobei die genetische Modifikation zur Erhöhung der Aktivität eines Proteins mit der Aktivität einer Glucan-Wasser-Dikinase im Vergleich zu entsprechenden nicht genetisch modifizierten Wildtyp-Pflanzenzellen führt

iii) Einführung einer genetischen Modifikation in die Pflanzenzelle, wobei die genetische Modifikation zur Reduktion der Aktivität eines Proteins mit der Aktivität einer GBSSI im Vergleich zu entsprechenden nicht genetisch modifizierten Wildtyp-Pflanzenzellen führt,

wobei die Schritte i bis iii in beliebiger Reihenfolge, einzeln oder beliebige Kombinationen der Schritte i bis iii gleichzeitig durchgeführt werden können

b) aus Pflanzenzellen von Schritt a) eine Pflanze regeneriert wird;

c) gegebenenfalls weitere Pflanzen mit Hilfe der Pflanzen nach Schritt b) erzeugt werden,

wobei gegebenenfalls aus Pflanzen gemäß den Schritten b) oder c) Pflanzenzellen isoliert werden und die Verfahrensschritte a) bis c) solange wiederholt werden, bis eine Pflanze erzeugt wurde, die eine erhöhte Aktivität eines Proteins mit der Aktivität einer Stärkesynthase II im Vergleich zu entsprechenden nicht genetisch modifizierten Wildtyp-Pflanzenzellen aufweist und eine erhöhte Aktivität eines Proteins mit der Aktivität einer Glucan-Wasser-Dikinase im Vergleich zu entsprechenden nicht genetisch modifizierten Wildtyp-Pflanzenzellen und eine reduzierte Aktivität eines Proteins mit der Aktivität einer GBSSI im Vergleich zu entsprechenden nicht genetisch modifizierten Wildtyp-Pflanzenzellen aufweist.

Weiterhin betrifft die vorliegende Erfindung auch ein Verfahren zur Herstellung einer genetisch modifizierten Pflanze, worin eine Pflanzenzelle, deren Stärke einen Amylosegehalt von weniger als 5 Gew.-% aufweist, genetisch modifiziert wird, wobei die genetische Modifikation die folgenden Schritte a) und b) in beliebiger Reihenfolge, einzeln oder gleichzeitig umfasst:

a) Einführung einer genetischen Modifikation in die Pflanzenzelle, wobei die genetische Modifikation zur Erhöhung der Aktivität eines Proteins mit der Aktivität einer Stärkesynthase II, im Vergleich zu entsprechenden nicht genetisch modifizierten Wildtyp-Pflanzenzellen führt,

b) Einführung einer genetischen Modifikation in die Pflanzenzelle, wobei die genetische Modifikation zur Erhöhung der Aktivität eines Proteins mit der Aktivität einer Glucan-Wasser-Dikinase, im Vergleich zu entsprechenden nicht genetisch modifizierten Wildtyp-Pflanzenzellen führt und

c) aus Pflanzenzellen von Schritt a) und b) eine Pflanze regeneriert wird;

d) gegebenenfalls weitere Pflanzen mit Hilfe der Pflanzen aus den Schritten a) und b) erzeugt werden,

wobei gegebenenfalls aus Pflanzen gemäß Schritt a) oder b) Pflanzenzellen isoliert werden und die Verfahrensschritte a) bis c) solange wiederholt werden, bis eine Pflanze erzeugt wurde, die ein fremdes Nukleinsäuremolekül, kodierend ein Protein mit der Aktivität einer Stärkesynthase 11 und ein fremdes Nukleinsäuremolekül, kodierend ein Protein mit der Aktivität einer Glucan-Wasser-Dikinase aufweist.

Ein bevorzugter Gegenstand der vorliegenden Erfindung betrifft Verfahren zur Herstellung einer monokotylen Pflanze, worin

a) eine Pflanzenzelle, genetisch modifiziert wird, wobei die genetische Modifikation die folgenden Schritte i bis iii in beliebiger Reihenfolge umfasst oder beliebige Kombinationen der folgenden Schritte i bis iii einzeln oder gleichzeitig durchgeführt werden

i) Einführung einer genetischen Modifikation in die Pflanzenzelle, wobei die genetische Modifikation zur Erhöhung der Aktivität eines Proteins mit der Aktivität einer Stärkesynthase II, im Vergleich zu entsprechenden nicht genetisch modifizierten Wildtyp-Pflanzenzellen führt

ii) Einführung einer genetischen Modifikation in die Pflanzenzelle, wobei die genetische Modifikation zur Erhöhung der Aktivität eines Proteins mit der Aktivität einer Glucan-Wasser-Dikinase, im Vergleich zu entsprechenden nicht genetisch modifizierten Wildtyp-Pflanzenzellen führt

iii) Einführung einer genetischen Modifikation in die Pflanzenzelle, wobei die genetische Modifikation zur Reduktion der Aktivität eines Proteins mit der Aktivität einer GBSSI im Vergleich zu entsprechenden nicht genetisch modifizierten Wildtyp-Pflanzenzellen führt

b) aus Pflanzenzellen enthaltend die genetische Modifikation gemäß den Schritten

i) a) i

ii) a) ii

iii) a) iii

iv) a) i und a) ii,

v) a) i und a) iii,

vi) a) ii und a) iii, oder

vii) a) i und a) ii und a) iii

eine Pflanze regeneriert wird

c) in Pflanzenzellen von Pflanzen gemäß Schritt

i) b) i eine genetische Modifikation gemäß Schritt a) ii,

ii) b) i eine genetische Modifikation gemäß Schritt a) iii,

iii) b) i eine genetische Modifikation gemäß Schritt a) ii und gleichzeitig eine genetische Modifikation gemäß Schritt a) iii,

iv) b) ii eine genetische Modifikation gemäß Schritt a) i,

v) b) ii eine genetische Modifikation gemäß Schritt a) iii,

vi) b) ii eine genetische Modifikation gemäß Schritt a) i und gleichzeitig eine genetische Modifikation gemäß Schritt a) iii,

vii) b) iii eine genetische Modifikation gemäß Schritt a) i,

viii) b) iii eine genetische Modifikation gemäß Schritt a) ii,

ix) b) iii eine genetische Modifikation gemäß Schritt a) i und gleichzeitig eine genetische Modifikation gemäß Schritt a) ii,

x) b) iv eine genetische Modifikation gemäß Schritt a) iii,

xi) b) v eine genetische Modifikation gemäß Schritt a) ii, oder

xii) b) vi eine genetische Modifikation gemäß Schritt a) i

eingeführt und eine Pflanze regeneriert wird

d) in Pflanzenzellen von Pflanzen gemäß Schritt

i) c) i eine genetische Modifikation gemäß Schritt a) iii,

ii) c) ii eine genetische Modifikation gemäß Schritt a) ii,

iii) c) iv eine genetische Modifikation gemäß Schritt a) iii,

iv) c) v eine genetische Modifikation gemäß Schritt a) ii,

v) c) vii eine genetische Modifikation gemäß Schritt a) ii,

vi) c) vii eine genetische Modifikation gemäß Schritt a) i, oder

vii) c) ix eine genetische Modifikation gemäß Schritt a) ii

eingeführt und eine Pflanze regeneriert wird

gegebenenfalls weitere Pflanzen mit Hilfe der Pflanzen nach einem der Schritte b) vii c) iii, c) vi, c) x, c) xi, c) xii oder nach einem der Schritte d) i bis d) vii erzeugt werden.

Für die laut Schritt a) in die Pflanzenzelle eingeführten genetischen Modifikationen gilt, dass es sich grundsätzlich um jede Art von Modifikation handeln kann, die zur Erhöhung der Aktivität eines Proteins mit der Aktivität einer Stärkesynthase II und/oder die zur Erhöhung der Aktivität eines Proteins mit der Aktivität einer Glucan-Wasser-Dikinase und/oder die zur Reduktion der Aktivität eines Proteins mit der Aktivität einer GBSSI führt.

Die Regeneration der Pflanzen gemäß der Schritte b) bis e) der erfindungsgemäßen Verfahren kann nach dem Fachmann bekannten Methoden erfolgen (z.B. beschrieben in "Plant Cell Culture Protocols", 1999, ed. by R.D. Hall, Humana Press, ISBN 0-89603-549-2).

Die Erzeugung weiterer Pflanzen der erfindungsgemäßen Verfahren kann z.B. erfolgen durch vegetative Vermehrung (beispielsweise über Stecklinge, Knollen oder über Kalluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt. Die Auswahl erfolgt dabei bevorzugt in der Weise, dass die weiteren Pflanzen (die je nach Verfahren gemäß Schritt c) oder Schritt d) oder Schritt e) erzeugt werden) die in den vorangegangenen Schritten eingeführten Modifikationen aufweisen.

Nachfolgend sind die Parameter für die Auswahl der erfindungsgemäßen Pflanzenzellen bzw. Pflanzen, die durch Kreuzung bzw. Transformation hergestellt werden, aufgeführt:

Im Falle der ausschließlichen Erhöhung mindestens eines Proteins mit der Aktivität einer Glucan-Wasser-Dikinase sind Pflanzen bzw. Pflanzenzellen geeignet, die einen Phosphatgehalt in C6 Position der Stärke von mindestens 2,5 nmol pro mg Stärke aufweisen. Im Falle der ausschließlichen Erhöhung mindestens eines Proteins mit der Aktivität einer Stärkesynthase II sind Pflanzen bzw. Pflanzenzellen geeignet, die eine SSII Aktivität aufweisen, die gegenüber der SSII Aktivität in den Pflanzenzellen bzw. Pflanzen, die zur Einführung des/r entsprechenden Nukleinsäuremoleküle bzw. zur Kreuzung verwendet werden, um mindestens den Faktor zwei erhöht ist.

Im Falle der Erhöhung mindestens eines Proteins mit der Aktivität einer Glucan-Wasser-Dikinase und mindestens eines Proteins mit der Aktivität einer Stärkesynthase II sind Pflanzen bzw. Pflanzenzellen geeignet, die einen Phosphatgehalt in C6 Position der Stärke von mindestens 2,5 nmol pro mg Stärke aufweisen und zusätzlich eine SSII Aktivität aufweisen, die gegenüber den Pflanzenzellen bzw. Pflanzen, die zur Einführung des/r entsprechenden Nukleinsäuremoleküle bzw. zur Kreuzung verwendet werden, um mindestens den Faktor zwei erhöht ist.

Im Falle der Verringerung der GBSSI Aktivität bzw. der Verwendung von waxy Mutanten sind Pflanzen geeignet, die einen apparenten Amylosegehalt von weniger als 5 Gew.-% aufweisen, wenn die Mutation homozygot vorliegt.

Auch die Höhe des Stärkephosphatgehaltes in C 6-Position ist als Selektionskriterium geeignet. Vorzugsweise werden Pflanzen selektiert, die die genetische Modifikation gemäß Schritt a) und b) enthalten und deren Stärkephosphatgehalt mindestens 2,5 nmol C6P/mg Stärke beträgt sowie deren Stärke einen apparenten Amylosegehalt von weniger als 5 Gew.-% aufweist.

In erfindungsgemäßen Verfahren zur Herstellung von genetisch modifizierten Pflanzen können die genetischen Modifikationen zur Erzeugung der erfindungsgemäßen genetisch modifizierten Pflanzenzellen gleichzeitig oder in aufeinander folgenden Schritten erfolgen. Es ist dabei unerheblich, ob für aufeinander folgende genetische Modifikationen, die zu einer erhöhten Aktivität eines Proteins mit der Aktivität einer Stärkesynthase II führt, die gleiche Methode verwendet wird, wie für die genetische Modifikation, die zu einer erhöhten Aktivität eines Proteins mit der Aktivität einer Glucan-Wasser-Dikinase führt bzw. wie für die genetische Modifikation, die zu einer reduzierten Aktivität eines Proteins mit der Aktivität einer GBSSI führt.

Zur Auswahl der erfindungsgemäßen Pflanzen bzw. derjenigen Pflanzen, die für weitere Modifikationen verwendet werden, können verschiedene Selektionskriterien gewählt werden.

In einer weiteren Ausführungsform des erfindungsgemäßen Verfahrens zur Herstellung einer genetisch modifizierten Pflanze folgt auf Schritt c) ein Verfahrensschritt c)-1, worin Pflanzen selektiert werden, deren Stärke einen apparenten Amylosegehalt von weniger als 5 Gew.-% sowie eine erhöhte Aktivität eines Proteins mit der Aktivität einer Stärkesynthase II gemäß Schritt a)i) aufweisen und/oder die eine erhöhte Aktivität eines Proteins mit der Aktivität einer Glucan-Wasser-Dikinase gemäß Schritt a)ii) aufweisen. Die selektierten Pflanzen werden dann für die weiteren Verfahrensschritte verwendet.

In einer weiteren Ausführungsform des erfindungsgemäßen Verfahrens zur Herstellung einer erfindungsgemäßen genetisch modifizierten Pflanze, codiert mindestens ein fremdes Nukleinsäuremolekül ein Protein mit der Aktivität einer Glucan-Wasser-Dikinase aus Kartoffel, Weizen, Reis, Mais, Sojabohne, Citrus, *Curcuma* oder *Arabidopsis.* Bevorzugt codiert mindestens ein fremdes Nukleinsäuremolekül ein Protein mit der Aktivität einer Glucan-Wasser-Dikinase aus Kartoffel und insbesondere bevorzugt ein Protein, das die unter SEQ ID NO 2 dargestellte Aminosäuresequenz aufweist oder von der unter SEQ ID NO 1 dargestellten Nukleinsäuresequenz codiert wird. Referenzen für Nukleinsäuresequenzen kodierend Proteine mit der Aktivität einer Glucan-Wasser-Dikinase aus den genannten Pflanzen sind bereits weiter oben angegeben.

In einer weiteren Ausführungsform des erfindungsgemäßen Verfahrens zur Herstellung einer erfindungsgemäßen genetisch modifizierten Pflanze, codiert mindestens ein fremdes Nukleinsäuremolekül ein Protein mit der Aktivität einer Stärkesynthase II aus Weizen, Gerste, *Aegilops,* Reis, Mais, Maniok, Bohne, Kartoffel, Erbse, Süßkartoffel, *Arabidopsis,* Taro, *Ostreococcus* oder *Chlamydomonas.* Bevorzugt codiert mindestens ein fremdes Nukleinsäuremolekül ein Protein mit der Aktivität einer Stärkesynthase II aus Weizen, insbesondere Seq ID No 3. Referenzen für die genannten Nukleinsäuresequenzen kodierend Proteine mit der Aktivität einer Stärkesynthase II aus den genannten Pflanzen sind bereits weiter oben angegeben.

Wie oben bereits für zur genetischen Modifikation in eine Pflanzenzelle oder Pflanze eingebrachte fremde Nukleinsäuremoleküle beschrieben, kann es sich in Schritt a) des erfindungsgemäßen Verfahrens zur Herstellung einer genetisch modifizierten Pflanze, deren Stärke einen Amylosegehalt von weniger als 5 Gew.-% aufweist, um ein einzelnes Nukleinsäuremolekül oder um mehrere Nukleinsäuremoleküle handeln. So können die fremden Nukleinsäuremoleküle, kodierend ein Protein mit der Aktivität einer Stärkesynthase II bzw. kodierend ein Protein mit der Aktivität einer Glucan-Wasser-Dikinase zusammen auf einem einzigen Nukleinsäuremolekül vorliegen oder sie können auf getrennten Nukleinsäuremolekülen vorliegen. Liegen die Nukleinsäuremoleküle kodierend ein Protein mit der Aktivität einer Stärkesynthase II und kodierend ein Protein mit der Aktivität einer Glucan-Wasser-Dikinase auf mehreren Nukleinsäuremolekülen vor, so können diese Nukleinsäuremoleküle gleichzeitig oder in aufeinander folgenden Schritten in eine Pflanzenzelle eingeführt werden.

In einer weiteren Ausführungsform des erfindungsgemäßen Verfahrens zur Herstellung einer erfindungsgemäßen genetisch modifizierten Pflanze, codiert mindestens ein fremdes Nukleinsäuremolekül ein Protein mit der Aktivität einer GBSS I aus einer monokotylen Pflanze, bevorzugt aus Reis, Weizen, Gerste, Mais, Aegilops, Sorghum oder Hafer.

Referenzen für die genannten Nukleinsäuresequenzen kodierend Proteine mit der Aktivität einer GBSS1 aus den genannten Pflanzen sind bereits weiter oben angegeben.

Bevorzugt codiert mindestens ein fremdes Nukleinsäuremolekül ein Protein mit der Aktivität einer GBSS1 aus Reis und insbesondere bevorzugt ein Protein, das von der unter SEQ ID NO 7 dargestellten Nukleinsäuresequenz oder von der unter SEQ ID NO 8 dargestellten Aminosäuresequenz codiert wird.

In einer weiteren bevorzugten Ausführungsform codiert mindestens ein fremdes Nukleinsäuremolekül ein Protein mit der Aktivität einer GBSS1 aus Weizen und insbesondere bevorzugt ein Protein, das von der unter SEQ ID NO 9 oder von der unter SEQ ID NO 10 dargestellten Aminosäuresequenz codiert wird.

In einer weiteren bevorzugten Ausführungsform codiert mindestens ein fremdes Nukleinsäuremolekül ein Protein mit der Aktivität einer GBSS1 aus Mais und insbesondere bevorzugt ein Protein, das von der unter SEQ ID NO 11 dargestellten Nukleinsäuresequenz oder von der unter SEQ ID NO 12 dargestellten Aminosäuresequenz codiert wird. Das fremde Nukleinsäuremolekül bewirkt dabei die Inhibierung der Aktivität einer GBSS I und damit die Synthese einer Stärke mit einem Amylosegehalt von weniger als 5 Gew.-%. Die weiter oben gemachten Angaben zur Verwendung der entsprechenden Nukleinsäuren für die Herstellung erfindungsgemäßer Pflanzenzellen oder Pflanzen sind hier entsprechend anzuwenden.

Bei dem bzw. den zur genetischen Modifikation verwendeten fremden Nukleinsäuremolekül(en) kann es sich um ein zusammengefügtes oder mehrere getrennte Nukleinsäurekonstrukte handeln, insbesondere um so genannte Einfach-, Zweifach-, oder Dreifachkonstrukte. So kann das fremde Nukleinsäuremolekül beispielsweise ein so genanntes "Dreifachkonstrukt" sein, worunter man einen einzigen Vektor zur Pflanzentransformation versteht, der sowohl die genetische Information zur Inhibierung der Expression eines endogenen GBSSI-Gens als auch zur Überexpression eines oder mehrerer SSII-Gene als auch zur Überexpression eines oder mehrerer GWD-Gene enthält.

Grundsätzlich ist bei der Konstruktion der fremden Nukleinsäuremoleküle zur Inhibition der GBSSI Aktivität die Verwendung von Antisense-, Cosuppressions-, Ribozym- und doppelsträngigen RNA-Konstrukten sowie Sense-Konstrukten geeignet, die zu einer Verringerung der Expression endogener Gene führt, die für GBSSI kodieren sowie die zu einer gleichzeitigen Erhöhung der Aktivität der Proteine mit den Aktivitäten einer SSII bzw. einer GWD führt.

Die fremden Nukleinsäuremoleküle können hierbei zeitgleich ("Cotransformation") oder auch nacheinander, d.h. zeitlich aufeinanderfolgend ("Supertransformation") in das Genom der Pflanzenzelle eingeführt werden.

Die fremden Nukleinsäuremoleküle können auch in verschiedene individuelle Pflanzen einer Spezies eingeführt werden. Es können dabei Pflanzen erzeugt werden, bei welchen die Aktivität eines Proteins mit der Aktivität einer GBSSI reduziert ist und/oder die Aktivität eines Proteins mit der Aktivität einer SSII bzw. GWD erhöht ist. Durch anschließendes Kreuzen können dann Pflanzen erzeugt werden, bei welchen die Aktivität eines Proteins mit der Aktivität einer GBSSIreduziert und die Aktivität eines Proteins mit der Aktivität einer SSII sowie einer GWD erhöht ist.

Unter dem Begriff "Identität" soll im Zusammenhang mit der vorliegenden Erfindung die Anzahl der übereinstimmenden Aminosäuren/Nukleotide (Identität) mit anderen Proteinen/Nukleinsäuren, ausgedrückt in Prozent verstanden werden.
Bevorzugt wird die Identität betreffend ein Protein mit der Aktivität einer Stärkesynthase II durch Vergleiche der unter SEQ ID NO 4 bzw. SEQ ID NO 6 angegebenen Aminosäuresequenzen bzw. die Identität betreffend ein Nukleinsäuremolekül kodierend ein Protein mit der Aktivität einer Stärkesynthase II durch Vergleiche der unter SEQ ID NO 3 bzw. SEQ ID NO 5 angegebenen Nukleinsäuresequenzen und die Identität betreffend ein Protein mit der Aktivität einer Glucan-Wasser-Dikinase durch Vergleiche der unter SEQ ID NO 2 angegebenen Aminosäuresequenz bzw. die Identität betreffend ein Nukleinsäuremolekül kodierend ein Protein mit der Aktivität einer Glucan-Wasser-Dikinase durch Vergleiche der unter SEQ ID NO 1 angegebenen Nukleinsäuresequenz und die Identität betreffend ein Nukleinsäuremolekül kodierend ein Protein mit der Aktivität einer GBSSI durch Vergleiche der unter SEQ ID NO 7 oder SEQ ID NO 9 oder SEQ ID NO 11 angegebenen Nukleinsäuresequenzen bzw. der unter SEQ ID NO 8 oder SEQ ID NO 10 oder SEQ ID NO 12 angegebenen Aminosäuresequenzen zu anderen Proteinen/Nukleinsäuren mit Hilfe von Computerprogrammen ermittelt.

Weisen Sequenzen, die miteinander verglichen werden, unterschiedliche Längen auf, ist die Identität so zu ermitteln, dass die Anzahl an Aminosäuren/Nukleotiden, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., Nucleic Acids Research 22 (1994), 4673-4680) ermittelt. ClustalW wird öffentlich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK) heruntergeladen werden.

Vorzugsweise wird das ClustalW Computerprogramm der Version 1.8 benutzt, um die Identität zwischen im Rahmen der vorliegenden Erfindung beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Vorzugsweise wird das ClustalW Computerprogramm der Version 1.8 benutzt, um die Identität zwischen z.B. der Nukleotidsequenz der im Rahmen der vorliegenden Erfindung beschriebenen Nukleinsäuremoleküle und der Nukleotidsequenz von anderen Nukleinsäuremolekülen zu bestimmen. Dabei sind folgende Parameter einzustellen: KTUPLE=2, TOPDIAGS=4, PAIRGAP=5, DNAMATRIX:IUB, GAPOPEN=10, GAPEXT=5, MAXDIV=40, TRANSITIONS: unweighted.

Identität bedeutet ferner, dass funktionelle und/oder strukturelle Äquivalenz zwischen den betreffenden Nukleinsäuremolekülen oder den durch sie codierten Proteinen, besteht. Bei den Nukleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Spezies oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten. Eine spezielle Form von Derivaten stellen z.B. Nukleinsäuremoleküle dar, die auf Grund der Degeneration des genetischen Codes von im Rahmen der vorliegenden Erfindung beschriebenen Nukleinsäuremolekülen abweichen.

Der Begriff "Hybridisierung" bedeutet im Rahmen der vorliegenden Erfindung eine Hybridisierung unter konventionellen Hybridisierungsbedingungen, vorzugsweise unter stringenten Bedingungen, wie sie beispielsweise in Sambrok et al., (Molecular Cloning, A Laboratory Manual, 3rd edition (2001) Cold Spring Harbour Laboratory Press, Cold Spring Harbour, NY; ISBN: 0879695773) beschrieben sind. Besonders bevorzugt bedeutet "hybridisieren" eine Hybridisierung unter den folgenden Bedingungen:

Hybridisierungspuffer:

2xSSC; 10xDenhardt-Lösung (Fikoll 400+PEG+BSA; Verhältnis 1:1:1); 0,1% SDS; 5 mM EDTA; 50 mM Na2HPO4; 250 $\mu$g/ml Heringssperma DNA; 50 $\mu$g/ml tRNA; oder 25 M Natriumphoshphatpuffer pH 7,2; 1 mM EDTA; 7% SDS

Hybridisierungstemperatur:

T=65 bis 68°C
Waschpuffer: 0,1xSSC; 0,1% SDS
Waschtemperatur: T=65 bis 68°C.

Nukleinsäuremoleküle, die mit den genannten Molekülen hybridisieren, können z.B. aus genomischen oder aus cDNA-Bibliotheken isoliert werden. Die Identifizierung und Isolierung derartiger Nukleinsäuremoleküle kann dabei unter Verwendung der genannten Nukleinsäuremoleküle oder Teile dieser Moleküle bzw. der reversen Komplemente dieser Moleküle erfolgen, z.B. mittels Hybridisierung nach Standardverfahren oder durch Amplifikation mittels PCR. Als Hybridisierungsprobe zur Isolierung einer Nukleinsäuresequenz, kodierend ein Protein mit der Aktivität einer Stärkesynthase II oder mit der Aktivität einer Glucan-Wasser-Dikinase oder mit der Aktivität einer GBSSI können z.B. Nukleinsäuremoleküle verwendet werden, die exakt die oder im Wesentlichen die unter SEQ ID NO 3 bzw SEQ ID NO 5 (Stärkesynthase II) oder unter SEQ ID NO 1 (Glucan-Wasser-Dikinase) oder unter SEQ ID NO 7, 9 oder 11 (GBSSI) angegebene Nukleotidsequenzen oder Teile dieser Sequenzen aufweisen.

Bei den als Hybridisierungsprobe verwendeten Fragmenten kann es sich auch um synthetische Fragmente oder Oligo-Nukleotide handeln, die mit Hilfe der gängigen Synthesetechniken hergestellt wurden und deren Sequenz im wesentlichen mit der eines im Rahmen der vorliegenden Erfindung beschriebenen Nukleinsäuremoleküls übereinstimmt. Hat man Gene identifiziert und isoliert, die mit den im Rahmen der vorliegenden Erfindung beschriebenen Nukleinsäuresequenzen hybridisieren, sollte eine Bestimmung der Sequenz und eine Analyse der Eigenschaften der von dieser Sequenz codierten Proteine erfolgen, um festzustellen, ob es sich um Proteine handelt, die die Aktivität einer Stärkesynthase II bzw. die Aktivität einer Glucan-Wasser-Dikinase bzw. die Aktivität einer GBSSI aufweisen.

Die mit den im Rahmen der vorliegenden Erfindung beschriebenen Nukleinsäuremolekülen hybridisierenden Moleküle umfassen insbesondere Fragmente, Derivate und allelische Varianten der genannten Nukleinsäuremoleküle. Der Begriff "Derivat" bedeutet im Zusammenhang mit der vorliegenden Erfindung, dass die Sequenzen dieser Moleküle sich von den Sequenzen der oben beschriebenen Nukleinsäuremoleküle an einer oder mehreren Positionen unterscheiden und einen hohen Grad an Identität zu diesen Sequenzen aufweisen. Die Abweichungen zu den oben beschriebenen Nukleinsäuremolekülen können dabei z.B. durch Deletion, Addition, Substitution, Insertion oder Rekombination entstanden sein.

Zur Expression von erfindungsgemäßen Nukleinsäuremolekülen, die ein Protein mit der Aktivität einer Stärkesynthase 11 und/oder ein Protein mit der Aktivität einer Glucan-Wasser-Dikinase und/oder ein Protein mit der Aktivität einer GBSSI kodieren, werden diese vorzugsweise mit regulatorischen DNA-Sequenzen verknüpft, die die Transkription in pflanzlichen Zellen gewährleisten. Hierzu zählen insbesondere Promotoren. Generell kommt für die Expression jeder in pflanzlichen Zellen aktive Promotor in Frage.

Der Promotor kann dabei so gewählt sein, dass die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. Sowohl in Bezug auf die Pflanze als auch in bezug auf das Nukleinsäuremolekül kann der Promotor homolog oder heterolog sein.

Geeignete Promotoren sind z.B. der Promotor der 35S RNA des Cauliflower Mosaic Virus und der Ubiquitin-Promotor aus Mais, der Ubiquitin-Promotor aus Reis (Liu et al., Plant Science 165, (2003), der Reis Actin Promoter (Zhang, et al., Plant Cell 3:1150-1160, 1991), der Cassava Vein Mosaic Virus (CVMV) Promotor (Verdaguer et. al., Plant Mol. Biol. 31: 1129-1139), der Mais Histon H3C4 Promotor (US 6,750,378) oder der *Cestrum* YLCV Promotor (Yellow Leaf Curling Virus; WO 01 73087; Stavolone et al., 2003, Plant Mol. Biol. 53, 703-713) für eine konstitutive Expression. Verwendbar ist auch ein Promotor, der eine Expression lediglich in photosynthetisch aktiven Geweben sicherstellt, z.B. der ST-LS1-Promotor (Stockhaus et al., Proc. Natl. Acad. Sci. USA 1987, 84: 7943-7947; Stockhaus et al., EMBO J. 1989, 8: 2445-2451) oder für eine endosperm-spezifische Expression der HMW-Promotor aus Weizen, der USP-Promotor aus *Vicia faba* (Fiedler et al., 1993, Plant Mol. Biol. 22: 669-679; Bäumlein et al., 1991, Mol. Gen. Genet. 225: 459-467), der Phaseolinpromotor aus Bohne, Promotoren von Zein-Genen aus Mais (Pedersen et al., 1982, Cell 29: 1015-1026; Quatroccio et al., 1990, Plant Mol. Biol. 15: 81-93), ein Glutelin-Promotor (Leisy et al., 1990, Plant Mol. Biol. 14: 41-50; Zheng et al., 1993, Plant J. 4: 357-366; Yoshihara et al., 1996, FEBS Lett. 383: 213-218), ein Globulin Promotor (Nakase et al., 1996, Gene 170(2): 223-226), ein Prolamin Promotor (Qu und Takaiwa, 2004, Plant Biotechnology Journal 2(2): 113-125). Es können jedoch auch Promotoren verwendet werden, die nur zu einem durch äußere Einflüsse determinierten Zeitpunkt aktiviert werden (siehe beispielsweise WO 93/07279). Von Interesse können auch Promotoren von heat-shock Proteinen sein, die eine einfache Induktion erlauben. Ferner können samenspezifische Promotoren verwendet werden, wie z.B. der USP-Promoter aus Vicia faba (s.o.).

Ferner kann eine Terminationssequenz (Polyandenylierungssignal) vorhanden sein, die der Addition eines Poly-A-Schwanzes an das Transkript dient. Dem Poly-A-Schwanz wird eine Funktion bei der Stabilisierung der Transkripte beigemessen. Derartige Elemente sind in der Literatur beschrieben (vgl. Gielen et al., 1989, EMBO J. 8: 23-29) und sind beliebig austauschbar.

Es können auch Intronsequenzen zwischen dem Promotor und der kodierenden Region vorhanden sein. Solche Intronsequenzen können zur Stabilität der Expression und zu einer erhöhten Expression in Pflanzen führen (Callis et al., 1987, Genes Devel. 1: 1183-1200; Luehrsen and Walbot 1991, Mol. Gen. Genet. 225: 81-93; Rethmeier et al. 1997, Plant Journal. 12(4): 895-899; Rose and Beliakoff 2000, Plant Physiol. 122 (2): 535-542; Vasil et al., 1989, Plant Physiol. 91: 1575-1579; Xu et al. 2003, Science in China Series C Vol. 46(6): 561-569). Geeignete Intronsequenzen sind beispielsweise das erste Intron des sh1-Gens aus Mais, das erste Intron des Poly-Ubiquitin Gens 1 aus Mais, das erste Intron des EPSPS Gens aus Reis oder eines der beiden ersten Introns des PAT1 Gens aus *Arabidopsis.*

Eine weitere Ausführungsform der vorliegenden Erfindung betrifft ein Verfahren zur Herstellung einer erfindungsgemäßen genetisch modifizierten monokotylen Pflanze, wobei eine Pflanzenzelle, deren Stärke einen apparenten Amylosegehalt von weniger als 5 Gew.-% aufweist,

a) genetisch modifiziert wird, wobei die genetische Modifikation zur Erhöhung der Aktivität eines Proteins mit der Aktivität einer Stärkesynthase II im Vergleich zu entsprechenden nicht genetisch modifizierten Wildtyp-Pflanzenzellen führt;
b) aus Pflanzenzellen von Schritt a) eine Pflanze regeneriert wird;
c) gegebenenfalls weitere Pflanzen mit Hilfe der Pflanzen nach Schritt b) erzeugt werden und
d) Pflanzen erhalten nach Schritt b) oder c) mit einer Pflanze gekreuzt werden, die eine Erhöhung der Aktivität eines Proteins mit der Aktivität einer Glucan-Wasser-Dikinase aufweist, im Vergleich zu entsprechenden nicht genetisch modifizierten Wildtyp-Pflanzenzellen.

Eine weitere Ausführungsform der vorliegenden Erfindung betrifft ein Verfahren zur Herstellung einer erfindungsgemäßen genetisch modifizierten monokotylen Pflanze, wobei eine Pflanzenzelle, deren Stärke einen apparenten Amylosegehalt von weniger als 5 Gew.-% aufweist,

a) genetisch modifiziert wird, wobei die genetische Modifikation zur Erhöhung der Aktivität Proteins mit der Aktivität einer Glucan-Wasser-Dikinase im Vergleich zu entsprechenden nicht genetisch modifizierten Wildtyp-Pflanzenzellen führt;

b) aus Pflanzenzellen von Schritt a) eine Pflanze regeneriert wird;

c) gegebenenfalls weitere Pflanzen mit Hilfe der Pflanzen nach Schritt b) erzeugt werden und

d) Pflanzen erhalten nach Schritt b) oder c) mit einer Pflanze gekreuzt werden, die eine Erhöhung der enzymatischen Aktivität eines Proteins mit der Aktivität einer Stärkesynthase II aufweist, im Vergleich zu entsprechenden nicht genetisch modifizierten Wildtyp-Pflanzenzellen.

Eine weitere Ausführungsform der vorliegenden Erfindung betrifft ein Verfahren zur Herstellung einer erfindungsgemäßen genetisch modifizierten monokotylen Pflanze, worin eine Pflanzenzelle genetisch modifiziert wird, wobei

a) i) die genetische Modifikation zur Erhöhung der Aktivität eines Proteins mit der Aktivität einer Glucan-Wasser-Dikinase führt;

a) ii) eine weitere genetische Modifikation durchgeführt wird, die zur Erhöhung der Aktivität eines Proteins mit der Aktivität einer Stärkesynthase II führt

im Vergleich zu entsprechenden nicht genetisch modifizierten Wildtyp-Pflanzenzellen;

wobei die Schritte a) i) und ii) in beliebiger Reihenfolge durchgeführt werden können

b) aus Pflanzenzellen von Schritt a) i) und ii) eine Pflanze regeneriert wird;

c) gegebenenfalls weitere Pflanzen mit Hilfe der Pflanzen nach Schritt b) erzeugt werden und

d) Pflanzen erhalten nach Schritt a bis c) mit einer Pflanze gekreuzt werden, deren Stärke damit einen Amylosegehalt von weniger als 5 Gew.-% aufweist, im Vergleich zu entsprechenden nicht genetisch modifizierten Wildtyp-Pflanzenzellen.

In den drei letztgenannten Verfahren zur Herstellung einer genetisch modifizierten Pflanze können die Pflanzen nach Schritt a) wie bereits oben beschrieben, genetisch modifiziert werden. Die Regeneration von Pflanzen nach Schritt b) und die Erzeugung weiterer Pflanzen nach Schritt c) und d) wurden ebenfalls bereits weiter oben dargestellt.

Eine Pflanze, die nach Schritt d) der ersten beiden Ausführungsformen mit Pflanzen oder Nachkommen der Pflanzen, erhalten aus Schritt b) oder c), gekreuzt wird, kann jede Pflanze sein, die eine Erhöhung der Aktivität eines Proteins mit der Aktivität einer Stärkesynthase II bzw. eine Erhöhung der Aktivität eines Proteins mit der Aktivität einer Glucan-Wasser-Dikinase aufweist, im Vergleich zu entsprechenden Wildtyp-Pflanzen. Die Erhöhung der Aktivität eines Proteins mit der Aktivität einer Stärkesynthase II bzw. eines Proteins mit der Aktivität einer Glucan-Wasser-Dikinase kann dabei durch jede Modifikation hervorgerufen sein, die zu einer Erhöhung der Aktivität der betreffenden Proteine in den entsprechenden Pflanzen führt. Es kann sich bei diesen Pflanzen um Mutanten oder mittels gentechnischer Methoden modifizierte Pflanzen handeln. Bei den Mutanten kann es sich sowohl um spontan (natürlich) auftretende Mutanten, als auch um solche handeln, die durch den gezielten Einsatz von Mutagenen (wie z.B. chemische Agentien, ionisierende Strahlung) oder gentechnischen Verfahren (z.B. Transposon activation tagging, T-DNA activation tagging, *in vivo*-Mutagenese) erzeugt wurden.

Bevorzugt werden für die Kreuzung in den beiden letztgenannten erfindungsgemäßen Verfahren Pflanzen verwendet, die eine Aktivität eines Proteins mit der Aktivität einer Stärkesynthase II aufweisen, die mindestens 3 fach, bevorzugt 6 fach, bevorzugt mindestens 8 fach und besonders bevorzugt mindestens 10 fach, erhöht ist, im Vergleich zu entsprechenden genetisch nicht modifizierten Wildtyp-Pflanzen.

Betreffende Pflanzen, die eine erhöhte Aktivität eines Proteins mit der Aktivität einer Glucan-Wasser-Dikinase aufweisen, werden für die Kreuzung in den beiden letztgenannten erfindungsgemäßen Verfahren vorzugsweise Pflanzen verwendet, die eine Stärke synthetisieren, die einen Stärkephosphatgehalt aufweist, der mindestens 2,5 nmol C6P/mg Stärke beträgt.

In einer bevorzugten Ausführungsform werden erfindungsgemäße Verfahren zur Herstellung einer genetisch modifizierten Pflanze zur Herstellung von erfindungsgemäßen Pflanzen oder von Pflanzen, die Eigenschaften erfindungsgemäßer Pflanzen aufweisen, verwendet.

Die vorliegende Erfindung betrifft auch die durch erfindungsgemäße Verfahren erhältlichen Pflanzen.

Es wurde überraschenderweise gefunden, dass erfindungsgemäße Pflanzenzellen und erfindungsgemäße Pflanzen, deren Stärke einen apparenten Amylosegehalt von weniger als 5 Gew.-% sowie eine eine Erhöhung der Aktivität eines Proteins mit der Aktivität einer Stärkesynthase II und eine Erhöhung der Aktivität eines Proteins mit der Aktivität einer Glucan-Wasser-Dikinase aufweisen, eine modifizierte Stärke synthetisieren. Insbesondere die Tatsache, dass Stärke, synthetisiert von erfindungsgemäßen Pflanzenzellen oder erfindungsgemäßen Pflanzen, ein erhöhtes Heißwasser Quellvermögen aufweist, war überraschend. Das erhöhte Heißwasser Quellvermögen von Stärken, isolierbar aus erfindungsgemäßen Pflanzenzellen und erfindungsgemäßen Pflanzen, verleiht den erfindungsgemäßen Stärken Eigenschaften, die sie für bestimmte Anwendungen besser geeignet machen als herkömmliche Stärken. Wird Stärke z.B. als Dickungsmittel eingesetzt, so führt das erhöhte Heißwasser Quellvermögen der Stärke dazu, dass deutlich weniger Stärke eingesetzt werden muss, um eine gleiche Dickungsleistung zu erzielen.

Ein weiterer Gegenstand der vorliegenden Erfindung betrifft modifizierte Stärke, dadurch gekennzeichnet, dass sie einen apparenten Amylosegehalt von weniger als 5 Gew.-% sowie ein erhöhtes Heißwasser Quellvermögen aufweist. Das Heißwasser Quellvermögen von erfindungsgemäßer modifizierter Stärke ist bevorzugt um mindestens den Faktor 1,5, besonders bevorzugt um mindestens den Faktor 2, insbesondere bevorzugt um mindestens den Faktor 2,5 und ganz besonders bevorzugt um mindestens den Faktor 3 erhöht im Vergleich zu Stärke, isoliert aus entsprechenden nicht genetisch modifizierten Wildtyp-Pflanzezellen bzw. isoliert aus entsprechenden nicht genetisch modifizierten Wildtyp-Pflanzen.

Methoden zur Ermittlung des Heißwasser Quellvermögens sind dem Fachmann bekannt und in der Literatur beschrieben (z.B. Leach et al., 1959, Cereal Chemistry 36: 534-544). Eine im Zusammenhang mit der vorliegenden Erfindung bevorzugt zu verwendende Methode für die Bestimmung des Heißwasser Quellvermögens ist weiter unten unter "Allgemeine Methoden" beschrieben.

Ein weiterer Gegenstand der vorliegenden Erfindung betrifft modifizierte Stärke, isoliert aus einer monokotylen Pflanzenzelle oder aus einer monokotylen Pflanze, mit einem apparenten Amylosegehalt von 5 Gew.-%, und dadurch charakterisiert, dass sie ein Heißwasser Quellvermögen von mindestens 60 g/g, bevorzugt von 60 bis 100 g/g aufweist. besonders bevorzugt von 70 bis 95 g/g, insbesondere bevorzugt von 80 bis 95 g/g und im speziellen bevorzugt von 80 bis 90 g/g aufweist.

Ein weiterer Gegenstand der vorliegenden Erfindung betrifft modifizierte Stärke, isoliert aus Reispflanzenzellen oder Reispflanzen, mit einem apparenten Amylosegehalt von 5 Gew.-% und dadurch charakterisiert, dass sie ein Heißwasser Quellvermögen von mindestens 60 g/g, bevorzugt von 60 bis 100 g/g, besonders bevorzugt von 70 bis 95 g/g, insbesondere bevorzugt von 80 bis 95 g/g und im speziellen bevorzugt von 80 bis 90 g/g aufweist.

Stärke, synthetisiert von erfindungsgemäßen genetisch modifizierten Pflanzenzellen oder erfindungsgemäßen genetisch modifizierten Pflanzen weist vorzugsweise einen erhöhten Gehalt an Phosphat in C6-Position der Stärke auf. Der Stärkephosphatgehalt von Stärke, isoliert aus erfindungsgemäßen Pflanzenzellen oder erfindungsgemäßen Pflanzen ist dabei deutlich höher als der Stärkephosphatgehalt, den man nach Kreuzung aus der Summe der Stärkephosphatgehalte der betreffenden Elternpflanzen erwarten würde.

Die Menge des in C6-Position der Glucosemoleküle gebundenen Stärkephosphates kann mit dem Fachmann bekannten Methoden wie z.B. photometrisch mittels gekoppelten enzymatischen Test oder mittels $^{31}$P-NMR nach der bei Kasemusuwan und Jane (1996, Cereal Chemistry 73: 702-707) beschriebenen Methode ermittelt werden. Bevorzugt wird im Zusammenhang mit der vorliegenden Erfindung die Menge an in C6-Position der Glucosemoleküle gebundenem Stärkephosphat ermittelt wie unter "Allgemeine Methoden" beschrieben.

Ein weiterer bevorzugter Gegenstand der vorliegenden Erfindung betrifft erfindungsgemäße modifizierte Stärke, dadurch charakterisiert, dass sie aus einer monokotylen Pflanzenzelle oder aus einer monokotylen Pflanze isoliert wurde und einen in C6-Position der Glucosemoleküle der Stärke gebundenen Stärkephosphatgehalt von mindestens 1,5 nmol pro mg Stärke, besonders bevorzugt von mindestens 2,5nmol pro mg Stärke aufweist. Insbesondere bevorzugt handelt es sich bei dieser erfindungsgemäßen modifizierten Stärke um Mais-, Reis- oder Weizenstärke.

In einer weiteren Ausführungsform der vorliegenden Erfindung handelt es sich bei den erfindungsgemäßen modifizierten Stärken um native Stärken.

Der Begriff "native Stärke" bedeutet im Zusammenhang mit der vorliegenden Erfindung, dass die Stärke nach dem Fachmann bekannten Methoden aus erfindungsgemäßen Pflanzen, erfindungsgemäßem erntebaren Pflanzenteilen, erfindungsgemäßen Stärke speichernden Teilen oder erfindungsgemäßem Vermehrungsmaterial von Pflanzen isoliert wird.

Die vorliegende Erfindung betrifft auch erfindungsgemäße modifizierte Stärke, erhältlich aus erfindungsgemäßen Pflanzenzellen oder erfindungsgemäßen Pflanzen, aus erfindungsgemäßem Vermehrungsmaterial oder aus erfindungsgemäßen erntebaren Pflanzenteilen, oder erhältlich aus Pflanzen, die mit einem erfindungsgemäßen Verfahren zur Herstellung einer genetisch modifizierten Pflanze hergestellt wurden.

Pflanzenzellen oder Pflanzen, die eine erfindungsgemäße modifizierte Stärke synthetisieren, sind ebenfalls Gegenstand der vorliegenden Erfindung.

Die vorliegende Erfindung betrifft ferner ein Verfahren zur Herstellung einer modifizierten Stärke, umfassend den Schritt der Extraktion der Stärke aus einer erfindungsgemäßen Pflanzenzelle oder einer erfindungsgemäßen Pflanze, aus erfindungsgemäßem Vermehrungsmaterial einer solchen Pflanze und/oder aus erfindungsgemäßen erntebaren Pflanzenteilen einer solchen Pflanze, vorzugsweise aus erfindungsgemäßen Stärke speichernden Teilen einer solchen Pflanze. Vorzugsweise umfasst ein solches Verfahren auch den Schritt des Erntens der kultivierten Pflanzen bzw. Pflanzenteile und/oder des Vermehrungsmaterials dieser Pflanzen vor der Extraktion der Stärke und besonders bevorzugt ferner den Schritt der Kultivierung erfindungsgemäßer Pflanzen vor dem Ernten.

Verfahren zur Extraktion der Stärke aus Pflanzen oder von Stärke speichernden Teilen von Pflanzen sind dem Fachmann bekannt. Weiterhin sind Verfahren zur Extraktion der Stärke aus verschiedenen Stärke speichernden Pflanzen beschrieben, z. B. in Starch: Chemistry and Technology (Hrsg.: Whistler, BeMiller und Paschall (1994), 2. Ausgabe, Academic Press Inc. London Ltd; ISBN 0-12-746270-8; siehe z.B. Kapitel XII, Seite 412-468: Mais und Sorghum Stärken: Herstellung; von Watson; Kapitel XIII, Seite 469-479: Tapioca, Arrowroot und Sago Stärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem; Maisstärke: Eckhoff et al., 1996, Cereal Chem. 73: 54-57, die Extraktion von Maisstärke im industriellen Maßstab wird in der Regel durch das so genannte "wet milling" erreicht.). Vorrichtungen, die für gewöhnlich bei Verfahren zur Extraktion von Stärke von Pflanzenmaterial verwendet werden, sind Separatoren, Dekanter, Hydrocyclone, Sprühtrockner und Wirbelschichttrockner.

Unter dem Begriff "Stärke speichernde Teile" sollen im Zusammenhang mit der vorliegenden Erfindung solche Teile einer Pflanze verstanden werden, in welchen Stärke, im Gegensatz zu transitorischer Blattstärke, zur Überdauerung von längeren Zeiträumen als Depot gespeichert wird. Bevorzugte Stärke speichernde Pflanzenteile sind z.B. Knollen, Speicherwurzeln und Körner, besonders bevorzugt sind Körner enthaltend ein Endosperm, insbesondere bevorzugt sind Körner enthaltend ein Endosperm von Mais-, Reis- oder Weizenpflanzen.

In einer bevorzugten Ausführungsform werden erfindungsgemäße Verfahren zur Herstellung einer modifizierten Stärke zur Herstellung einer erfindungsgemäßen Stärke verwendet.

Modifizierte Stärke, erhältlich nach einem erfindungsgemäßen Verfahren zur Herstellung modifizierter Stärke, ist ebenfalls Gegenstand der vorliegenden Erfindung.

Weiterhin ist die Verwendung erfindungsgemäßer Pflanzenzellen oder erfindungsgemäßer Pflanzen zur Herstellung einer modifizierten Stärke Gegenstand der vorliegenden Erfindung.

Dem Fachmann ist bekannt, dass die Eigenschaften von Stärke durch z.B. thermische, chemische, enzymatische oder mechanische Derivatisierung verändert werden können. Derivatisierte Stärken sind für verschiedene Anwendungen im Nahrungsmittel- und/oder Nicht-Nahrungsmittelbereich besonders geeignet. Die erfindungsgemäßen Stärken sind als Ausgangssubstanz besser geeignet zur Herstellung von derivatisierten Stärken als herkömmliche Stärken, da sie z.B. durch den höheren Gehalt an Stärkephosphat einen höheren Anteil an reaktiven funktionalen Gruppen aufweisen. Weiterhin können die Derivatisierungen auf Grund des erhöhten Heißwasser Quellvermögens erfindungsgemäßer Stärken bei höheren Temperaturen durchgeführt werden, ohne dabei die Stärkekornstruktur wesentlich zu zerstören.

Die vorliegende Erfindung betrifft daher auch Verfahren zur Herstellung einer derivatisierten Stärke, worin erfindungsgemäße modifizierte Stärke nachträglich derivatisiert wird. Weiterhin betrifft die vorliegende Erfindung eine derivatisierte Stärke, hergestellt nach einem der bekannten Verfahren.

Unter dem Begriff "derivatisierte Stärke" soll im Zusammenhang mit der vorliegenden Erfindung eine erfindungsgemäße modifizierte Stärke verstanden werden, deren Eigenschaften nach der Isolierung aus pflanzlichen Zellen mit Hilfe von chemischen, enzymatischen, thermischen oder mechanischen Verfahren verändert wurde.

In einer weiteren Ausführungsform der vorliegenden Erfindung handelt es sich bei der erfindungsgemäßen derivatisierten Stärke um mit Hitze und/oder mit Säure behandelte Stärke.

In einer weiteren Ausführungsform handelt es sich bei den derivatisierten Stärken um Stärkeether, insbesondere um Stärke-Alkylether, O-Allylether, Hydroxylalkylether, O-Carboxylmethylether, stickstoffhaltige Stärkeether, phosphathaltige Stärkeether oder schwefelhaltige Stärkeether.

In einer weiteren Ausführungsform handelt es sich bei den derivatisierten Stärken um vernetzte Stärken.

In einer weiteren Ausführungsform handelt es sich bei den derivatisierten Stärken um Stärke-Pfropf-Polymerisate.

In einer weiteren Ausführungsform handelt es sich bei den derivatisierten Stärken um oxidierte Stärken.

In einer weiteren Ausführungsform handelt es sich bei den derivatisierten Stärken um Stärkeester, insbesondere um Stärkeester, die unter Verwendung von organischen Säuren in die Stärke eingeführt wurden. Besonders bevorzugt handelt es sich um so genannte Phosphat-, Nitrat-, Sulfat-, Xanthat-, Acetat- oder Citratstärken.

Die erfindungsgemäßen derivatisierten Stärken eignen sich für verschiedene Verwendungen in der Pharmaindustrie, im Nahrungsmittel- und/oder Nicht-Nahrungsmittelbereich. Methoden zur Herstellung von erfindungsgemäßen derivatisierten Stärken sind dem Fachmann bekannt und in der allgemeinen Literatur ausreichend beschrieben. Eine Übersicht zur Herstellung von derivatisierten Stärken findet sich z.B. bei Orthoefer (in Corn, Chemistry and Technology, 1987, eds. Watson und Ramstad, Chapter 16: 479-499).

Derivatisierte Stärke, erhältlich nach dem erfindungsgemäßen Verfahren zur Herstellung einer derivatisierten Stärke, ist ebenfalls Gegenstand der vorliegenden Erfindung.

Ferner ist die Verwendung erfindungsgemäßer modifizierter Stärken zur Herstellung von derivatisierter Stärke Gegenstand der vorliegenden Erfindung.

Produkte, enthaltend eine erfindungsgemäße Stärke, sind ebenfalls von der vorliegenden Erfindung umfasst.

Mischungen, enthaltend die erfindungsgemäße Stärke, sind ebenfalls von der vorliegenden Erfindung erfasst.

Stärke speichernde Teile von Pflanzen werden oft zu Mehlen verarbeitet. Beispiele für Teile von Pflanzen, aus welchen Mehle hergestellt werden, sind z.B. Knollen von Kartoffelpflanzen und Körner von Getreidepflanzen. Zur Herstellung von Mehlen aus Getreidepflanzen werden die endospermhaltigen Körner dieser Pflanzen gemahlen und gesiebt. Stärke ist ein Hauptbestandteil des Endosperms. Bei anderen Pflanzen, die kein Endosperm, sondern andere Stärke speichernde Teile, wie z.B. Knollen oder Wurzeln enthalten, wird Mehl häufig durch Zerkleinern, Trocknen und anschließendem Mahlen der betreffenden Speicherorgane hergestellt. Die Stärke des Endosperms oder enthaltend in Stärke speichernden Teilen von Pflanzen ist ein wesentlicher Anteil des Mehls, welches aus den betreffenden Pflanzenteilen hergestellt wird. Die Eigenschaften von Mehlen werden daher auch durch die in dem betreffenden Mehl vorliegende Stärke beeinflusst. Erfindungsgemäße Pflanzenzellen und erfindungsgemäße Pflanzen synthetisieren eine veränderte Stärke im Vergleich zu entsprechenden nicht genetisch modifizierten Wildtyp-Pflanzenzellen bzw. nicht genetisch modifizierten Wildtyp-Pflanzen. Mehle, hergestellt aus erfindungsgemäßen Pflanzenzellen, erfindungsgemäßen Pflanzen, erfindungsgemäßem Vermehrungsmaterial oder erfindungsgemäßen erntebaren Teilen weisen daher veränderte Eigenschaften auf. Die Eigenschaften von Mehlen können auch durch Mischen von Stärke mit Mehlen oder durch das Mischen von Mehlen mit unterschiedlichen Eigenschaften beeinflusst werden.

Ein weiterer Gegenstand der vorliegenden Erfindung betrifft daher Mehle, enthaltend eine erfindungsgemäße Stärke.

Ein weiterer Gegenstand der vorliegenden Erfindung betrifft Mehle, herstellbar aus erfindungsgemäßen Pflanzenzellen, erfindungsgemäßen Pflanzen, Stärke speichernden Teilen erfindungsgemäßer Pflanzen, aus erfindungsgemäßem Vermehrungsmaterial oder aus erfindungsgemäßen erntebaren Pflanzenteilen. Bevorzugte Stärke speichernde Teile erfindungsgemäßer Pflanzen für die Herstellung von Mehlen sind Knollen, Speicherwurzeln und ein Endosperm enthaltende Körner. Im Zusammenhang mit der vorliegenden Erfindung besonders bevorzugt sind Körner von Pflanzen der (systematischen) Familie Poaceae, insbesondere bevorzugt stammen Körner von Mais-, Reis- oder Weizenpflanzen. Unter dem Begriff "Mehl" soll im Zusammenhang mit der vorliegenden Erfindung ein durch Mahlen von Pflanzenteilen erhaltenes Pulver verstanden werden. Gegebenenfalls werden Pflanzenteile vor dem Mahlen getrocknet und gesiebt.

Erfindungsgemäße Mehle zeichnen sich auf Grund der in ihnen vorliegenden erfindungsgemäßen Stärke dadurch aus, dass sie ein erhöhtes Heißwasser Quellvermögen aufweisen. Dieses ist z.B. bei der Verarbeitung von Mehlen in der Lebensmittelindustrie für viele Anwendungen, insbesondere bei der Herstellung von Backwaren, gewünscht.

Ein bevorzugter Gegenstand der vorliegenden Erfindung betrifft Mehle, hergestellt aus Körnern einer monokotylen waxy-Pflanze, dadurch gekennzeichnet, dass sie ein Heißwasser Quellvermögen von mindestens 25 g/g, bevorzugt von 25 bis 50 g/g, besonders bevorzugt von 30 bis 45 g/g und insbesondere bevorzugt von 35 bis 45 g/g aufweisen.

Die Bestimmung des Heißwasser Quellvermögens von Mehlen erfolgt dabei analog zu der bereits beschriebenen Methode zur Bestimmung des Heißwasser Quellvermögens für Stärke, mit dem Unterschied, dass hierbei Mehle anstelle von Stärke eingesetzt werden. Eine bevorzugte Methode zur Bestimmung des Heißwasser Quellvermögens von Mehlen ist unter "Allgemeine Methoden" beschrieben.

Ein weiterer Gegenstand der vorliegenden Erfindung ist ein Verfahren zur Herstellung von Mehlen, umfassend den Schritt des Mahlens von erfindungsgemäßen Pflanzenzellen, erfindungsgemäßen Pflanzen, von Teilen erfindungsgemäßer Pflanzen, Stärke speichernden Teilen von erfindungsgemäßen Pflanzen, erfindungsgemäßem Vermehrungsmaterial oder erfindungsgemäßem erntebaren Material.

Mehle können durch Mahlen von Stärke speichernden Teilen von erfindungsgemäßen Pflanzen hergestellt werden. Dem Fachmann ist bekannt, wie er Mehle herstellt. Vorzugsweise umfasst ein Verfahren zur Herstellung von Mehlen auch den Schritt des Erntens der kultivierten Pflanzen bzw. Pflanzenteile und/oder des Vermehrungsmaterials bzw. der Stärke speichernden Teile dieser Pflanzen vor dem Mahlen und besonders bevorzugt ferner den Schritt der Kultivierung erfindungsgemäßer Pflanzen vor dem Ernten.

Produkte, enthaltend ein erfindungsgemäßes Mehl, sind ebenfalls von der vorliegenden Erfindung umfasst.

In einer weiteren Ausführungsform der vorliegenden Erfindung umfasst das Verfahren zur Herstellung von Mehlen eine Prozessierung von erfindungsgemäßen Pflanzen, von Stärke speichernden Teilen erfindungsgemäßer Pflanzen, von erfindungsgemäßem Vermehrungsmaterial oder von erfindungsgemäßem erntebaren Material vor dem Mahlen.

Die Prozessierung kann dabei z.B. eine Hitzebehandlung und/oder eine Trocknung sein. Hitzebehandlung gefolgt von einer Trocknung des Hitze behandelten Materials wird z.B. bei der Herstellung von Mehlen aus Speicherwurzeln oder Knollen wie z.B. aus Kartoffelknollen angewendet, bevor das Mahlen erfolgt. Die Zerkleinerung von erfindungsgemäßen Pflanzen, von Stärke speichernden Teilen erfindungsgemäßer Pflanzen, von erfindungsgemäßem Vermehrungsmaterial oder von erfindungsgemäßem erntebaren Material vor dem Mahlen kann ebenfalls eine Prozessierung im Sinne der vorliegenden Erfindung darstellen. Die Entfernung von pflanzlichem Gewebe vor dem Mahlen, wie z.B. das Entspelzen der Körner, stellt auch eine Prozessierung vor dem Mahlen in Sinne der vorliegenden Erfindung dar.

In einer weiteren Ausführungsform der vorliegenden Erfindung umfasst das Verfahren zur Herstellung von Mehlen nach dem Mahlen eine Prozessierung des Mahlgutes. Das Mahlgut kann dabei z.B. nach dem Mahlen gesiebt werden, um verschiedene Typenmehle herzustellen.

Mischungen, enthaltend ein erfindungsgemäßes Mehl, sind ebenfalls von der vorliegenden Erfindung erfasst.

Ein weiterer Gegenstand der vorliegenden Erfindung ist die Verwendung von erfindungsgemäßen genetisch modifizierten Pflanzenzellen, erfindungsgemäßen Pflanzen, von Teilen erfindungsgemäßer Pflanzen, Stärke speichernden Teilen von erfindungsgemäßen Pflanzen, erfindungsgemäßem Vermehrungsmaterial oder erfindungsgemäßem erntebaren Material zur Herstellung von Mehlen.

Der Offenbarungsgehalt sämtlicher in der Patentanmeldung zitierter Dokumente soll in den Offenbarungsgehalt der vorliegenden Beschreibung der Erfindung eingeschlossen werden.

**Beschreibung der Sequenzen**

SEQ ID NO 1: Nucleinsäuresequenz codierend ein Protein mit der Aktivität einer Glucan-Wasser-Dikinase aus *Solanum tuberosum.*

SEQ ID NO 2: Aminosäuresequenz des durch SEQ ID NO 1 codierten Proteins mit der Aktivität einer Glucan-Wasser-Dikinase aus *Solanum tuberosum.*

SEQ ID NO 3: Nucleinsäuresequenz codierend ein Protein mit der Aktivität einer Stärkesynthase II aus *Triticum aestivum.*

SEQ ID NO 4: Aminosäuresequenz des durch SEQ ID NO 3 codierten Proteins mit der Aktivität einer Stärkesynthase II aus *Triticum aestivum.*

SEQ ID NO 5: Nucleinsäuresequenz codierend ein Protein mit der Aktivität einer Stärkesynthase II aus *Oryza sativa.*

SEQ ID NO 6: Aminosäuresequenz des durch SEQ ID NO 5 codierten Proteins mit der Aktivität einer Stärkesynthase II aus *Oryza sativa.*

SEQ ID NO 7: Nucleinsäuresequenz codierend ein Protein mit der Aktivität einer GBSS I aus *Oryza sativa.*

SEQ ID NO 8: Aminosäuresequenz des durch SEQ ID NO 7 codierten Proteins mit der Aktivität einer GBSS I aus *Oryza sativa.*

SEQ ID NO 9: Nucleinsäuresequenz codierend ein Protein mit der Aktivität einer GBSS I aus *Triticum aestivum.*

SEQ ID NO 10: Aminosäuresequenz des durch SEQ ID NO 9 codierten Proteins mit der Aktivität einer GBSS I aus *Triticum aestivum*

SEQ ID NO 11: Nucleinsäuresequenz codierend ein Protein mit der Aktivität einer GBSS I aus *Zea mays.*

SEQ ID NO 12: Aminosäuresequenz des durch SEQ ID NO 11 codierten Proteins mit der Aktivität einer GBSS I aus *Zea mays*

**Allgemeine Methoden**

Im Folgenden werden Methoden beschrieben, welche zur Durchführung der erfindungsgemäßen Verfahren verwendet werden können. Diese Methoden stellen konkrete Ausführungsformen der vorliegenden Erfindung dar, beschränken die vorliegende Erfindung jedoch nicht auf diese Methoden. Dem Fachmann ist bekannt, dass er durch Modifikation der beschriebenen Methoden und/oder durch Ersetzen einzelner Methodenteile durch alternative Methodenteile die Erfindung in gleicher Weise ausführen kann. Der Inhalt aller zitierten Veröffentlichungen ist durch die Nennung des Zitates in die Beschreibung der Anmeldung mit aufgenommen.

**1.Transformation und Regeneration von Reispflanzen**

Reispflanzen wurden nach der von Hiei et al. (1994, Plant Journal 6(2), 271-282) beschriebenen Methode transformiert.

Die Kulturführung der Reispflanzen im Gewächshaus erfolgte unter nachfolgenden Bedingungen:

Aussaat: Substrat: Mischung aus 100% Weißtorf mit 100 I Sand/qm sowie Ton:180 kg/qm in Rosentöpfen 1,6 I (Hersteller: H. Meyer, Deutschland). pH: 5,4-6,2;
Gründüngung: Hakaphos (Compo, Deutschland) 14%N -16%P -18%K + 2%Mg; 2kg/qm;
Düngung: 3,5 g/Pflanze bis zur Blüte: $NH_4NO_3$ (1,75g) und Flory 2 Basis (Hersteller: Euflor, Deutschland): 1,75g; 3%N -16%P - 15%K + 5%Mg.
Temperatur: Tag 28°C / Nacht: 24°C (16h/8h); Rel. Luftfeuchte: 85-95%;
Licht: 16 h, 350μEinstein/s x qm

**2. Herkunft der zur Transformation verwendeten Sequenzen und Konstrukte**

Zur Transformation von Reis wurde die Sequenz T.a.-SSIIa aus Weizen verwendet. Isolierung und Klonierung erfolgte wie in WO 97-45545 beschrieben (unter der damaligen Bezeichnung "pTaSS1"). Der verwendete Transformationsvektor AH32-191 ist in Beispiel 2 beschrieben.
Weiterhin wurde die Sequenz einer Glucan-Wasser-Dikinase aus Kartoffel (R1 St) verwendet. Isolierung und Klonierung erfolgte wie in Beispiel 5 beschrieben. Der verwendete Transformationsvektor pML82 ist in WO 05/095619 beschrieben.

Das waxy-Merkmal wurde durch eine entsprechende Mutante eingebracht, welche in Beispiel 1 charakterisiert ist.

### 3. Analyse der Expressionshöhe eines Gens mittels Northern-Blot

Die Expression einer Nucleinsäure, die ein Protein codiert, wurde mittels Northern-Blot Analyse untersucht. Hierzu wurden für jede unabhängige mittels Transformation erhaltene Pflanze drei unreife Reiskörner (ca. 15 Tage nach der Blüte) geerntet und in flüssigem Stickstoff eingefroren. Zur Homogenisierung wurden die gefrorenen Reiskörner in einer 96 Loch-Mikrotiter-Platte mit einer 4,5 mm Stahlkugel in einer Retsch-Mühle (Modell MM300) für 30 Sekunden bei einer Frequenz von 30 Hertz zerkleinert. Anschließend wurde die RNA mittels Promega RNA-Extraktionskit nach Angaben des Herstellers isoliert (SV 96 Total RNA Isolation System, Bestell-Nr. Z3505, Promega, Mannheim). Die Konzentration der RNA in den einzelnen Proben wurde durch photometrische Messung der Absorption bei 260 nm bestimmt.

Pro Probe wurden jeweils 2 $\mu$g RNA auf ein einheitliches Volumen gebracht und mit einem identischen Volumen RNA-Probenpuffer (65% (v/v) Formamid, 8% Formaldehyd, 13% (v/v) Gelpuffer (s. o.), 50 $\mu$g/ml Ethidiumbromid) versetzt. Nach Erhitzen (10 min, 65°C) und sofortigem Abkühlen auf Eis wurde die RNA über ein 1,2% (w/v) Agarosegel (20 mM MOPS pH 8,0, 5 mM Na-Acetat, 1 mM EDTA, 6% (v/v) Formaldehyd) unter Verwendung von RNA-Laufpuffer (20 mM MOPS pH 8,0, 5 mM Na-Acetat, 1 mM EDTA) bei einer konstanten Stromstärke von 50-80 mA für ca. 2 Stunden aufgetrennt.

Nachfolgend wurde die RNA mittels Diffusionsblot unter Verwendung von 10x SSC (1,5 M NaCl, 150 mM Na-Citrat pH 7,0) auf eine Hybond-N-Membran transferiert und mittels UV-Bestrahlung auf der Membran immobilisiert.

Für die Hybridisierung des Northern-Blots zum Nachweis der Expression eines Nucleinsäuremoleküls, das ein Protein mit der Aktivität einer Stärkesynthase II aus Weizen codiert, wurde ein ca. 1 kb Spel/BspHl-Fragment des Plasmids AH32-191 (Bp 4568-5686), welches den 5'-Bereich der cDNA umfasst, verwendet. Die radioaktive Markierung des DNA-Fragmentes erfolgte mittels des Random primed DNA labeling Kit der Firma Roche (Bestell-Nr. 1004 760) unter Verwendung von $^{32}$P-alpha-dCTP nach Angaben des Herstellers.

Die Nylonmembran, enthaltend die transferierte RNA, wurde für vier Stunden bei 60°C unter leichtem Schütteln im Wasserbad mit Hybridisierungspuffer (250 mM Na-Phosphat-Puffer pH 7,2, 1 mM EDTA, 6% (w/v) SDS, 1% (w/v) BSA) inkubiert, bevor zum Hybridisierungspuffer die radioaktiv markierte DNA hinzugefügt wurde. Nach 16 Stunden Inkubation wurde der Hybridisierungspuffer entfernt und die Membran zur Entfernung unspezifisch gebundener DNA-Moleküle auf einander folgend einmal mit 3xSSC und einmal mit 2xSSC (s. o.) bei 60°C unter leichtem Schütteln im Wasserbad gewaschen.

Zum Nachweis markierter RNA erfolgte eine Autoradiographie der Nylonmembran auf einen Röntgenfilm bei -70°C für ein bis drei Tage.

### 4. Bestimmung der Aktivität eines Proteins mit der Aktivität einer Stärkesynthase II mittels Aktivitätsgele (Zymogramm)

Der Nachweis der Aktivität von Proteinen mit der Aktivität einer Stärkesynthase in unreifen Reiskörnern erfolgte mittels Aktivitätsgelen (Zymogrammen), bei denen Proteinextrakte unter nativen Bedingungen in einem Polyacrylamidgel aufgetrennt und nachfolgend mit entsprechenden Substraten inkubiert werden. Das entstandene Reaktionsprodukt (alpha-Glucan) wurde mittels Lugol'scher Lösung im Gel angefärbt.

Einzelne unreife Reiskörner (ca. 15 Tage nach Blüte) wurden in flüssigem Stickstoff eingefroren und in 150-200 $\mu$l kaltem Extraktionspuffer (50 mM Tris/HCl pH 7,6, 2,5 mM EDTA, 2 mM DTT, 4 mM PMSF, 0,1% (w/v) Glykogen, 10% (v/v) Glyzerin) homogenisiert. Nach Zentrifugation (15 min, 13000 g, 4°C) wurde der klare Überstand in ein frisches Reaktionsgefäss überführt und ein Aliquot des Extraktes zur Bestimmung des Proteingehaltes nach Bradford (1976, Anal Biochem 72: 248-254) verwendet.

Die Auftrennung der Proteinextrakte erfolgte mittels kontinuierlichem 7,5% Polyacrylamid-Gel (7,5% Acrylamid:Bisacrylamid 37,5:1; 25 mM Tris/HCl pH 7,6, 192 mM Gylcin, 0,1% (w/v) APS, 0,05% (v/v) TEMED) unter Verwendung von einfach konzentriertem Laufpuffer (25 mM Tris/HCl, 192 mM Glycin). Für jede Probe wurden jeweils Mengen entsprechend 15 $\mu$g Protein aufgetragen und die Elektrophorese für 2 bis 2,5 Stunden bei 4°C durchgeführt.

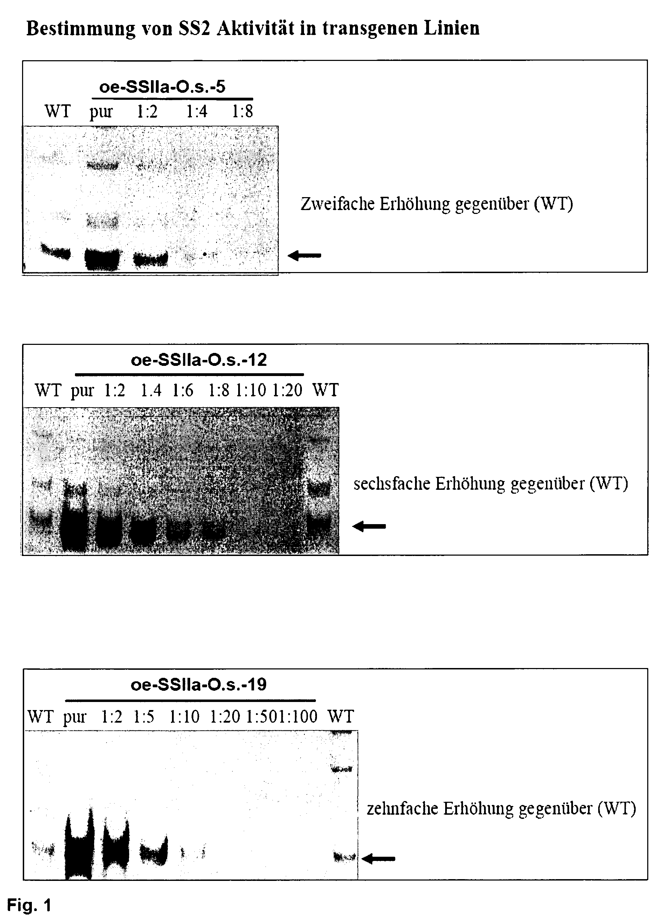

Nachfolgend wurden die Gele in 15 ml Inkubationspuffer ( 0,5 mM Natriumcitrat pH 7,0, 25 mM Kaliumacetat, 2 mM EDTA, 2 mM DTT, 0,1% (w/v) Amylopektin, 50 mM Tricine/NaOH pH 8,5, 1 mM ADP-Glucose) über Nacht bei Raumtemperatur unter ständigem Schütteln inkubiert. Die Anfärbung der gebildeten Stärke erfolgte mittels Lugol'scher Lösung. Um zu ermitteln, um das wieviel Fache die Aktivität eines Proteins mit der Aktivität einer Stärkesynthase II im Vergleich zu entsprechenden nicht genetisch modifizierten Wildtyp-Pflanzen erhöht ist, wurden Proteinextrakte der genetisch modifizierten Linien jeweils sequentiell verdünnt und entsprechend der oben beschriebenen Methode elektrophoretisch aufgetrennt. Die Durchführung der weiteren Schritte erfolgte wie oben bereits beschrieben. Nach Anfärbung der Zymogramme mit Lugol'scher Lösung wurde ein optischer Vergleich der Intensität der angefärbten Produkte, produziert durch ein Protein mit der Aktivität einer Stärkesynthase II (in Fig. 1 mit einem Pfeil gekennzeichnet) für die verschiedenen Verdünnungen der Proteinextrakte von genetisch modifizierten Pflanzen mit den betreffenden Produkten des unverdünnten Wildtyp-Proteinextraktes durchgeführt. Da die Intensität der Färbung der Produkte direkt korreliert mit der Aktivität eines Proteins mit der Aktivität einer Stärkesynthase II, weisen Banden der Produkte mit gleichen Intensitäten die gleiche Aktivität auf. Weist die Bande des Produktes eines Proteins mit der Aktivität einer Stärkesynthase II im verdünnten Proteinextrakt die gleiche Intensität auf, wie die betreffende Bande des Produktes von entsprechendem, unverdünntem Proteinextrakt aus Wildtyp-Pflanzen, so entspricht der Verdünnungsfaktor dem Grad der Erhöhung der Aktivität in der betreffenden genetisch modifizierten Pflanze (Vergleiche dazu Fig. 1).

## 5. Erzeugung von Pflanzen aus isolierten Reisembryonen (Embryo rescue)

Samen werden von der Rispe abgetrennt und die Hüllspelzen entfernt. Das Endosperm wird mit einem Skalpell vom Embryo abgetrennt und für entsprechende Analysen verwendet. Der Embryo wird zur Verbesserung der Benetzbarkeit kurz mit 70% Ethanol behandelt und nachfolgend zur Sterilisation für 20 Minuten in einer Lösung enthaltend 2% NaOCl und einen Tropfen handelsübliches Spülmittel inkubiert.
Anschließend wird die Sterilisationslösung möglichst vollständig entfernt und der Embryo wird einmal für eine Minute und nachfolgend zweimal für jeweils 10 Minuten mit sterilem demineralisierten Wasser gewaschen. Die Samen werden in Petrischalen auf mit Agar verfestigtem Medium enthaltend jeweils ein Viertel der Salzkonzentration von MS-Medium (Murashige-Skoog Medium) und 4% Saccharose ausgelegt. Anschließend werden die Petrischalen mit Parafilm verschlossen und bei 23°C im Dunkeln inkubiert. Nach der Keimung (ca. 5-7 Tage nach dem Auslegen der Embryos) werden die Petrischalen ins Licht überführt. Haben die Hypocotyle der Keimlinge eine Länge von ca. 2 cm erreicht, werden die Pflanzen in Gläser enthaltend mit Agar verfestigtes MS-Medium mit 2% Saccharose überführt. Nach ausreichender Wurzelbildung können die Pflanzen in Erde getopft werden.

## 6. Prozessierung von Reiskörnern und Herstellung von Reismehlen

Zur Erzeugung von ausreichenden Mengen an Untersuchungsmaterial wurden Reispflanzen im Gewächshaus angezogen und nach Erreichen vollständiger Reife geerntet. Zum weiteren Trocknen wurden die reifen Reiskörner für 3-7 Tage bei 37°C gelagert.
Nachfolgend wurden die Körner mittels eines Entspelzers (Laboratory Paddy sheller, Grainman, Miami, Florida, USA) von den Spelzen befreit und der erhaltene braune Reis wurde durch 1-minütiges Polieren (Pearlest Rice Polisher, Kett, Villa Park, CA, USA) zu weißem Reis prozessiert. Für Untersuchungen der Kornzusammensetzung sowie der Stärkeeigenschaften wurden die weißen Körner mittels einer Labormühle (Cyclotec, Sample mill, Foss, Dänemark) zu sog. Reismehl vermahlen.

## 7. Extraktion von Reisstärke aus Reismehl

Die Extraktion von Reisstärke aus Reismehl erfolgte in Anlehnung an die bei Wang und Wang (2004; Journal of Cereal Science 39: 291-296) beschriebene Methode.
Ca. 10g Reismehl wurden mit 40ml 0,05% (w/v) NaOH für 16-18 Stunden auf einem Schüttler bei Raumtemperatur inkubiert. Nachfolgend wurde die Suspension zur Vervollständigung des Aufschlusses in einen Warring Blender überführt und für 15 Sekunden bei geringer Geschwindigkeit sowie anschließend 45 Sekunden bei hoher Geschwindigkeit durchmischt. Zur Abtrennung von größeren Bestandteilen (z.B. Zellwand) wurde die Suspension nacheinander durch Siebe mit einer Maschenweite von 125 $\mu$m und 63 $\mu$m gegeben. Nach Zentrifugation bei 1500 rpm für 15 Minuten (Microfuge 3.OR; Heraeus) wurde der Überstand abgegossen und die an der Oberfläche des Sedimentes liegende Proteinschicht mit einem Spatel entfernt. Das restliche Sediment wurde nochmals in 0,05 % (w/v) NaOH resuspendiert und der oben beschriebene Vorgang wiederholt. Nachfolgend wurde das Sediment in Wasser resuspendiert und der pH-Wert der Suspension mit HCl auf 6,5 bis 7 eingestellt. Die erhaltene Reisstärke wurde insgesamt dreimal mit Wasser gewaschen, wobei jeder Waschschritt eine Sedimentation (Zentrifugation bei 1500 rpm, 15 min, RT), ein Verwerfen des Überstandes und das Resuspendieren des Sedimentes in frischem Wasser umfasste. Vor dem letzten Waschschritt wurde erneut der pH-Wert überprüft und ggf. mit HCl auf pH 7 eingestellt. Das Sediment des letzten Waschschrittes wurde in Aceton resuspendiert, sedimentiert und der Überstand verworfen. Nach erneutem Resuspendieren des Sedimentes in Aceton wurde die Suspension in eine Petrischale gegossen und unter dem Abzug für mindestens 18 Stunden bei Raumtemperatur getrocknet.
In einem letzten Schritt wurde die so erhaltene Reisstärke durch Mörsern in ein feines Pulver überführt, welches direkt für weitere Untersuchungen eingesetzt werden kann.

**8. Bestimmung des Heißwasser Quellvermögens (SP, Swelling Power)**

100 mg Probe (Stärke oder Mehl) werden in 10 ml Wasser suspendiert und anschließend für 20 Minuten bei 92,5°C gequollen. Während der Inkubation der Probe bei 92,5°C wird die Suspension mehrfach (während der ersten 2 Minuten kontinuierlich, dann nach 3, 4, 5, 10, 15 bzw. 25 Minuten) durch vorsichtiges Drehen der Probenbehälter um 360° gemischt. Nach insgesamt 30 Minuten Inkubation bei 92,5°C wird die Suspension ca. 1 Minute in Eiswasser abgekühlt, bevor eine Inkubation bei 25°C für 5 Minuten erfolgt. Nach Zentrifugation (Raumtemperatur, 1000xg, 15 Minuten) wird der erhaltene Überstand vorsichtig von dem gelartigen Sediment abgezogen und das Gewicht des Sedimentes ermittelt. Das Heißwasser Quellvermögen wird nach folgender Formel errechnet:

$$SP = (\text{Gewicht des gelartigen Sedimentes}) / (\text{Gewicht der eingewogenen Probe (Mehl oder Stärke)})$$

**9. Bestimmung des Stärke-Phosphatgehaltes in C6-Position der Glucosemoleküle**

In der Stärke können die Positionen C2, C3 und C6 der Glucoseeinheiten phosphoryliert sein. Zur Bestimmung des C6-P-Gehaltes der Stärke bzw. des Mehls (modifiziert nach Nielsen et al., 1994, Plant Physiol. 105: 111-117) wurden 50 mg Reismehl oder -stärke in 500 $\mu$l 0,7 M HCl 4 h bei 95°C unter ständigem Schütteln hydrolysiert. Anschließend wurden die Ansätze für 10 min bei 15.500xg zentrifugiert und die Überstände mittels einer Filtermembran (0,45 $\mu$M) von Schwebstoffen und Trübungen gereinigt. Von dem klaren Hydrolysat wurden 20 $\mu$l mit 180 $\mu$l Imidazol-Puffer (300 mM Imidazol, pH 7,4; 7,5 mM MgCl2, 1 mM EDTA und 0,4 mM NADP) gemischt und die Proben im Photometer bei 340 nm vermessen. Nach Erfassung der Basisabsorption wurde eine Enzymreaktion durch Zugabe von 2 Einheiten (units) Glucose-6-Phosphat Dehydrogenase (von *Leuconostoc mesenteroides,* Boehringer Mannheim) gestartet. Die gemessene Änderung (OD) beruht auf einer äquimolaren Umsetzung von Glucose-6-Phosphat und NADP zu 6-Phosphorglukonat und NADPH, wobei die Bildung des NADPH bei der o. g. Wellenlänge erfasst wird. Die Reaktion wurde bis zum Erreichen eines Endpunktes verfolgt. Aus dem Ergebnis dieser Messung kann der Gehalt an Glucose-6-Phosphat im Hydrolysat errechnet werden:

$$\text{nmol Glucose-6-Phosphat/mg FG} = \frac{OD \times \text{Messvolumen } (200\mu l) \times \text{Volumen des Hydrolysates } (500\mu l)}{\text{Extinktionskoeffizient} \times \text{Volumen der Messprobe } (20\mu l) \times \text{mg Einwaage } (50mg)}$$

Um keine fehlerhaften Ergebnisse durch nicht-vollständige Hydrolyse der Stärke im eingewogenen Material (Mehl oder Stärke) zu erhalten, wurde anschließend der Grad der Hydrolyse bestimmt. Dazu wurde den jeweiligen auf Glucose-6-Phosphat vermessenen Hydrolysaten 10 $\mu$l Hydrolysat entnommen, mit 10 $\mu$l 0,7 M NaOH neutralisiert, mit Wasser auf ein Endvolumen von 2ml gebracht (Verdünnung 1:200). 4 $\mu$l dieser Verdünnung wurden mit 196 $\mu$l Messpuffer (100mM Imidazol pH 6,9; 5 mM MgCl2, 1 mM ATP, 0,4 mM NADP) versetzt und zur photometrischen Bestimmung des Glucosegehaltes verwendet. Nach Ermittlung der Basisabsorption bei 340nm wurde die Reaktion durch Zugabe von 2 $\mu$l Enzym-Mix (Hexokinase 1:10; Glucose-6-Phosphat Dehydrogenase aus Hefe 1:10 in Messpuffer) bis zur Erreichung des Endpunktes im Photometer (340nm) verfolgt. Das Messprinzip entspricht dem der ersten Reaktion. Aus den erhaltenen Messwerten kann für die jeweilige Probe die Glucosemenge berechnet werden:

$$mmol\ Glucose/g\ FG = \frac{\begin{array}{l}OD \times Messvolumen\ (200\mu l) \\ \times Volumen\ des\ Hydrolysates\ (500\mu l) \\ \times Gesamtvolumen\ der\ Verdünnung\ (2ml)\end{array}}{\begin{array}{l}Extinktionskoeffizient \\ \times Volumen\ der\ Messprobe\ (20\mu l) \\ \times für\ die\ Verdünnung\ eingesetztes\ Volumen\ (10\mu l) \\ \times mg\ Einwaage\ (50mg)\end{array}}$$

Die Menge an nachgewiesener Glucose der einzelnen Proben entspricht dabei dem Anteil der Stärke, der für die C6-Phosphat Bestimmung zur Verfügung steht. Zur Vereinfachung bei der weiteren Berechnung wird der Glucosegehalt in Stärkegehalt umgerechnet.

$$Stärkegehalt\ (\%) = \frac{\begin{array}{l}Glucosegehalt\ (mmol/G\ FG) \\ \times Molekulargewicht\ von\ Glucose\ in\ Stärke\ (162\ g/mol) \\ \times Umrechnungsfaktor\ (\%=100)\end{array}}{Umrechnungsfaktor\ (mmol\ zu\ mol =1000)}$$

Nachfolgend wird das Ergebnis der Glucose-6-Phosphat-Messung mit dem Stärkegehalt der entsprechenden Probe in Relation gesetzt, um so den Gehalt an Glucose-6-Phosphat pro mg hydrolysierter Stärke auszudrücken:

$$nmol\ Glc\text{-}6\text{-}P/mg\ Stärke = \frac{nmol\ Glucose\text{-}6\text{-}Phosphat\ /mg\ Einwaage \times 100}{Stärkegehalt\ (\ mg\ Stärke/\ 100mg\ Einwaage)}$$

Anders als bei einem Bezug der Menge an Glucose-6-Phosphat auf das eingewogene Gewicht der Probe (Mehl oder Stärke) wird durch diese Form der Berechnung die Menge an Glucose-6-Phosphat lediglich auf den Teil der Stärke bezogen, welcher vollständig zu Glucose hydrolysiert wurde.

10. Bestimmung des Gehaltes an apparenter Amlyose

Die Bestimmung des Gehaltes an apparenter Amylose erfolgte in Anlehnung an die Methode von Juliano (1971, Cereal Science Today 16 (10): 334-340).
Für jede Probe wurden zweimal 50 mg Reismehl in 100 ml Erlenmeyerkolben eingewogen und auf einander folgend mit 1 ml 95% Ethanol und 9 ml 1 M NaOH befeuchtet.
Parallel werden zur Anfertigung einer Standardkurve Kolben mit definierten Mengen an reiner Amylose aus Kartoffelstärke in der gleichen Weise behandelt wie die Mehlproben. Die Kolben wurden zur Durchmischung kurz geschwenkt und anschließend für 20 Minuten im kochenden Wasserbad unter leichtem Schütteln inkubiert. Nach 5-10 Minuten Abkühlen bei RT wurde das Volumen mit Wasser auf 100 ml aufgefüllt.
Ein Aliquot von 100$\mu$l wurde mit 1 ml Messlösung (10 mM Essigsäure, 0,004% (w/v) $I_2$; 0,04% (w/v) KI) versetzt, gut durchmischt und die Absorption bei 620nm gegen einen entsprechenden Blindwert bestimmt. Die Berechnung des Amylose-Gehaltes erfolgte mit Hilfe der Amylose-Standards, die zur Erstellung einer Eichkurve verwendet werden.

**11. Quantitative PCR**

RNA wurde von einzelnen unreifen Reissamen (10 - 12 Tage nach Blüte) präpariert. Nach Homogenisierung der in flüssigem Stickstoff eingefrorenen Samen mit einer 4 mm Stahlkugel (Retsch-Mühle, 30 Hz, 45 sec) wurde mit dem "SV 96 Total RNA Isolation System" von Promega die RNA nach Protokoll No. 294 (Promega) präpariert. Die RNA wurde mit je 10 $\mu$l "RQ1 RNase-Free DNase" (Promega) nach Herstellervorschrift behandelt.
Gleiche Mengen RNA von je vier Samen einer Pflanze wurde vereinigt.
Die quantitative RT-PCR wurde mit den Reagenzien des "Access RT-PCR System" von Promega durchgeführt.
Die Reaktionsbedingungen für die RT-PCR waren: 30 min 55 °C, 2 min 94 °C, 40 x (15 sec 94 °C, 1 min 60 °C). Das Fluoreszenzsignal wurde vom Gerät ABI Prism 7700 (Applied Biosystems) jeweils während der kombinierten Annealing/Extensionsphase aufgenommen.
Als Kontrollen wurden jeweils Ansätze ohne reverse Trankriptase im Ansatz mitgeführt.
Die Berechnung der relativen Expression wurde nach M. W. Pfaffl (2001, A new mathematical model for relative quantification in real-time RT-PCR, Nucleic Acids Research 29, No 9 00) durchgeführt.

**Beispiele**

**1. Herstellung und Auswahl der waxy (GBSSI knock out)-Mutante**

Die waxy Mutante ging aus einer Agrobakterien vermittelten Transformation von Reis hervor. Eine Analyse der Nachkommenschaft ergab, dass der waxy Phänotyp der Reiskörner unabhängig von der mit der Transformation eingebrachten Resistenz gegenüber Phosphinotricin vererbt wird. Eine Sequenzanalyse des GBSSI (waxy)-Genes ergab, dass das Auftreten des waxy-Phänotyps auf den Austausch von zwei Nukleotiden zurückzuführen ist, durch die ein vorzeitiges Stopcodon erzeugt wird, was zu einem verkürzten und vermutlich inaktiven Protein führt. Die RFLP-Analyse des apparenten Amylose-Gehaltes der in den Reiskörner enthaltenen Stärke bestätigte mit einem Wert von kleiner 5 Gew.%, dass es sich bei der identifizierten Mutante, um eine "waxy"-Mutante handelt. Daher sind nachfolgend unter dem Begriff "waxy-Phänotyp" Waxy-Mutanten zu verstehen, deren Stärke einen Gehalt an apparenter Amylose unter 5% aufweist.
Für die Kombination mit den transgenen Ansätzen wurden die Linien 738-104 und 738-106 verwendet, welche homozygot für die o.g. Mutation sind.

|  |  | BamHI |
| --- | --- | --- |
| M202 |  | GAG TGG GAT CCT AGC |
| Waxy_Mutante |  | GAG TGA AAT CCT AGC |
|  |  | Stop |

**2. Herstellung des pflanzlichen Expressionsvektors pAH32-191, der eine codierende Sequenz für ein Protein mit der Aktivität einer Stärkesynthase II umfasst**

Die vollständige codierende Sequenz des Proteins mit der Aktivität einer Stärkesynthase II aus Weizen (T.a.-SSII) wurde aus dem Plasmid pCF31 (beschrieben in WO 97/45545 unter der Bezeichnung pTaSS1) mittels der Restriktionsendonucleasen Ecl136II und Xho I herausgeschnitten und in das mit den Restriktionsenducleasen Eco RV und Xho I geschnittene Plasmid pIR103-123 (beschrieben in WO 05/030941) kloniert. Der erhaltene Expressionsvektor wurde mit pAH32-191 bezeichnet. Der pflanzliche Expressionsvektor pIR103-123 dient der endospermspezifischen Expression des Zielgenes unter Kontrolle des endospermspezifischen Globulin-Promotors (Nakase et al. (1996) Gene 170(2): 223-226) aus Reis. Zusätzlich enthält der pflanzliche Expressionsvektor pIR103-123 das bar-Gen unter Kontrolle des CaMV35S Promotors, welches als Selektionsmarker für die Transformation von Pflanzen verwendet wurde.

**3. Herstellung von Reispflanzen, die eine erhöhte Aktivität eines Proteins mit der Aktivität einer Stärkesynthase II aufweisen**

Reispflanzen (Varietät M202) wurden mittels Agrobakterien, die das Plasmid pAH32-191 enthalten, unter Verwendung der bei Hiei et al. (1994, Plant Journal 6(2), 271-282) beschriebenen Methode transformiert. Die erhaltenen Pflanzen erhielten die Bezeichnung oe-SSII-O.s.-X, wobei X unabhängige aus der Transformation hervorgegangene Pflanzen bezeichnet.

**4. Analyse der Reispflanzen, die mit dem Expressionsvektor pAH32-191 transformiert wurden**

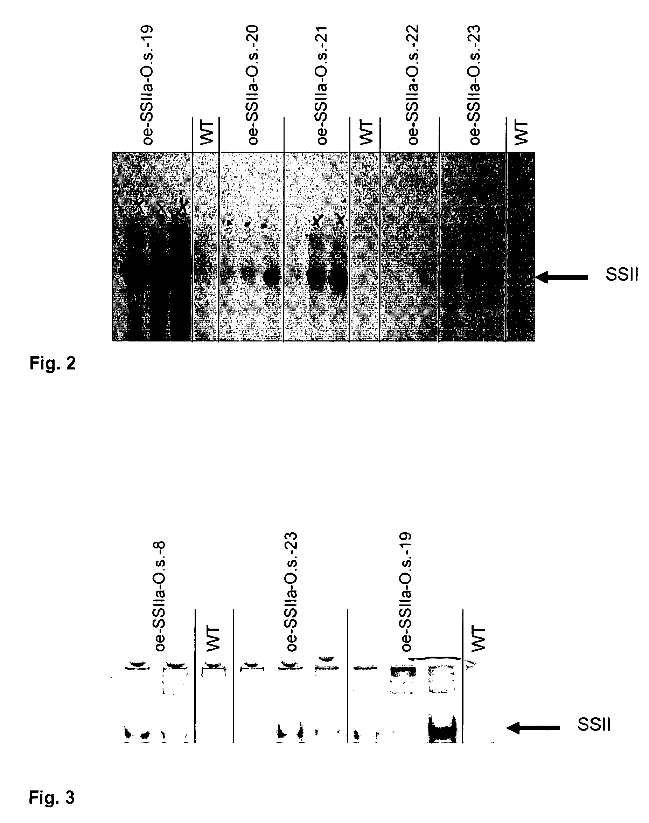

Aus der Transformation mit dem Expressionsvektor pAH32-191 hervorgegangene Reispflanzen (T0 Pflanzen) der Linien mit der Bezeichnung oe-SSII-O.s.-X wurden im Gewächshaus in Erde kultiviert. Aus unreifen Körnern (T1-Samen) verschiedener Linien wurde RNA isoliert und eine Northern-Blot Analyse unter Verwendung einer SSII-spezifischen Sonde nach der unter Allgemeine Methoden beschriebenen Methode durchgeführt. Es konnten mehrere Linien identifiziert werden, die eine erhöhte Menge an Transkript der Stärkesynthase II aus Weizen im Vergleich zu entsprechenden genetisch nicht modifizierten Wildtyp-Pflanzen aufwiesen (siehe beispielhafte Darstellung in Fig. 2). Zusätzlich wurde eine erhöhte Aktivität eines Proteins mit der Aktivität einer Stärkesynthase II in Proteinextrakten unreifer T1-Samen verschiedener Linien der o.g Transformation mittels Zymogrammen nachgewiesen (siehe beispielhafte Darstellung in Fig. 1 und 2). Die Durchführung der Analyse mittels Zymogrammen erfolgte wie unter "Allgemeine Methoden" beschrieben.

Aufgrund der Ergebnisse der beschriebenen Analysen wurde die folgende Linie für die Kombination mit anderen Ansätzen ausgewählt:

oe-SSII-O.s-01502

Anhand verschiedener Analysen konnte gezeigt werden, dass diese Linie homozygot für die Integrationen der T-DNA (s) des Vektors pAH32-191 ist.

**5. Herstellung von Reispflanzen, die eine erhöhte Aktivität eines Proteins mit der Aktivität einer Glucan-Wasser-Dikinase aufweisen**

Reispflanzen (Varietät M202) wurden mittels Agrobakterien, die das Plasmid pML82 (beschrieben in WO 05/095619) enthalten, unter Verwendung der bei Hiei et al. (1994, Plant Journal 6(2), 271-282) beschriebenen Methode transformiert. Die erhaltenen Pflanzen erhielten die Bezeichnung oe-GWD-O.s.-X, wobei X unabhängige aus der Transformation hervorgegangene Pflanzen bezeichnet.

**6. Analyse der Reispflanzen, die mit dem Expressionsvektor pML82 transformiert wurden**

Aus der Transformation mit dem Expressionsvektor pML82 hervorgegangene Reispflanzen (T0 Pflanzen) der Linien mit der Bezeichnung oe-GWD-O.s.-X wurden im Gewächshaus in Erde kultiviert. Aus einzelnen, reifen Körnern (T1-Samen) verschiedener Linien wurde Mehl hergestellt. Hierfür wurden einzelne Körner in einem Eppendorf-Reaktionsgefäß mittels einer Wolfram-Carbit Kugel in einer Kugelmühle (Firma Retsch, Modell MM300) für 30 Sekunden bei einer Frequenz von 30 Hertz zerkleinert. Anschließend erfolgte eine Bestimmung des Stärkephosphatgehaltes in der C6-Position von Glucosemolekülen der in dem Mehl enthaltenen Stärke wie unter "Allgemeine Methoden" beschrieben.

Für ausgewählte Pflanzen wurden folgende Ergebnisse erhalten:

Tabelle 1: Stärkephosphatgehalt in C6-Position der Glucosemoleküle einzelner T1-Samen unterschiedlicher Linien mit der Bezeichnung oe-GWD-O.s.-X im Vergleich zu Samen von entsprechenden nicht genetisch modifizierten Wildtyp-Pflanzen (WT) der Varietät M202.

| Linie | nmol C6P/mg Einwaage |
|---|---|
| oe-GWD-O.s.-2 | 1,68 |
| oe-GWD-O.s.-4 | 1,70 |
| oe-GWD-O.s.-9 | 1,47 |
| WT | 0,30 |

Wie aus Tabelle 1 hervorgeht, konnten unabhängige Linien, hervorgegangen aus der Transformation mit dem pflanzlichen Expressionsvektor pML82, identifiziert werden, die im Vergleich zu entsprechenden nicht genetisch modifizierten Wildtyp-Pflanzen einen erhöhten Gehalt an Stärkephosphat in C6-Position der Glucosemoleküle aufweisen. Es ist bekannt, dass Pflanzenzellen, die eine erhöhte Expression eines Proteins mit der Aktivität einer Glucan-Wasser-Dikinase aufweisen, eine Stärke synthetisieren, die einen höheren Stärkephosphatgehalt aufweist im Vergleich zu entsprechenden genetisch nicht modifizierten Wildtyp-Pflanzen (siehe z.B. WO 02/34923).

Aufgrund der oben beschriebenen Analysen wurden die folgenden Linien für die Kombination mit anderen Ansätzen ausgewählt:

oe-GWD-O.s.-2
oe-GWD-O.s.-4
oe-GWD-O.s.-9

Anhand verschiedener Analysen konnte gezeigt werden, dass diese Linien homozygot für die Integrationen der T-DNA (s) des Vektors pML82 sind.

**7. Herstellung von Pflanzen, die sowohl einen waxy-Phänotyp als auch über eine erhöhte Aktivität eines Proteins mit der Aktivität einer Glucan-Wasser-Dikinase aufweisen**

Durchführung der Kreuzungen:

Tabelle 2: Kreuzungen der Kombination von 738-104/4 (M202 waxy) mit oe-GWD-O.s.

| Pedigree Kreuzung | | Name Mutter | Plasmid Mutter | Name Vater | Plasmid Vater |
|---|---|---|---|---|---|
| XPOS0001 | | M202 waxy | --- | oe-GWD-O.s. | pML82 |
| | -01 | 738-106 | --- | oe-GWD-O.s-2 | pML82 |
| | -02 | 738-104 | --- | oe-GWD-O.s-2 | pML82 |
| | -03 | 738-104 | --- | oe-GWD-O.s-4 | pML82 |
| | -04 | 738-106 | --- | oe-GWD-O.s-4 | pML82 |
| | -05 | 738-104 | --- | oe-GWD-O.s-9 | pML82 |
| | -06 | 738-106 | --- | oe-GWD-O.s-9 | pML82 |

Das Endosperm der aus der Kreuzung hervorgegangenen F1-Samen wurde auf den Stärkephosphatgehalt in C6-Position der Glucosemoleküle (C6P) untersucht. Die Embryonen derjenigen Körner, die einen im Vergleich zur Mutter deutlich erhöhten Stärkephosphatgehalt (C6P) aufwiesen, wurden mittels Gewebe-Kulturtechniken zur Keimung gebracht. Nach Erreichen einer ausreichenden Größe wurden entsprechende Pflanzen zur Produktion von F2-Samen ins Gewächshaus überführt.

Aus den reifen F2-Samen wurden mittels optischer Begutachtung Körner mit waxy-Phänotyp aussortiert und im Gewächshaus ausgelegt. Nach Keimung wurden die Pflanzen mit Basta® (Bayer CropScience) besprüht und von Basta®-toleranten Pflanzen Blattproben genommen. Die Identifizierung homozygoter Pflanzen für die Integration der T-DNA des Vektors pML82 erfolgte anhand einer Kopienzahl-Bestimmung mittels Invader-Technologie (http: //www.twt.com/invader chemistry /invaderchem.htm; Ledford et al (2000, J. of Mol. Diagnostics 2(2): 97-104; Mein et al., 2000, Genome Res.10: 330 - 343) für das bar-Gen. Die so selektierten Pflanzen wurden zur Produktion von F3-Samen im Gewächshaus weiterkultiviert.

Einige reife F3-Samen der potentiell doppelt homozygoten Pflanzen wurden einzeln hinsichtlich ihres Gehaltes an Stärkephosphat (C6P) untersucht. Es wurden diejenigen Pflanzen weiterverwendet, für die alle Körner einen erwartet hohen Gehalt an Stärkephosphat (C6P) aufwiesen.

Das Saatgut aller doppelt homozygoter Pflanzen einer Elternkombination wurde zusammengefasst (gepoolt) und für eine weitere Vermehrung sowie Analysen von Korn- und Mehleigenschaften verwendet.

Für die Kombination mit der Linie oe-SSII-O.s. wurde das Event XPOS0001-05 ausgewählt, welches homozygot sowohl für die waxy-Mutation als auch für die T-DNA des Vektors pML82 ist.

**8. Herstellung von Pflanzen, die einen waxy-Phänotyp sowie eine erhöhte Aktivität eines Proteins mit der Aktivität einer Glucan-Wasser-Dikinase und eine erhöhte Aktivität eines Proteins mit der Aktivität einer Stärkesynthase II aufweisen**

Durchgeführte Kreuzungen:

Tabelle 3: Kreuzungen der Kombination von oe-SSII-O.s. mit XPOS0001-05

| Pedigree Kreuzung | Mutter | Plasmid der Mutter | Vater | Plasmid des Vaters |
|---|---|---|---|---|
| XPOS0025-01 | oe-SSII-O.s.-01502 | pAH31-191 | XPOS0001-05 | pML82 |
| XPOS0026-01 | XPOS0001-05 | pML82 | oeSSII-O.s.-01502 | pAH32-191 |

Die Identifizierung erfolgreicher Kreuzungsevents erfolgte mittels der Messung des Stärkephosphat-Gehaltes des F1-Endosperms, da der Stärkephosphatgehalt der Kombination deutlich höher ist als der der Elternlinien.

**9. Analyse von Pflanzen, die einen waxy-Phänotyp sowie eine erhöhte Aktivität eines Proteins mit der Aktivität einer Glucan-Wasser-Dikinase und eine erhöhte Aktivität eines Proteins mit der Aktivität einer Stärkesynthase 11 aufweisen**

Embryos von F1-Samen, deren Endosperm mit einen Stärkephosphat-Gehalt von mehr als 5nmol C6P/mg Stärke deutlich über dem der beiden Kreuzungspartner (2,5 nmol/mg Stärke für oe-GWD-O.s. und mindestens 0,8nmol/mg Stärke für oe-SSII-O.s.) lag, wurden mittels Gewebekultur-Techniken zur Keimung gebracht und die entsprechenden Pflanzen nach Erreichen einer ausreichenden Größe zur Produktion von F2-Samen ins Gewächshaus überführt.

Zur Identifizierung von Nachkommen, die für beide Transgene sowie die waxy-Mutation homozygot sind, wurde der oben geschilderte Vorgang für F2-Samen, die optisch hinsichtlich eines "waxy-Phänotyp" vorselektiert wurden, einschließlich des "Embryo rescues" wiederholt.

10. Selektion und Analyse der F2-Pflanzen

Basierend auf den Ergebnissen der Stärkephosphat-Messung wurden F2-Samen selektiert (C6P > 8 nmol/mg Stärke), deren Embryos zur Keimung gebracht und die entsprechenden F2-Pflanzen im Gewächshaus kultiviert.

Aus Blattmaterial der F2-Pflanzen wurde genomische DNA extrahiert und mittels Quantitativer PCR) die Kopienzahl der beiden Transgene bzw. des bar-Gens (Summe der Werte für die beiden Transgene) bestimmt.

Der Nachweis der Homozygotie für die waxy-Mutation erfolgte anhand eines RFLPs (Bam HI) im GBSSI-Gen (Definition bzw. Methode) der waxy-Mutante.

F2-Pflanzen, die potentiell homozygot für beide Transgene und homozygot für den waxy-RFLP sind, wurden im Gewächshaus weiter kultiviert und zur Produktion von F3-Samen verwendet.

**11. Selektion der F3-Pflanzen / Analyse von F3-Samen**

Zur Identifizierung dreifach homozygoter Linien wurden von entsprechend ausgewählten Pflanzen einige Einzelkörner optisch hinsichtlich eines waxy-Phänotypes untersucht und nachfolgend auf ihren Stärkephosphat-Gehalt untersucht. Weisen alle Körner einen waxy-Phänotyp auf und fällt der Stärkephosphat-Gehalt für alle Körner einer Pflanze annähernd gleich hoch aus, geht man davon aus, dass die Pflanze für die waxy-Mutation sowie die T-DNA von pML82 und pAH32-191 homozygot ist.

**12. Herstellung von F4-Material**

Folgende Linien gingen aus der o.g. Analyse als dreifach homozygot hervor:

XPOS002501-1-37
XPOS002501-1-13
XPOS002601-1-19

Von diesen Linien wurden Pflanzen im Gewächshaus angezogen und die erzeugten F4-Samen nach Ernte und Trocknung für alle Nachkommen einer Linie gepoolt.

**13. Funktionalitäten und Analyse der Inhaltsstoffe des F4-Materials**

a) Kornzusammensetzung

Gehalt an apparenter Amylose:

Tabelle 4: Gehalt an apparenter Amylose in Reismehlen und -stärken für die Einzelgen-Ansätze sowie die Dreifach-Kombination

| Probenbezeichnung | Apparenter Amylose-Gehalt von Reismehlen (%Amylose/FG) | Apparenter Amylose-Gehalt von Reisstärken (%Amylose/FG) |
|---|---|---|
| Wildtyp | 8,9 | 11,8 |
| oe-GWD-0.s.-4 | 10,6 | 14,4 |
| oe-GWD-O.s.-9 | 10,6 | 14,3 |
| oe-SSII-O.s.-01502 | 6,6 | 9,2 |
| 738-104/6 | 2,3 | 2,2 |
| XPOS025-01-1-37 | 3,7 | 3,5 |
| XPOS025-01-1-13 | 3,7 | 3,7 |
| XPOS026-01-1-19 | 3,9 | 4,1 |

Es zeigte sich, dass die Kombinationen XPOS0025/6 einen Amylose-Gehalt über dem der waxy-Mutante (738-104/6) aufweisen.

Stärkephosphatgehalt (C6P-Gehalte)

Tabelle 5: Gehalt an Stärkephosphat in C6-Position von Reismehlen bzw. -stärken für die Einzeigen-Ansätze sowie die Dreifach-Kombinationen

| Probenbezeichnung | Gehalt an Stärkephosphat in C6-Position von Stärken enthalten in Reismeh-len (nmol C6P/mg Stärke) | Gehalt an Stärkephosphat in C6-Position von Reis-stärken (nmol C6P/mg Stärke) |
|---|---|---|
| Wildtyp | 0,46 | 0,37 |
| oe-GWD-O.s.-4 | 2,85 | 2,65 |
| oe-GWD-O.s.-9 | 3,27 | 2,56 |
| oe-SSII-O.s.-01502 | 1,22 | 0,91 |
| 738-104/6 | 0,52 | 0,38 |
| XPOS025-01-1-37 | 11,45 | 9,50 |
| XPOS025-01-1-13 | 11,20 | 10,24 |
| XPOS026-01-1-19 | 11,06 | 10,23 |

Der Gehalt an Stärkephosphat in C6-Position der Dreifachkombination ist deutlich höher als der der Einzelgen-Ansätze.

**b) Funktionalitäten von Reismehlen und -stärken Heisswasser Quellvermögen**

Tabelle 6: Heißwasser Quellvermögen von Reismehlen bzw. -stärken der Einzelgen-Ansätze sowie der Dreifachkombination

| Probenbezeichnung | Heisswasserquellvermögen von Reismehlen (g/g) | Heisswasserquellvermögen von Reisstärken (g/g) |
|---|---|---|
| Wildtyp | 15,7 | 31,9 |
| oe-GWD-O.s.-4 | 21,6 | 38,6 |
| oe-GWD-O.s.-9 | 21,3 | 39,9 |
| oe-SSII-O.s.-01502 | 20,2 | 40,8 |

(fortgesetzt)

| Probenbezeichnung | Heisswasserquellvermögen von Reismehlen (g/g) | Heisswasserquellvermögen von Reisstärken (g/g) |
|---|---|---|
| 738-104/6 | 19,9 | 47,3 |
| XPOS025-01-1-37 | 40,6 | 86,0 |
| XPOS025-01-1-13 | 41,9 | 89,1 |
| XPOS026-01-1-19 | 38,3 | 87,2 |

Die Ermittlung des Heißwasser Quellvermögens von Mehlen bzw. Stärken, hergestellt aus F4-Samen der o.g. Linien und von Wildtyp-Pflanzen erfolgte wie unter "Allgemeine Methode" beschrieben.
Das Heisswasser Quellvermögen der Dreifachkombination liegt deutlich über dem der Einzelgen-Ansätze.

**Beschreibung der Figuren**

Fig. 1 zeigt Zymogrammme zur Bestimmung der Aktivität von Proteinen mit der Aktivität einer Stärkesynthase II im Vergleich zum Wildtyp. Verwendet wurden Gesamtproteinextrakte aus unreifen Körnern (15 Tage nach Blühbeginn) von Wildtyp-Pflanzen (WT) und den von drei unabhängig voneinander aus der Transformation mit dem Expressionsvektor AH32-191 hervorgegangenen genetisch modifizierten Pflanzen (oe-SSII-O.s.-5, oe-SSII-O.s.-12, oe-SSII-O.s.-19). In den Spuren WT und pur sind jeweils gleiche Mengen an Protein der jeweiligen Extrakte aufgetragen. Die Proteinextrakte der genetisch modifizierten Pflanzen wurden sequentiell (1:2, 1:4, 1:6, 1:8, 1:10, 1:20, 1:50 oder 1:100) verdünnt und diese Verdünnungen ebenfalls getrennt voneinander elektrophoretisch aufgetrennt. Durch Vergleich der Intensität der nach Anfärbung mit Lugol'scher Lösung im Zymogrammm vorliegenden spezifischen Produkte synthetisiert durch ein Protein mit der Aktivität einer Stärkesynthase II (durch einen Pfeil gekennzeichnet) von Proteinextrakten aus Wildtyp-Pflanzen mit der Intensität der betreffenden Banden von Proteinextrakten aus genetisch veränderten Pflanzen, kann die Erhöhung der Aktivität einer Stärkesynthase II gegenüber Wildtyp-Pflanzen ermittelt werden. Gleiche Intensitäten bedeuten dabei gleiche Aktivitäten.
Fig.2 zeigt das das Autoradiogram einer Northern-Blot Analyse unreifer T1-Samen der Reislinien oe-SSII-O.s.-19, oe-SSII-O.s.-20, oe-SSII-O.s.-21, oe-SSII-O.s.-22, oe-SSII-O.s.-23 im Vergleich zu nicht genetisch modifizierten Wildtyp-Pflanzen (WT). Dazu wurde aus jeweils drei Samen von unabhängig aus der Transformation mit dem Expressionsvektor AH32-191 hervorgegangenen Linien RNA extrahiert und nach der unter Allgemeine Methoden, Punkt 8 beschriebenen Methode analysiert. Die mit einer markierten Nucleinsäuresonde codierend für ein Protein mit der Aktivität einer Stärkesynthase II aus Weizen hybridisierende Bande ist mit SSII gekennzeichnet.
Fig. 3 Zeigt ein Zymogramm von Proteinextrakten aus unreifen T1-Samen der Reislinien oe-SSII-O.s.-8, oe-SSII-O.s.-19, oe-SSII-O.s.-23 im Vergleich zu Samen von nicht genetisch modifizierten Wildtyp-Pflanzen (WT) nach Anfärbung mit Lugol'scher Lösung. Je Linie wurden Proteinextrakte aus zwei (oe-SSII-O.s.-8) bzw. drei (oe-SSII-O.s.-19, oe-SSII-O.s.-23) verschiedenen Körnern analysiert. Die Analyse mittels Zymogramm erfolgte dabei nach der unter Allgemeine Methoden, Punkt 9 beschriebenen Methode. Die Bande im Zymogramm, die für ein Protein mit der Aktivität einer Stärkesynthase II spezifisch ist, ist mit SSII gekennzeichnet.

**Patentansprüche**

1. Genetisch modifizierte monokotyle Pflanzenzelle, **dadurch gekennzeichnet, dass** ihre Stärke einen Gehalt an apparenter Amylose von weniger als 5 Gew.-% sowie dass sie zusätzlich eine erhöhte Aktivität eines Proteins mit der enzymatischen Aktivität einer Stärkesynthase II und eine erhöhte Aktivität eines Proteins mit der enzymatischen Aktivität einer Glucan-Wasser-Dikinase aufweist, im Vergleich zu entsprechenden genetisch nicht modifizierten Wildtyp-Pflanzenzellen.

2. Genetisch modifizierte monokotyle Pflanzenzelle nach Anspruch 1, wobei die genetische Modifikation in der Einführung mindestens eines fremden Nukleinsäuremoleküles in das Genom der Pflanze besteht.

3. Genetisch modifizierte monokotyle Pflanzenzelle nach einem der Ansprüche 1 oder 2, die eine modifizierte Stärke synthetisiert im Vergleich zu Stärke, isoliert aus entsprechenden genetisch nicht modifizierten Wildtyp-Pflanzenzellen.

4. Genetisch modifizierte monokotyle Pflanzenzelle nach einem der Ansprüche 1, 2 oder 3, die eine Stärke mit erhöhtem Heißwasser Quellvermögen synthetisiert.

5. Genetisch modifizierte monokotyle Pflanzenzelle nach Anspruch 4, deren Stärke ein erhöhtes Heißwasser Quellvermögen zwischen 60 bis 100 g/g aufweist.

6. Monokotyle Pflanze enthaltend genetisch modifizierte Pflanzenzellen nach einem der Ansprüche 1 bis 5.

7. Monokotyle Pflanze nach Anspruch 6, ausgewählt aus der Gruppe umfassend Reis, Mais und Weizen.

8. Vermehrungsmaterial von monokotylen Pflanzen nach einem der Ansprüche 6 oder 7, enthaltend genetisch modifizierte Pflanzenzellen nach einem der Ansprüche 1 bis 5.

9. Verfahren zur Herstellung einer genetisch modifizierten monokotylen Pflanze, worin

   a) eine Pflanzenzelle genetisch modifiziert wird, wobei die genetische Modifikation die folgenden Schritte i bis iii umfasst

   i) Einführung einer genetischen Modifikation in die Pflanzenzelle, wobei die genetische Modifikation zur Erhöhung der Aktivität eines Proteins mit der Aktivität einer Stärkesynthase II im Vergleich zu entsprechenden nicht genetisch modifizierten Wildtyp-Pflanzenzellen führt,
   ii) Einführung einer genetischen Modifikation in die Pflanzenzelle, wobei die genetische Modifikation zur Erhöhung der Aktivität eines Proteins mit der Aktivität einer Glucan-Wasser-Dikinase im Vergleich zu entsprechenden nicht genetisch modifizierten Wildtyp-Pflanzenzellen führt
   iii) Einführung einer genetischen Modifikation in die Pflanzenzelle, wobei die genetische Modifikation zur Reduktion der Aktivität eines Proteins mit der Aktivität einer GBSSI im Vergleich zu entsprechenden nicht genetisch modifizierten Wildtyp-Pflanzenzellen führt,

wobei die Schritte i bis iii in beliebiger Reihenfolge, einzeln oder beliebige Kombinationen der Schritte i bis iii gleichzeitig durchgeführt werden können
b) aus Pflanzenzellen von Schritt a) eine Pflanze regeneriert wird;
c) gegebenenfalls weitere Pflanzen mit Hilfe der Pflanzen nach Schritt b) erzeugt werden,

wobei gegebenenfalls aus Pflanzen gemäß den Schritten b) oder c) Pflanzenzellen isoliert werden und die Verfahrensschritte a) bis c) solange wiederholt werden, bis eine Pflanze erzeugt wurde, die eine erhöhte Aktivität eines Proteins mit der Aktivität einer Stärkesynthase II im Vergleich zu entsprechenden nicht genetisch modifizierten Wildtyp-Pflanzenzellen aufweist und eine erhöhte Aktivität eines Proteins mit der Aktivität einer Glucan-Wasser-Dikinase im Vergleich zu entsprechenden nicht genetisch modifizierten Wildtyp-Pflanzenzellen und eine reduzierte Aktivität eines Proteins mit der Aktivität einer GBSSI im Vergleich zu entsprechenden nicht genetisch modifizierten Wildtyp-Pflanzenzellen aufweist.

10. Verfahren zur Herstellung einer modifizierten Stärke, umfassend den Schritt der Extraktion der Stärke aus genetisch modifizierten Pflanzenzellen nach einem der Ansprüche 1 bis 5, aus Pflanzen nach einem der Ansprüche 6 oder 7, aus Vermehrungsmaterial nach Anspruch 8 oder aus Pflanzen, erhältlich nach einem Verfahren nach Anspruch 9.

11. Verwendung von Teilen von Pflanzen nach einem der Ansprüche 6 oder 7, aus Vermehrungsmaterial nach Anspruch 8 oder aus Pflanzen, erhältlich nach einem Verfahren nach Anspruch 9, zur Herstellung von Stärke.

12. Modifizierte Stärke erhältlich nach einem Verfahren nach Anspruch 10.

13. Modifizierte Stärke nach Anspruch 12, **dadurch gekennzeichnet, dass** sie einen apparenten Amylosegehalt von weniger als 5 Gew.-% sowie ein erhöhtes Heißwasser Quellvermögen zwischen 60-100 g/g aufweist, im Vergleich zu Stärke, isoliert aus entsprechenden nicht genetisch modifizierten monokotylen Wildtyp - Pflanzen.

14. Verfahren zur Herstellung einer derivatisierten Stärke, worin modifizierte Stärke nach einem der Ansprüche 12 oder 13 nachträglich derivatisiert wird.

15. Derivatisierte Stärke, erhältlich nach einem Verfahren nach Anspruch 14.

16. Verwendung von modifizierter Stärke nach einem der Ansprüche 12 oder 13 zur Herstellung von derivatisierter Stärke.

17. Mehl, enthaltend eine modifizierte Stärke, erhältlich nach einem Verfahren nach Anspruch 10 oder enthaltend eine modifizierte Stärke nach einem der Ansprüche 12 oder 13

18. Verfahren zur Herstellung von Mehlen, umfassend den Schritt des Mahlens von Teilen von Pflanzen nach einem der Ansprüche 6 oder 7, von Vermehrungsmaterial nach Anspruch 8 oder von Pflanzen, erhältlich nach einem Verfahren nach Anspruch 9.

19. Verwendung von genetisch modifizierten monokotylen Pflanzenzellen nach einem der Ansprüche 1 bis 5, monokotylen Pflanzen nach einem der Ansprüche 6 oder 7, von Vermehrungsmaterial nach Anspruch 8 oder von monokotylen Pflanzen, erhältlich nach einem Verfahren nach Anspruch 9, zur Herstellung von Mehlen.

20. Produkte, enthaltend eine Stärke nach einem der Ansprüche 10, 12, 13 oder 15.

21. Produkte, enthaltend ein Mehl nach Anspruch 17.

## Bestimmung von SS2 Aktivität in transgenen Linien

**oe-SSlla-O.s.-5**

WT    pur    1:2    1:4    1:8

Zweifache Erhöhung gegenüber (WT)

**oe-SSlla-O.s.-12**

WT  pur  1:2  1.4  1:6  1:8 1:10 1:20  WT

sechsfache Erhöhung gegenüber (WT)

**oe-SSlla-O.s.-19**

WT  pur   1:2  1:5   1:10  1:20 1:501:100  WT

zehnfache Erhöhung gegenüber (WT)

**Fig. 1**

Fig. 2

Fig. 3

**EP 1 950 303 A1**

**Europäisches Patentamt**

**EUROPÄISCHER RECHERCHENBERICHT**

Nummer der Anmeldung

EP 07 09 0009

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,A | WO 97/45545 A (HOECHST SCHERING AGREVO GMBH [DE]; BLOCK MARTINA [DE]; LOERZ HORST [DE] 4. Dezember 1997 (1997-12-04) * Seite 1 - Seite 19; Ansprüche 1,10,12,17,18,27 * * Seite 31 * ----- | 1-21 | INV. C12N15/82 A01H5/10 C08B30/00 A23L1/00 |
| D,A | WO 2005/002359 A (SYNGENTA PARTICIPATIONS AG [CH]; LANAHAN MICHAEL B [US]; BASU SHIP S []) 13. Januar 2005 (2005-01-13) * Seite 1 - Seite 6; Ansprüche 1,2,11; Abbildung 4; Beispiele 2-4 * ----- | 1-21 | |
| D,A | WO 92/11376 A (AMYLOGENE HB [SE]) 9. Juli 1992 (1992-07-09) * Seite 1 - Seite 12; Ansprüche 1-4,11-13,15 * ----- | 1-21 | |
| X | SITOHY MAHMOUD Z ET AL: "Granular properties of different starch phosphate monoesters" STARCH, Bd. 53, Nr. 1, Januar 2001 (2001-01), Seiten 27-34, XP002433254 ISSN: 0038-9056 * das ganze Dokument * ----- | 12-17, 20,21 | RECHERCHIERTE SACHGEBIETE (IPC) C12N A01H |
| X | LANDERITO N A ET AL: "PREPARATION AND PROPERTIES OF STARCH PHOSPHATES USING WAXY, COMMON, AND HIGH-AMYLOSE CORN STARCHES. II. REACTIVE EXTRUSION METHOD" CEREAL CHEMISTRY, AACC INTERNATIONAL, ST PAUL, MN, US, Bd. 82, Nr. 3, 2005, Seiten 271-276, XP009074584 ISSN: 0009-0352 * das ganze Dokument * ----- -/-- | 12-17, 20,21 | |

Der vorliegende Recherchenbericht wurde für alle Patentansprüche erstellt

| Recherchenort | Abschlußdatum der Recherche | Prüfer |
|---|---|---|
| Den Haag | 11. Mai 2007 | Oderwald, Harald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches Patentamt**

**EUROPÄISCHER RECHERCHENBERICHT**

Nummer der Anmeldung

EP 07 09 0009

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 0 522 942 A2 (MATSUTANI KAGAKU KOGYO KK [JP] MATSUTANI KAGAKU KOGYO KK [US]) 13. Januar 1993 (1993-01-13) * das ganze Dokument * ----- | 12-17, 20,21 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 11. Mai 2007 | Oderwald, Harald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 07 09 0009

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-05-2007

| Im Recherchenbericht angeführtes Patentdokument | Datum der Veröffentlichung | Mitglied(er) der Patentfamilie | Datum der Veröffentlichung |
|---|---|---|---|
| WO 9745545 A | 04-12-1997 | AT 356211 T | 15-03-2007 |
| | | AU 737403 B2 | 16-08-2001 |
| | | AU 3030297 A | 05-01-1998 |
| | | BR 9709487 A | 10-08-1999 |
| | | CA 2256461 A1 | 04-12-1997 |
| | | CN 1219970 A | 16-06-1999 |
| | | CZ 9803890 A3 | 17-02-1999 |
| | | EP 0907741 A1 | 14-04-1999 |
| | | JP 2001503964 T | 27-03-2001 |
| | | SK 163698 A3 | 13-04-1999 |
| | | US 6307125 B1 | 23-10-2001 |
| WO 2005002359 A | 13-01-2005 | BR PI0410544 A | 20-06-2006 |
| | | CA 2526480 A1 | 13-01-2005 |
| | | CN 1826412 A | 30-08-2006 |
| | | EP 1629102 A2 | 01-03-2006 |
| WO 9211376 A | 09-07-1992 | AT 243752 T | 15-07-2003 |
| | | AT 332370 T | 15-07-2006 |
| | | AU 9114891 A | 22-07-1992 |
| | | CA 2098171 A1 | 21-06-1992 |
| | | DE 69133285 D1 | 31-07-2003 |
| | | DE 69133285 T2 | 13-05-2004 |
| | | DK 563189 T3 | 13-10-2003 |
| | | EP 0563189 A1 | 06-10-1993 |
| | | ES 2195999 T3 | 16-12-2003 |
| | | FI 932804 A | 17-06-1993 |
| | | HU 66754 A2 | 28-12-1994 |
| | | JP 6507064 T | 11-08-1994 |
| | | JP 2003034702 A | 07-02-2003 |
| | | JP 2004097219 A | 02-04-2004 |
| | | LV 13228 B | 20-02-2005 |
| | | NO 932227 A | 11-08-1993 |
| | | PL 169848 B1 | 30-09-1996 |
| | | SE 467358 B | 06-07-1992 |
| | | US 6784338 B1 | 31-08-2004 |
| EP 0522942 A2 | 13-01-1993 | AT 164854 T | 15-04-1998 |
| | | DE 69225025 D1 | 14-05-1998 |
| | | DE 69225025 T2 | 26-11-1998 |
| | | JP 2989039 B2 | 13-12-1999 |
| | | JP 5015296 A | 26-01-1993 |
| | | US 5362510 A | 08-11-1994 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5300145 A
- WO 0234923 A
- WO 05002359 A
- WO 05095617 A
- WO 9211376 A
- US 4280851 A
- US 6299907 B
- WO 9711188 A
- WO 0077229 A
- US 6462256 B
- WO 9704112 A
- WO 9704113 A
- WO 9837213 A
- WO 9837214 A
- WO 9953050 A
- EP 0321201 B1
- WO 9515972 A
- EP 120516 A
- WO 9506128 A
- EP 0513849 A
- EP 0465875 A
- EP 0292435 A
- US 6750378 B
- WO 0173087 A
- WO 9307279 A
- WO 9745545 A
- WO 05095619 A
- WO 05030941 A

**In der Beschreibung aufgeführte Nicht-Patentliteratur**

- **BALL ; MORELL.** *Annu. Rev, Plant Biol.,* 2003, vol. 54, 207-233
- **TELTOW et al.** *J. Expt. Bot.,* 2004, vol. 55 (406), 2131-2145
- **JANE et al.** *Cereal Foods World,* 1996, vol. 41 (11), 827-832
- **TAKEDA ; HIZUKURI.** *Starch/Stärke,* 1971, vol. 23, 267-272
- **BLENNOW et al.** *Int. J. of Biological Macromolecules,* 2000, vol. 27, 211-218
- **BLENNOW et al.** *Carbohydrate Polymers,* 2000, vol. 41, 163-174
- **RITTE et al.** *PNAS,* 2002, vol. 99, 7166-7171
- **LORBERTH et al.** *Nature Biotechnology,* 1998, vol. 16, 473-477
- **KÖTTING et al.** *Plant Physiol.,* 2005, vol. 137, 2424-252
- **BAUNSGAARD et al.** *Plant Journal,* 2005, vol. 41, 595-605
- **RITTE et al.** *FEBS Letters,* 2006, vol. 580, 4872-4876
- **NARAYANA ; MOORTHY.** *Starch/Stärke,* 2002, vol. 54, 559-592
- **HIZUKURI ; TAKAGI.** *Carbohydr. Res.,* 1984, vol. 134, 1-10
- **TAKEDA et al.** *Carbohydr. Res.,* 1984, vol. 132, 83-92
- **VOET ; VOET.** Biochemistry. John Wiley & Sons, 1990
- **SHURE et al.** *Cell,* 1983, vol. 35, 225-233
- **HOVENKAMP-HERMELINK et al.** *Theoretical and Applied Genetics,* 1987, vol. 75, 217-221
- **VISSER et al.** *Mol. Gen. Genet.,* 1991, vol. 225, 289-296
- **KOSSMANN ; LLOYD.** *Critical Reviews in Plant Sciences,* 2000, vol. 19 (3), 171-226
- **LEACH et al.** *Cereal Chemistry,* 1959, vol. 36, 534-544
- **SINGH et al.** *Journal of the Science of Food and Agriculture,* 2002, vol. 82, 1376-1383
- **TAKIZAWA et al.** *Brazilian Archives of Biology and Technology,* 2004, vol. 47 (6), 921-931
- **YAMAMORI ; QUYNH.** *Theor Appl Genet,* 2000, vol. 100, 23-38
- **YASUI et al.** *Starch/Stärke,* 2002, vol. 54, 179-184
- **SHI et al.** *J. Cereal Sci.,* 1998, vol. 27, 289-299
- **SODHI ; SINGH.** *Food Chemistry,* 2003, vol. 80, 99-108
- **CHEN et al.** *Starch/Stärke,* 2003, vol. 55, 203-212
- **VAN HUNG ; MORITA.** *Starch/Stärke,* 2005, vol. 57, 413-420
- **LIU et al.** *Starch/Stärke,* 1999, vol. 52, 249-252
- Bioanalytik, Spektrum akad. Verlag, 1998
- **RITTE et al.** *Plant Journal,* 2000, vol. 21, 387-391

- Untersuchungen der Expression und Funktion der Stärkesynthase II (SSII) aus Weizen (Triticum aestivum). **WALTER.** Dissertation am Fachbereich Biologie der Universität Hamburg
- **MIKKELSEN et al.** *Biochemical Journal,* 2004, vol. 377, 525-532
- **NISHI et al.** *Plant Physiology,* 2001, vol. 127, 459-472
- **LI et al.** *Funct Integr Genomics,* 2003, vol. 3, 76-85
- **TIEN-SHIN YU et al.** *Plant Cell,* 2001, vol. 13, 1907-1918
- **MIKKELSEN ; BLENNOW.** *Biochemical Journal,* 2005, vol. 385, 355-361
- **MIKKELSEN et al.** *Biochemistry,* 2006, vol. 45, 4674-4682
- **SPRAGUE et al.** *J. Am. Soc. Agron.,* 1943, vol. 35, 817-822
- **SANO.** *Theor. Appl. Genet.,* 1984, vol. 68, 467-473
- **VILLAREAL ; JULIANO.** *Starch/Staerke,* 1986, vol. 38, 118-119
- **ROHDE et al.** *Nucleic Acids Res,* 1988, vol. 16, 7185-7186
- **NAKAMURA et al.** *Mol. Gen. Genet.,* 1995, vol. 248, 253-259
- **HOVENKAMP-HERMELINK et al.** *Theor. Appl. Genet.,* 1987, vol. 75, 217-221
- **OKUNO ; SAKAGUCHI.** *J. Hered,* 1982, vol. 73, 467
- **KOSSMANN ; LLOYD.** Understanding and Influencing Starch Biochemistry. *Critical Reviews in Plant Sciences,* 2000, vol. 19 (3), 171-226
- **EHRENBERG ; HUSAIN.** *Mutation Research,* 1981, vol. 86, 1-113
- **MÜLLER.** *Biologisches Zentralblatt,* 1972, vol. 91 (1), 31-48
- **JAUHAR ; SIDDIQ.** *Indian Journal of Genetics,* 1999, vol. 59 (1), 23-28
- **RAO.** *Cytologica,* 1977, vol. 42, 443-450
- **GUPTA ; SHARMA.** *Oryza,* 1990, vol. 27, 217-219
- **SATOH ; OMURA.** *Japanese Journal of Breeding,* 1981, vol. 31 (3), 316-326
- **ARORA et al.** *Annals of Biology,* 1992, vol. 8 (1), 65-69
- **SCARASCIA-MUGNOZZA et al.** *Mutation Breeding Review,* 1993, vol. 10, 1-28
- **SVEC et al.** *Cereal Research Communications,* 1998, vol. 26 (4), 391-396
- **SHASHIDHARA et al.** *Journal of Maharashtra Agricultural Universities,* 1990, vol. 15 (1), 20-23
- **THORNEYCROFT et al.** *Journal of Experimental Botany,* 2001, vol. 52 (361), 1593-1601
- **RAMACHANDRAN ; SUNDARESAN.** *Plant Physiology and Biochemistry,* 2001, vol. 39, 234-252
- **MAES et al.** *Trends in Plant Science,* 1999, vol. 4 (3), 90-96
- **HIROCHIKA.** *Current Opinion in Plant Biology,* 2001, vol. 4, 118-122
- **HANLEY et al.** *The Plant Journal,* 2000, vol. 22 (4), 557-566
- **KUMAR ; HIROCHIKA.** *Trends in Plant Science,* 2001, vol. 6 (3), 127-134
- **GRECO et al.** *Plant Physiology,* 2001, vol. 125, 1175-1177
- **LIU et al.** *Molecular and General Genetics,* 1999, vol. 262, 413-420
- **HIROYUKI et al.** *The Plant Journal,* 1999, vol. 19 (5), 605-613
- **JEON ; GYNHEUNG.** *Plant Science,* 2001, vol. 161, 211-219
- **KOPREK et al.** *The Plant Journal,* 2000, vol. 24 (2), 253-263
- **AARTS et al.** *Nature,* 1993, vol. 363, 715-717
- **SCHMIDT ; WILLMITZER.** *Molecular and General Genetics,* 1989, vol. 220, 17-24
- **ALTMANN et al.** *Theoretical and Applied Genetics,* 1992, vol. 84, 371-383
- **TISSIER et al.** *The Plant Cell,* 1999, vol. 11, 1841-1852
- **BELZILE ; YODER.** *The Plant Journal,* 1992, vol. 2 (2), 173-179
- **FREY et al.** *Molecular and General Genetics,* 1989, vol. 217, 172-177
- **KNAPP et al.** *Molecular and General Genetics,* 1988, vol. 213, 285-290
- **KRYSAN et al.** *The Plant Cell,* 1999, vol. 11, 2283-2290
- **ATIPIROZ-LEEHAN ; FELDMANN.** *Trends in Genetics,* 1997, vol. 13 (4), 152-156
- **PARINOV ; SUNDARESAN.** *Current Opinion in Biotechnology,* 2000, vol. 11, 157-161
- **JEON ; AN.** *Plant Science,* 2001, vol. 161, 211-219
- **JEON et al.** *The Plant Journal,* 2000, vol. 22 (6), 561-570
- **YOUNG et al.** *Plamt Physiology,* 2001, vol. 125, 513-518
- **PARINOV et al.** *The Plant Cell,* 1999, vol. 11, 2263-2270
- **THORNEYCROFT et al.** *Journal of Experimental Botany,* 2001, vol. 52, 1593-1601
- **MCKINNEY et al.** *The Plant Journal,* 1995, vol. 8 (4), 613-622
- **NAM et al.** *The Plant Cell,* 1989, vol. 1, 699-705
- **LEISTER ; DEAN.** *The Plant Journal,* 1993, vol. 4 (4), 745-750
- **CASTIGLIONI et al.** *Genetics,* 1998, vol. 149, 2039-2056
- **MEKSEM et al.** *Molecular Genetics and Genomics,* 2001, vol. 265, 207-214

- **MEYER et al.** *Molecular and General Genetics,* 1998, vol. 259, 150-160
- **KONIECZNY ; AUSUBEL.** *The Plant Journal,* 1993, vol. 4, 403-410
- **JARVIS et al.** *Plant Mol. Biol,* 1994, vol. 24, 685-687
- **BACHEM et al.** *The Plant Journal,* 1996, vol. 9 (5), 745-753
- **QI et al.** *Nucleic Acids Research,* 2001, vol. 29 (22), 116
- **DRENKARD et al.** *Plant Physiology,* 2000, vol. 124, 1483-1492
- **CHO et al.** *Nature Genetics,* 1999, vol. 23, 203-207
- **MCCALLUM et al.** *Plant Physiology,* 2000, vol. 123, 439-442
- **METTE et al.** *EMBO J.,* 2000, vol. 19, 5194-5201
- **JORGENSEN.** *Trends Biotechnol.,* 1990, vol. 8, 340-344
- **NIEBEL et al.** *Top. Microbiol. Immunol.,* 1995, vol. 197, 91-103
- **FLAVELL et al.** *Curr. Top. Microbiol. Immunol.,* 1995, vol. 197, 43-46
- **PALAUQUI ; VAUCHERET.** *Plant Mol. Biol.,* 1995, vol. 29, 149-159
- **VAUCHERET et al.** *Mol. Gen. Genet.,* 1995, vol. 248, 311-317
- **DE BORNE et al.** *Mol. Gen. Genet.,* 1994, vol. 243, 613-621
- **WATERHOUSE et al.** *Proc. Natl. Acad. Sci. USA,* 1998, vol. 95, 13959-13964
- **WANG ; WATERHOUSE.** *Plant Mol. Biol.,* 2000, vol. 43, 67-82
- **SINGH et al.** *Biochemical Society Transactions,* 2000, vol. 28 (6), 925-927
- **LIU et al.** *Biochemical Society Transactions,* 2000, vol. 28 (6), 927-929
- **SMITH et al.** *Nature,* 2000, vol. 407, 319-320
- **NAP et al.** *6th International Congress of Plant Molecular Biology,* 18. Juni 2000, 7-27
- **FEYTER et al.** *Mol. Gen. Genet.,* 1996, vol. 250, 329-338
- **KIPP et al.** Poster Session beim 5th International Congress of Plant Molecular Biology. 21. September 1997
- **R. A. DIXON ; C. J. ARNTZEN.** Meeting report zu Metabolic Engineering in Transgenic Plants, Keystone Symposia. *TIBTECH,* 1997, vol. 15, 441-447
- **KREN et al.** *Hepatology,* 1997, vol. 25, 1462-1468
- **COLE-STRAUSS et al.** *Science,* 1996, vol. 273, 1386-1389
- **BEETHAM et al.** *PNAS,* 1999, vol. 96, 8774-8778
- **CONRAD ; MANTEUFEL.** *Trends in Plant Science,* 2001, vol. 6, 399-402
- **DE JAEGER et al.** *Plant Molecular Biology,* 2000, vol. 43, 419-428
- **JOBLING et al.** *Nature Biotechnology,* 2003, vol. 21, 77-80
- **JULIANO.** *Cereal Science Today,* 1971, vol. 16 (10), 334-340
- **SAMBROK et al.** Molecular Cloning, A Laboratory Manual. Cold Spring Harbour Laboratory Press, 2001
- **AUSUBEL et al.** Short Protocols in Molecular Biology. John Wiley & Sons, 2002
- **HOEKEMA.** The Binary Plant Vector System. Offsetdrukkerij Kanters B.V, 1985
- **FRALEY et al.** *Crit. Rev. Plant Sci.,* vol. 4, 1-46
- **AN et al.** *EMBO J.,* 1985, vol. 4, 277-287
- **CHAN et al.** *Plant Mol. Biol.,* 1993, vol. 22, 491-506
- **HIEI et al.** *Plant J.,* 1994, vol. 6, 271-282
- **DENG et al.** *Science in China,* 1990, vol. 33, 28-34
- **WILMINK et al.** *Plant Cell Reports,* 1992, vol. 11, 76-80
- **MAY et al.** *Bio/Technology,* 1995, vol. 13, 486-492
- **CONNER ; DOMISSE.** *Int. J. Plant Sci.,* 1992, vol. 153, 550-555
- **RITCHIE et al.** *Transgenic Res.,* 1993, vol. 2, 252-265
- **WAN ; LEMAUX.** *Plant Physiol.,* 1994, vol. 104, 37-48
- **VASIL et al.** *Bio/Technology,* 1993, vol. 11, 1553-1558
- **RITALA et al.** *Plant Mol. Biol.,* 1994, vol. 24, 317-325
- **SPENCER et al.** *Theor. Appl. Genet.,* 1990, vol. 79, 625-631
- **FROMM et al.** *Biotechnology,* 1990, vol. 8, 833-844
- **GORDON-KAMM et al.** *Plant Cell,* 1990, vol. 2, 603-618
- **KOZIEL et al.** *Biotechnology,* 1993, vol. 11, 194-200
- **MOROC et al.** *Theor. Appl. Genet.,* 1990, vol. 80, 721-726
- **KRENS et al.** *Nature,* 1982, vol. 296, 72-74
- **NEHRA et al.** *Plant J.,* 1994, vol. 5, 285-297
- **BECKER et al.** *Plant Journal,* 1994, vol. 5, 299-307
- Plant Cell Culture Protocols. Humana Press, 1999
- **THOMPSON et al.** *Nucleic Acids Research,* 1994, vol. 22, 4673-4680
- **LIU et al.** *Plant Science,* 2003, vol. 165
- **ZHANG et al.** *Plant Cell,* 1991, vol. 3, 1150-1160
- **VERDAGUER.** *Plant Mol. Biol,* vol. 31, 1129-1139

- **STAVOLONE et al.** *Plant Mol. Biol,* 2003, vol. 53, 703-713
- **STOCKHAUS et al.** *Proc. Natl. Acad. Sci. USA,* 1987, vol. 84, 7943-7947
- **STOCKHAUS et al.** *EMBO J.,* 1989, vol. 8, 2445-2451
- **FIEDLER et al.** *Plant Mol. Biol,* 1993, vol. 22, 669-679
- **BÄUMLEIN et al.** *Mol. Gen. Genet.,* 1991, vol. 225, 459-467
- **PEDERSEN et al.** *Cell,* 1982, vol. 29, 1015-1026
- **QUATROCCIO et al.** *Plant Mol. Biol,* 1990, vol. 15, 81-93
- **LEISY et al.** *Plant Mol. Biol,* 1990, vol. 14, 41-50
- **ZHENG et al.** *Plant J.,* 1993, vol. 4, 357-366
- **YOSHIHARA et al.** *FEBS Lett.,* 1996, vol. 383, 213-218
- **NAKASE et al.** *Gene,* 1996, vol. 170 (2), 223-226
- **QU ; TAKAIWA.** *Plant Biotechnology Journal,* 2004, vol. 2 (2), 113-125
- **GIELEN et al.** *EMBO J.,* 1989, vol. 8, 23-29
- **CALLIS et al.** *Genes Devel.,* 1987, vol. 1, 1183-1200
- **LUEHRSEN ; WALBOT.** *Mol. Gen. Genet.,* 1991, vol. 225, 81-93
- **RETHMEIER et al.** *Plant Journal,* 1997, vol. 12 (4), 895-899
- **ROSE ; BELIAKOFF.** *Plant Physiol.,* 2000, vol. 122 (2), 535-542
- **VASIL et al.** *Plant Physiol.,* 1989, vol. 91, 1575-1579
- **XU et al.** *Science in China Series C,* 2003, vol. 46 (6), 561-569
- **KASEMUSUWAN ; JANE.** *Cereal Chemistry,* 1996, vol. 73, 702-707
- Starch: Chemistry and Technology. Academic Press Inc. London Ltd, 1994
- Starch: Chemistry and Technology. 412-468
- Starch: Chemistry and Technology. 469-479
- Starch: Chemistry and Technology. 479-490
- Starch: Chemistry and Technology. 491-506
- Starch: Chemistry and Technology. 507-528
- **ECKHOFF et al.** *Cereal Chem.,* 1996, vol. 73, 54-57
- **CORN.** Chemistry and Technology. 1987, vol. 16, 479-499
- **HIEI et al.** *Plant Journal,* 1994, vol. 6 (2), 271-282
- **BRADFORD.** *Anal Biochem,* 1976, vol. 72, 248-254
- **WANG ; WANG.** *Journal of Cereal Science,* 2004, vol. 39, 291-296
- **NIELSEN et al.** *Plant Physiol.,* 1994, vol. 105, 111-117
- **M. W. PFAFFL.** A new mathematical model for relative quantification in real-time RT-PCR. *Nucleic Acids Research,* 2001, vol. 29 (9 00
- **LEDFORD et al.** *J. of Mol. Diagnostics,* 2000, vol. 2 (2), 97-104, http://www.twt.com/invader chemistry /invaderchem.htm
- **MEIN et al.** *Genome Res.,* 2000, vol. 10, 330-343

SEQUENCE LISTING

<110>   Bayer CropScience GmbH

<120>   Genetisch modifizierte Pflanzen, die eine Stärke mit geringem
        Amylosegehalt und erhöhtem Quellvermögen synthetisieren

<130>   BCS 07-5002

<160>   12

<170>   PatentIn version 3.3

<210>   1
<211>   4851
<212>   DNA
<213>   Solanum tuberosum


<220>
<221>   transit_peptide
<222>   (1)..(77)


<220>
<221>   CDS
<222>   (105)..(4499)


<300>
<308>   EMBL / Y09533
<309>   1998-07-30
<313>   (1)..(4499)


<400>   1
catcttcatc gaatttctcg aagcttcttc gctaatttcc tggtttcttc actcaaaatc        60


gacgtttcta gctgaacttg agtgaattaa gccagtggga ggat atg agt aat tcc       116
                                                 Met Ser Asn Ser
                                                 1


tta ggg aat aac ttg ctg tac cag gga ttc cta acc tca aca gtg ttg       164
Leu Gly Asn Asn Leu Leu Tyr Gln Gly Phe Leu Thr Ser Thr Val Leu
5                   10                  15                  20


gaa cat aaa agt aga atc agt cct cct tgt gtt gga ggc aat tct ttg       212
Glu His Lys Ser Arg Ile Ser Pro Pro Cys Val Gly Gly Asn Ser Leu
                25                  30                  35


ttt caa caa caa gtg atc tcg aaa tca cct tta tca act gag ttt cga       260
Phe Gln Gln Gln Val Ile Ser Lys Ser Pro Leu Ser Thr Glu Phe Arg

<pre>
                40                        45                        50

ggt aac agg tta aag gtg cag aaa aag aaa ata cct atg gaa aag aag    308
Gly Asn Arg Leu Lys Val Gln Lys Lys Lys Ile Pro Met Glu Lys Lys
        55                    60                    65

cgt gct ttt tct agt tct cct cat gct gta ctt acc act gat acc tct    356
Arg Ala Phe Ser Ser Ser Pro His Ala Val Leu Thr Thr Asp Thr Ser
        70                    75                    80

tct gag cta gca gaa aag ttc agt cta ggg ggg aat att gag cta cag    404
Ser Glu Leu Ala Glu Lys Phe Ser Leu Gly Gly Asn Ile Glu Leu Gln
85                    90                    95                   100

gtt gat gtt agg cct ccc act tca ggt gat gtg tcc ttt gtg gat ttt    452
Val Asp Val Arg Pro Pro Thr Ser Gly Asp Val Ser Phe Val Asp Phe
                105                   110                   115

caa gta aca aat ggt agt gat aaa ctg ttt ttg cac tgg ggg gca gta    500
Gln Val Thr Asn Gly Ser Asp Lys Leu Phe Leu His Trp Gly Ala Val
                120                   125                   130

aaa ttc ggg aaa gaa aca tgg tct ctt ccg aat gat cgt cca gat ggg    548
Lys Phe Gly Lys Glu Thr Trp Ser Leu Pro Asn Asp Arg Pro Asp Gly
        135                   140                   145

acc aaa gtg tac aag aac aaa gca ctt aga act cca ttt gtt aaa tct    596
Thr Lys Val Tyr Lys Asn Lys Ala Leu Arg Thr Pro Phe Val Lys Ser
        150                   155                   160

ggc tct aac tcc atc ctg aga ctg gag ata cga gac act gct atc gaa    644
Gly Ser Asn Ser Ile Leu Arg Leu Glu Ile Arg Asp Thr Ala Ile Glu
165                   170                   175                   180

gct att gag ttt ctc ata tac gat gaa gcc cac gat aaa tgg ata aag    692
Ala Ile Glu Phe Leu Ile Tyr Asp Glu Ala His Asp Lys Trp Ile Lys
                185                   190                   195

aat aat ggt ggt aat ttt cgt gtc aaa ttg tca aga aaa gag ata cga    740
Asn Asn Gly Gly Asn Phe Arg Val Lys Leu Ser Arg Lys Glu Ile Arg
                200                   205                   210

ggc cca gat gtt tct gtt cct gag gag ctt gta cag atc caa tca tat    788
Gly Pro Asp Val Ser Val Pro Glu Glu Leu Val Gln Ile Gln Ser Tyr
                215                   220                   225

ttg agg tgg gag agg aag gga aaa cag aat tac ccc cct gag aaa gag    836
Leu Arg Trp Glu Arg Lys Gly Lys Gln Asn Tyr Pro Pro Glu Lys Glu
        230                   235                   240
</pre>

```
aag gag gaa tat gag gct gct cga act gtg cta cag gag gaa ata gct      884
Lys Glu Glu Tyr Glu Ala Ala Arg Thr Val Leu Gln Glu Glu Ile Ala
245             250             255             260

cgt ggt gct tcc ata cag gac att cga gca agg cta aca aaa act aat      932
Arg Gly Ala Ser Ile Gln Asp Ile Arg Ala Arg Leu Thr Lys Thr Asn
            265             270             275

gat aaa agt caa agc aaa gaa gag cct ctt cat gta aca aag agt gat      980
Asp Lys Ser Gln Ser Lys Glu Glu Pro Leu His Val Thr Lys Ser Asp
            280             285             290

ata cct gat gac ctt gcc caa gca caa gct tac att agg tgg gag aaa     1028
Ile Pro Asp Asp Leu Ala Gln Ala Gln Ala Tyr Ile Arg Trp Glu Lys
            295             300             305

gca gga aag ccg aac tat cct cca gaa aag caa att gaa gaa ctc gaa     1076
Ala Gly Lys Pro Asn Tyr Pro Pro Glu Lys Gln Ile Glu Glu Leu Glu
            310             315             320

gaa gca aga aga gaa ttg caa ctt gag ctt gag aaa ggc att acc ctt     1124
Glu Ala Arg Arg Glu Leu Gln Leu Glu Leu Glu Lys Gly Ile Thr Leu
325             330             335             340

gat gag ttg cgg aaa acg att aca aaa ggg gag ata aaa act aag gtg     1172
Asp Glu Leu Arg Lys Thr Ile Thr Lys Gly Glu Ile Lys Thr Lys Val
            345             350             355

gaa aag cac ctg aaa aga agt tct ttt gcc gtt gaa aga atc caa aga     1220
Glu Lys His Leu Lys Arg Ser Ser Phe Ala Val Glu Arg Ile Gln Arg
            360             365             370

aag aag aga gac ttt ggg cat ctt att aat aag tat act tcc agt cct     1268
Lys Lys Arg Asp Phe Gly His Leu Ile Asn Lys Tyr Thr Ser Ser Pro
            375             380             385

gca gta caa gta caa aag gtc ttg gaa gaa cca cca gcc tta tct aaa     1316
Ala Val Gln Val Gln Lys Val Leu Glu Glu Pro Pro Ala Leu Ser Lys
            390             395             400

att aag ctg tat gcc aag gag aag gag gag cag att gat gat ccg atc     1364
Ile Lys Leu Tyr Ala Lys Glu Lys Glu Glu Gln Ile Asp Asp Pro Ile
405             410             415             420

cta aat aaa aag atc ttt aag gtc gat gat ggg gag cta ctg gta ctg     1412
Leu Asn Lys Lys Ile Phe Lys Val Asp Asp Gly Glu Leu Leu Val Leu
            425             430             435
```

```
gta gca aag tcc tct ggg aag aca aaa gta cat cta gct aca gat ctg        1460
Val Ala Lys Ser Ser Gly Lys Thr Lys Val His Leu Ala Thr Asp Leu
            440             445             450

aat cag cca att act ctt cac tgg gca tta tcc aaa agt cct gga gag        1508
Asn Gln Pro Ile Thr Leu His Trp Ala Leu Ser Lys Ser Pro Gly Glu
            455             460             465

tgg atg gta cca cct tca agc ata ttg cct cct ggg tca att att tta        1556
Trp Met Val Pro Pro Ser Ser Ile Leu Pro Pro Gly Ser Ile Ile Leu
        470             475             480

gac aag gct gcc gaa aca cct ttt tca gcc agt tct tct gat ggt cta        1604
Asp Lys Ala Ala Glu Thr Pro Phe Ser Ala Ser Ser Ser Asp Gly Leu
485             490             495             500

act tct aag gta caa tct ttg gat ata gta att gaa gat ggc aat ttt        1652
Thr Ser Lys Val Gln Ser Leu Asp Ile Val Ile Glu Asp Gly Asn Phe
            505             510             515

gtg ggg atg cca ttt gtt ctt ttg tct ggt gaa aaa tgg att aag aac        1700
Val Gly Met Pro Phe Val Leu Leu Ser Gly Glu Lys Trp Ile Lys Asn
            520             525             530

caa ggg tcg gat ttc tat gtt ggc ttc agt gct gca tcc aaa tta gca        1748
Gln Gly Ser Asp Phe Tyr Val Gly Phe Ser Ala Ala Ser Lys Leu Ala
            535             540             545

ctc aag gct gct ggg gat ggc agt gga act gca aag tct tta ctg gat        1796
Leu Lys Ala Ala Gly Asp Gly Ser Gly Thr Ala Lys Ser Leu Leu Asp
        550             555             560

aaa ata gca gat atg gaa agt gag gct cag aag tca ttt atg cac cgg        1844
Lys Ile Ala Asp Met Glu Ser Glu Ala Gln Lys Ser Phe Met His Arg
565             570             575             580

ttt aat att gca gct gac ttg ata gaa gat gcc act agt gct ggt gaa        1892
Phe Asn Ile Ala Ala Asp Leu Ile Glu Asp Ala Thr Ser Ala Gly Glu
            585             590             595

ctt ggt ttt gct gga att ctt gta tgg atg agg ttc atg gct aca agg        1940
Leu Gly Phe Ala Gly Ile Leu Val Trp Met Arg Phe Met Ala Thr Arg
            600             605             610

caa ctg ata tgg aac aaa aac tat aac gta aaa cca cgt gaa ata agc        1988
Gln Leu Ile Trp Asn Lys Asn Tyr Asn Val Lys Pro Arg Glu Ile Ser
            615             620             625

aag gct cag gac aga ctt aca gac ttg ttg cag aat gct ttc acc agt        2036
```

```
Lys Ala Gln Asp Arg Leu Thr Asp Leu Leu Gln Asn Ala Phe Thr Ser
    630                 635                 640

cac cct cag tac cgt gaa att ttg cgg atg att atg tca act gtt gga    2084
His Pro Gln Tyr Arg Glu Ile Leu Arg Met Ile Met Ser Thr Val Gly
645                 650                 655                 660

cgt gga ggt gaa ggg gat gta gga cag cga att agg gat gaa att ttg    2132
Arg Gly Gly Glu Gly Asp Val Gly Gln Arg Ile Arg Asp Glu Ile Leu
                    665                 670                 675

gtc atc cag agg aac aat gac tgc aag ggt ggt atg atg caa gaa tgg    2180
Val Ile Gln Arg Asn Asn Asp Cys Lys Gly Gly Met Met Gln Glu Trp
                680                 685                 690

cat cag aaa ttg cat aat aat act agt cct gat gat gtt gtg atc tgt    2228
His Gln Lys Leu His Asn Asn Thr Ser Pro Asp Asp Val Val Ile Cys
            695                 700                 705

cag gca tta att gac tac atc aag agt gat ttt gat ctt ggt gtt tat    2276
Gln Ala Leu Ile Asp Tyr Ile Lys Ser Asp Phe Asp Leu Gly Val Tyr
        710                 715                 720

tgg aaa acc ctg aat gag aac gga ata aca aaa gag cgt ctt ttg agt    2324
Trp Lys Thr Leu Asn Glu Asn Gly Ile Thr Lys Glu Arg Leu Leu Ser
725                 730                 735                 740

tat gac cgt gct atc cat tct gaa cca aat ttt aga gga gat caa aag    2372
Tyr Asp Arg Ala Ile His Ser Glu Pro Asn Phe Arg Gly Asp Gln Lys
                745                 750                 755

ggt ggt ctt ttg cgt gat tta ggt cac tat atg aga aca ttg aag gca    2420
Gly Gly Leu Leu Arg Asp Leu Gly His Tyr Met Arg Thr Leu Lys Ala
            760                 765                 770

gtt cat tca ggt gca gat ctt gag tct gct att gca aac tgc atg ggc    2468
Val His Ser Gly Ala Asp Leu Glu Ser Ala Ile Ala Asn Cys Met Gly
        775                 780                 785

tac aaa act gag gga gaa ggc ttt atg gtt gga gtc cag ata aat cct    2516
Tyr Lys Thr Glu Gly Glu Gly Phe Met Val Gly Val Gln Ile Asn Pro
        790                 795                 800

gta tca ggc ttg cca tct ggc ttt cag gac ctc ctc cat ttt gtc tta    2564
Val Ser Gly Leu Pro Ser Gly Phe Gln Asp Leu Leu His Phe Val Leu
805                 810                 815                 820

gac cat gtg gaa gat aaa aat gtg gaa act ctt ctt gag aga ttg cta    2612
Asp His Val Glu Asp Lys Asn Val Glu Thr Leu Leu Glu Arg Leu Leu
```

825 830 835

```
gag gct cgt gag gag ctt agg ccc ttg ctt ctc aaa cca aac aac cgt       2660
Glu Ala Arg Glu Glu Leu Arg Pro Leu Leu Leu Lys Pro Asn Asn Arg
            840             845             850

cta aag gat ctg ctg ttt ttg gac ata gca ctt gat tct aca gtt aga       2708
Leu Lys Asp Leu Leu Phe Leu Asp Ile Ala Leu Asp Ser Thr Val Arg
            855             860             865

aca gca gta gaa agg gga tat gaa gaa ttg aac aac gct aat cct gag       2756
Thr Ala Val Glu Arg Gly Tyr Glu Glu Leu Asn Asn Ala Asn Pro Glu
        870             875             880

aaa atc atg tac ttc atc tcc ctc gtt ctt gaa aat ctc gca ctc tct       2804
Lys Ile Met Tyr Phe Ile Ser Leu Val Leu Glu Asn Leu Ala Leu Ser
885             890             895             900

gtg gac gat aat gaa gat ctt gtt tat tgc ttg aag gga tgg aat caa       2852
Val Asp Asp Asn Glu Asp Leu Val Tyr Cys Leu Lys Gly Trp Asn Gln
            905             910             915

gct ctt tca atg tcc aat ggt ggg gac aac cat tgg gct tta ttt gca       2900
Ala Leu Ser Met Ser Asn Gly Gly Asp Asn His Trp Ala Leu Phe Ala
            920             925             930

aaa gct gtg ctt gac aga acc cgt ctt gca ctt gca agc aag gca gag       2948
Lys Ala Val Leu Asp Arg Thr Arg Leu Ala Leu Ala Ser Lys Ala Glu
            935             940             945

tgg tac cat cac tta ttg cag cca tct gcc gaa tat cta gga tca ata       2996
Trp Tyr His His Leu Leu Gln Pro Ser Ala Glu Tyr Leu Gly Ser Ile
        950             955             960

ctt ggg gtg gac caa tgg gct ttg aac ata ttt act gaa gaa att ata       3044
Leu Gly Val Asp Gln Trp Ala Leu Asn Ile Phe Thr Glu Glu Ile Ile
965             970             975             980

cgt gct gga tca gca gct tca tta tcc tct ctt ctt aat aga ctc gat       3092
Arg Ala Gly Ser Ala Ala Ser Leu Ser Ser Leu Leu Asn Arg Leu Asp
            985             990             995

ccc gtg ctt cgg  aaa act gca aat cta  gga agt tgg cag att  atc       3137
Pro Val Leu Arg  Lys Thr Ala Asn Leu  Gly Ser Trp Gln Ile  Ile
            1000            1005            1010

agt cca gtt gaa  gcc gtt gga tat gtt  gtc gtt gtg gat gag  ttg       3182
Ser Pro Val Glu  Ala Val Gly Tyr Val  Val Val Val Asp Glu  Leu
            1015            1020            1025
```

```
ctt tca gtt cag  aat gaa atc tac gag  aag ccc acg atc tta  gta      3227
Leu Ser Val Gln  Asn Glu Ile Tyr Glu  Lys Pro Thr Ile Leu  Val
            1030              1035                   1040


gca aaa tct gtt  aaa gga gag gag gaa  att cct gat ggt gct  gtt      3272
Ala Lys Ser Val  Lys Gly Glu Glu Glu  Ile Pro Asp Gly Ala  Val
            1045              1050                   1055


gcc ctg ata aca  cca gac atg cca gat  gtt ctt tca cat gtt  tct      3317
Ala Leu Ile Thr  Pro Asp Met Pro Asp  Val Leu Ser His Val  Ser
            1060              1065                   1070


gtt cga gct aga  aat ggg aag gtt tgc  ttt gct aca tgc ttt  gat      3362
Val Arg Ala Arg  Asn Gly Lys Val Cys  Phe Ala Thr Cys Phe  Asp
            1075              1080                   1085


ccc aat ata ttg  gct gac ctc caa gca  aag gaa gga agg att  ttg      3407
Pro Asn Ile Leu  Ala Asp Leu Gln Ala  Lys Glu Gly Arg Ile  Leu
            1090              1095                   1100


ctc tta aag cct  aca cct tca gac ata  atc tat agt gag gtg  aat      3452
Leu Leu Lys Pro  Thr Pro Ser Asp Ile  Ile Tyr Ser Glu Val  Asn
            1105              1110                   1115


gag att gag ctc  caa agt tca agt aac  ttg gta gaa gct gaa  act      3497
Glu Ile Glu Leu  Gln Ser Ser Ser Asn  Leu Val Glu Ala Glu  Thr
            1120              1125                   1130


tca gca aca ctt  aga ttg gtg aaa aag  caa ttt ggt ggt tgt  tac      3542
Ser Ala Thr Leu  Arg Leu Val Lys Lys  Gln Phe Gly Gly Cys  Tyr
            1135              1140                   1145


gca ata tca gca  gat gaa ttc aca agt  gaa atg gtt gga gct  aaa      3587
Ala Ile Ser Ala  Asp Glu Phe Thr Ser  Glu Met Val Gly Ala  Lys
            1150              1155                   1160


tca cgt aat att  gca tat ctg aaa gga  aaa gtg cct tcc tcg  gtg      3632
Ser Arg Asn Ile  Ala Tyr Leu Lys Gly  Lys Val Pro Ser Ser  Val
            1165              1170                   1175


gga att cct acg  tca gta gct ctt cca  ttt gga gtc ttt gag  aaa      3677
Gly Ile Pro Thr  Ser Val Ala Leu Pro  Phe Gly Val Phe Glu  Lys
            1180              1185                   1190


gta ctt tca gac  gac ata aat cag gga  gtg gca aaa gag ttg  caa      3722
Val Leu Ser Asp  Asp Ile Asn Gln Gly  Val Ala Lys Glu Leu  Gln
            1195              1200                   1205
```

44

```
att ctg atg aaa  aaa cta tct gaa gga  gac ttc agc gct ctt  ggt          3767
Ile Leu Met Lys  Lys Leu Ser Glu Gly  Asp Phe Ser Ala Leu  Gly
        1210             1215                  1220

gaa att cgc aca  acg gtt tta gat ctt  tca gca cca gct caa  ttg          3812
Glu Ile Arg Thr  Thr Val Leu Asp Leu  Ser Ala Pro Ala Gln  Leu
        1225             1230                  1235

gtc aaa gag ctg  aag gag aag atg cag  ggt tct ggc atg cct  tgg          3857
Val Lys Glu Leu  Lys Glu Lys Met Gln  Gly Ser Gly Met Pro  Trp
        1240             1245                  1250

cct ggt gat gaa  ggt cca aag cgg tgg  gaa caa gca tgg atg  gcc          3902
Pro Gly Asp Glu  Gly Pro Lys Arg Trp  Glu Gln Ala Trp Met  Ala
        1255             1260                  1265

ata aaa aag gtg  tgg gct tca aaa tgg  aat gag aga gca tac  ttc          3947
Ile Lys Lys Val  Trp Ala Ser Lys Trp  Asn Glu Arg Ala Tyr  Phe
        1270             1275                  1280

agc aca agg aag  gtg aaa ctg gat cat  gac tat ctg tgc atg  gct          3992
Ser Thr Arg Lys  Val Lys Leu Asp His  Asp Tyr Leu Cys Met  Ala
        1285             1290                  1295

gtc ctt gtt caa  gaa ata ata aat gct  gat tat gca ttt gtc  att          4037
Val Leu Val Gln  Glu Ile Ile Asn Ala  Asp Tyr Ala Phe Val  Ile
        1300             1305                  1310

cac aca acc aac  cca tct tcc gga gac  gac tca gaa ata tat  gcc          4082
His Thr Thr Asn  Pro Ser Ser Gly Asp  Asp Ser Glu Ile Tyr  Ala
        1315             1320                  1325

gag gtg gtc agg  ggc ctt ggg gaa aca  ctt gtt gga gct tat  cca          4127
Glu Val Val Arg  Gly Leu Gly Glu Thr  Leu Val Gly Ala Tyr  Pro
        1330             1335                  1340

gga cgt gct ttg  agt ttt atc tgc aag  aaa aag gat ctc aac  tct          4172
Gly Arg Ala Leu  Ser Phe Ile Cys Lys  Lys Lys Asp Leu Asn  Ser
        1345             1350                  1355

cct caa gtg tta  ggt tac cca agc aaa  ccg atc ggc ctt ttc  ata          4217
Pro Gln Val Leu  Gly Tyr Pro Ser Lys  Pro Ile Gly Leu Phe  Ile
        1360             1365                  1370

aaa aga tct atc  atc ttc cga tct gat  tcc aat ggg gaa gat  ttg          4262
Lys Arg Ser Ile  Ile Phe Arg Ser Asp  Ser Asn Gly Glu Asp  Leu
        1375             1380                  1385

gaa ggt tat gcc  ggt gct ggc ctc tac  gac agt gta cca atg  gat          4307
```

```
       Glu Gly Tyr Ala   Gly Ala Gly Leu Tyr   Asp Ser Val Pro Met   Asp
                   1390                  1395                  1400

       gag gag gaa aaa   gtt gta att gat tac   tct tcc gac cca ttg   ata         4352
       Glu Glu Glu Lys   Val Val Ile Asp Tyr   Ser Ser Asp Pro Leu   Ile
                   1405                  1410                  1415

       act gat ggt aac   ttc cgc cag aca atc   ctg tcc aac att gct   cgt         4397
       Thr Asp Gly Asn   Phe Arg Gln Thr Ile   Leu Ser Asn Ile Ala   Arg
                   1420                  1425                  1430

       gct gga cat gct   atc gag gag cta tat   ggc tct cct caa gac   att         4442
       Ala Gly His Ala   Ile Glu Glu Leu Tyr   Gly Ser Pro Gln Asp   Ile
                   1435                  1440                  1445

       gag ggt gta gtg   agg gat gga aag att   tat gtc gtt cag aca   aga         4487
       Glu Gly Val Val   Arg Asp Gly Lys Ile   Tyr Val Val Gln Thr   Arg
                   1450                  1455                  1460

       cca cag atg tga   ttatattctc gttgtatgtt gttcagagaa gaccacagat            4539
       Pro Gln Met


       gtgatcatat tctcattgta tcagatctgt gaccacttac ctgatacctc ccatgaagtt        4599

       acctgtatga ttatacgtga tccaaagcca tcacatcatg ttcaccttca gctattggag        4659

       gagaagtgag aagtaggaat tgcaatatga ggaataataa gaaaaacttt gtaaaagcta        4719

       aattagctgg gtatgatata gggagaaatg tgtaaacatt gtactatata tagtatatac        4779

       acacgcatta tgtattgcat tatgcactga ataatatcgc agcatcaaag aagaaatcct        4839

       ttgggtggtt tc                                                            4851


       <210>   2
       <211>   1464
       <212>   PRT
       <213>   Solanum tuberosum


       <400>   2


       Met Ser Asn Ser   Leu Gly Asn Asn Leu   Leu Tyr Gln Gly Phe   Leu Thr
       1                 5                  10                  15


       Ser Thr Val Leu   Glu His Lys Ser Arg   Ile Ser Pro Pro Cys   Val Gly
                   20                  25                  30
```

```
Gly Asn Ser Leu Phe Gln Gln Gln Val Ile Ser Lys Ser Pro Leu Ser
        35                  40                  45


Thr Glu Phe Arg Gly Asn Arg Leu Lys Val Gln Lys Lys Lys Ile Pro
    50                  55                  60


Met Glu Lys Lys Arg Ala Phe Ser Ser Ser Pro His Ala Val Leu Thr
65                  70                  75                  80


Thr Asp Thr Ser Ser Glu Leu Ala Glu Lys Phe Ser Leu Gly Gly Asn
                85                  90                  95


Ile Glu Leu Gln Val Asp Val Arg Pro Pro Thr Ser Gly Asp Val Ser
            100                 105                 110


Phe Val Asp Phe Gln Val Thr Asn Gly Ser Asp Lys Leu Phe Leu His
            115                 120                 125


Trp Gly Ala Val Lys Phe Gly Lys Glu Thr Trp Ser Leu Pro Asn Asp
    130                 135                 140


Arg Pro Asp Gly Thr Lys Val Tyr Lys Asn Lys Ala Leu Arg Thr Pro
145                 150                 155                 160


Phe Val Lys Ser Gly Ser Asn Ser Ile Leu Arg Leu Glu Ile Arg Asp
                165                 170                 175


Thr Ala Ile Glu Ala Ile Glu Phe Leu Ile Tyr Asp Glu Ala His Asp
            180                 185                 190


Lys Trp Ile Lys Asn Asn Gly Gly Asn Phe Arg Val Lys Leu Ser Arg
        195                 200                 205


Lys Glu Ile Arg Gly Pro Asp Val Ser Val Pro Glu Glu Leu Val Gln
    210                 215                 220
```

47

Ile Gln Ser Tyr Leu Arg Trp Glu Arg Lys Gly Lys Gln Asn Tyr Pro
225                 230             235                 240

Pro Glu Lys Glu Lys Glu Glu Tyr Glu Ala Ala Arg Thr Val Leu Gln
                245             250             255

Glu Glu Ile Ala Arg Gly Ala Ser Ile Gln Asp Ile Arg Ala Arg Leu
            260             265             270

Thr Lys Thr Asn Asp Lys Ser Gln Ser Lys Glu Glu Pro Leu His Val
        275             280             285

Thr Lys Ser Asp Ile Pro Asp Asp Leu Ala Gln Ala Gln Ala Tyr Ile
        290             295             300

Arg Trp Glu Lys Ala Gly Lys Pro Asn Tyr Pro Pro Glu Lys Gln Ile
305             310             315             320

Glu Glu Leu Glu Glu Ala Arg Arg Glu Leu Gln Leu Glu Leu Glu Lys
            325             330             335

                            .

Gly Ile Thr Leu Asp Glu Leu Arg Lys Thr Ile Thr Lys Gly Glu Ile
        340             345             350

Lys Thr Lys Val Glu Lys His Leu Lys Arg Ser Ser Phe Ala Val Glu
        355             360             365

Arg Ile Gln Arg Lys Lys Arg Asp Phe Gly His Leu Ile Asn Lys Tyr
        370             375             380

Thr Ser Ser Pro Ala Val Gln Val Gln Lys Val Leu Glu Glu Pro Pro
385             390             395             400

Ala Leu Ser Lys Ile Lys Leu Tyr Ala Lys Glu Lys Glu Glu Gln Ile
            405             410             415

Asp Asp Pro Ile Leu Asn Lys Lys Ile Phe Lys Val Asp Asp Gly Glu
        420             425             430

Leu Leu Val Leu Val Ala Lys Ser Ser Gly Lys Thr Lys Val His Leu
        435             440             445

Ala Thr Asp Leu Asn Gln Pro Ile Thr Leu His Trp Ala Leu Ser Lys
    450             455             460

Ser Pro Gly Glu Trp Met Val Pro Pro Ser Ser Ile Leu Pro Pro Gly
465             470             475             480

Ser Ile Ile Leu Asp Lys Ala Ala Glu Thr Pro Phe Ser Ala Ser Ser
            485             490             495

Ser Asp Gly Leu Thr Ser Lys Val Gln Ser Leu Asp Ile Val Ile Glu
        500             505             510

Asp Gly Asn Phe Val Gly Met Pro Phe Val Leu Leu Ser Gly Glu Lys
        515             520             525

Trp Ile Lys Asn Gln Gly Ser Asp Phe Tyr Val Gly Phe Ser Ala Ala
    530             535             540

Ser Lys Leu Ala Leu Lys Ala Ala Gly Asp Gly Ser Gly Thr Ala Lys
545             550             555             560

Ser Leu Leu Asp Lys Ile Ala Asp Met Glu Ser Glu Ala Gln Lys Ser
            565             570             575

Phe Met His Arg Phe Asn Ile Ala Ala Asp Leu Ile Glu Asp Ala Thr
        580             585             590

Ser Ala Gly Glu Leu Gly Phe Ala Gly Ile Leu Val Trp Met Arg Phe
        595             600             605

Met Ala Thr Arg Gln Leu Ile Trp Asn Lys Asn Tyr Asn Val Lys Pro

610                    615                    620

Arg Glu Ile Ser Lys Ala Gln Asp Arg Leu Thr Asp Leu Leu Gln Asn
625                 630              635                 640

Ala Phe Thr Ser His Pro Gln Tyr Arg Glu Ile Leu Arg Met Ile Met
                645              650                 655

Ser Thr Val Gly Arg Gly Gly Glu Gly Asp Val Gly Gln Arg Ile Arg
            660              665                 670

Asp Glu Ile Leu Val Ile Gln Arg Asn Asn Asp Cys Lys Gly Gly Met
        675                 680              685

Met Gln Glu Trp His Gln Lys Leu His Asn Asn Thr Ser Pro Asp Asp
    690                 695              700

Val Val Ile Cys Gln Ala Leu Ile Asp Tyr Ile Lys Ser Asp Phe Asp
705                 710                 715                 720

Leu Gly Val Tyr Trp Lys Thr Leu Asn Glu Asn Gly Ile Thr Lys Glu
            725                 730                 735

Arg Leu Leu Ser Tyr Asp Arg Ala Ile His Ser Glu Pro Asn Phe Arg
            740                 745                 750

Gly Asp Gln Lys Gly Gly Leu Leu Arg Asp Leu Gly His Tyr Met Arg
        755                 760              765

Thr Leu Lys Ala Val His Ser Gly Ala Asp Leu Glu Ser Ala Ile Ala
    770                 775              780

Asn Cys Met Gly Tyr Lys Thr Glu Gly Glu Gly Phe Met Val Gly Val
785                 790              795                 800

Gln Ile Asn Pro Val Ser Gly Leu Pro Ser Gly Phe Gln Asp Leu Leu
            805              810                 815

His Phe Val Leu Asp His Val Glu Asp Lys Asn Val Glu Thr Leu Leu
820                     825                 830

Glu Arg Leu Leu Glu Ala Arg Glu Glu Leu Arg Pro Leu Leu Leu Lys
        835                 840                 845

Pro Asn Asn Arg Leu Lys Asp Leu Leu Phe Leu Asp Ile Ala Leu Asp
    850                 855                 860

Ser Thr Val Arg Thr Ala Val Glu Arg Gly Tyr Glu Glu Leu Asn Asn
865                 870                 875                 880

Ala Asn Pro Glu Lys Ile Met Tyr Phe Ile Ser Leu Val Leu Glu Asn
                885                 890                 895

Leu Ala Leu Ser Val Asp Asp Asn Glu Asp Leu Val Tyr Cys Leu Lys
            900                 905                 910

Gly Trp Asn Gln Ala Leu Ser Met Ser Asn Gly Gly Asp Asn His Trp
        915                 920                 925

Ala Leu Phe Ala Lys Ala Val Leu Asp Arg Thr Arg Leu Ala Leu Ala
        930                 935                 940

Ser Lys Ala Glu Trp Tyr His His Leu Leu Gln Pro Ser Ala Glu Tyr
945                 950                 955                 960

Leu Gly Ser Ile Leu Gly Val Asp Gln Trp Ala Leu Asn Ile Phe Thr
                965                 970                 975

Glu Glu Ile Ile Arg Ala Gly Ser Ala Ala Ser Leu Ser Ser Leu Leu
        980                 985                 990

Asn Arg Leu Asp Pro Val Leu Arg  Lys Thr Ala Asn Leu  Gly Ser Trp
        995                 1000                1005

```
Gln Ile  Ile Ser Pro Val Glu  Ala Val Gly Tyr Val  Val Val Val
    1010             1015         1020


Asp Glu  Leu Leu Ser Val Gln  Asn Glu Ile Tyr Glu  Lys Pro Thr
    1025             1030         1035


Ile Leu  Val Ala Lys Ser Val  Lys Gly Glu Glu Glu  Ile Pro Asp
    1040             1045         1050


Gly Ala  Val Ala Leu Ile Thr  Pro Asp Met Pro Asp  Val Leu Ser
    1055             1060         1065


His Val  Ser Val Arg Ala Arg  Asn Gly Lys Val Cys  Phe Ala Thr
    1070             1075         1080


Cys Phe  Asp Pro Asn Ile Leu  Ala Asp Leu Gln Ala  Lys Glu Gly
    1085             1090         1095


Arg Ile  Leu Leu Leu Lys Pro  Thr Pro Ser Asp Ile  Ile Tyr Ser
    1100             1105         1110


Glu Val  Asn Glu Ile Glu Leu  Gln Ser Ser Ser Asn  Leu Val Glu
    1115             1120         1125


Ala Glu  Thr Ser Ala Thr Leu  Arg Leu Val Lys Lys  Gln Phe Gly
    1130             1135         1140


Gly Cys  Tyr Ala Ile Ser Ala  Asp Glu Phe Thr Ser  Glu Met Val
    1145             1150         1155


Gly Ala  Lys Ser Arg Asn Ile  Ala Tyr Leu Lys Gly  Lys Val Pro
    1160             1165         1170


Ser Ser  Val Gly Ile Pro Thr  Ser Val Ala Leu Pro  Phe Gly Val
    1175             1180         1185
```

```
Phe Glu  Lys Val Leu Ser Asp  Asp Ile Asn Gln Gly  Val Ala Lys
    1190                 1195              1200


Glu Leu  Gln Ile Leu Met Lys  Lys Leu Ser Glu Gly  Asp Phe Ser
    1205                 1210              1215

                                      .

Ala Leu  Gly Glu Ile Arg Thr  Thr Val Leu Asp Leu  Ser Ala Pro
    1220                 1225              1230


Ala Gln  Leu Val Lys Glu Leu  Lys Glu Lys Met Gln  Gly Ser Gly
    1235                 1240              1245


Met Pro  Trp Pro Gly Asp Glu  Gly Pro Lys Arg Trp  Glu Gln Ala
    1250                 1255              1260


Trp Met  Ala Ile Lys Lys Val  Trp Ala Ser Lys Trp  Asn Glu Arg
    1265                 1270              1275


Ala Tyr  Phe Ser Thr Arg Lys  Val Lys Leu Asp His  Asp Tyr Leu
    1280                 1285              1290


Cys Met  Ala Val Leu Val Gln  Glu Ile Ile Asn Ala  Asp Tyr Ala
    1295                 1300              1305


Phe Val  Ile His Thr Thr Asn  Pro Ser Ser Gly Asp  Asp Ser Glu
    1310                 1315              1320


Ile Tyr  Ala Glu Val Val Arg  Gly Leu Gly Glu Thr  Leu Val Gly
    1325                 1330              1335


Ala Tyr  Pro Gly Arg Ala Leu  Ser Phe Ile Cys Lys  Lys Lys Asp
    1340                 1345              1350


Leu Asn  Ser Pro Gln Val Leu  Gly Tyr Pro Ser Lys  Pro Ile Gly
    1355                 1360              1365


Leu Phe  Ile Lys Arg Ser Ile  Ile Phe Arg Ser Asp  Ser Asn Gly
```

1370                    1375                    1380

Glu Asp  Leu Glu Gly Tyr Ala  Gly Ala Gly Leu Tyr  Asp Ser Val
    1385                    1390                    1395

Pro Met  Asp Glu Glu Glu Lys  Val Val Ile Asp Tyr  Ser Ser Asp
    1400                    1405                    1410

Pro Leu  Ile Thr Asp Gly Asn  Phe Arg Gln Thr Ile  Leu Ser Asn
    1415                    1420                    1425

Ile Ala  Arg Ala Gly His Ala  Ile Glu Glu Leu Tyr  Gly Ser Pro
    1430                    1435                    1440

Gln Asp  Ile Glu Gly Val Val  Arg Asp Gly Lys Ile  Tyr Val Val
    1445                    1450                    1455

Gln Thr  Arg Pro Gln Met
    1460

<210>  3
<211>  3000
<212>  DNA
<213>  Triticum aestivum


<220>
<221>  CDS
<222>  (227)..(2623)

<400>  3
ctccaccgcg gtggcggccg ctctagaact agtggatccc ccgggctgca ggaattcggc      60

acgagcttcg gcctgacccc gttcgtttac ccccacacag agcacactcc agtccagtcc     120

agcccactgc caccgcgcta ctctccactc ccactgccac cacctccgcc tgcgccgcgc     180

tctgggcgga ccaacccgcg aaccgtacca tctcccgccc cgatcc atg tcg tcg     235
                                                    Met Ser Ser
                                                     1

gcg gtc gcg tcc gcc gca tcc ttc ctc gcg ctc gcg tca gcc tcc ccc     283

```
Ala Val Ala Ser Ala Ala Ser Phe Leu Ala Leu Ala Ser Ala Ser Pro
     5                     10                15

ggg aga tca cgc agg cgg gcg agg gtg agc gcg cag cca ccc cac gcc    331
Gly Arg Ser Arg Arg Arg Ala Arg Val Ser Ala Gln Pro Pro His Ala
20                  25                30                    35

ggg gcc ggc agg ttg cac tgg ccg ccg tgg ccg ccg cag cgc acg gct    379
Gly Ala Gly Arg Leu His Trp Pro Pro Trp Pro Pro Gln Arg Thr Ala
                40                45                50

cgc gac gga gct gtg gcg gcg ctc gcc gcc ggg aag aag gac gcg ggg    427
Arg Asp Gly Ala Val Ala Ala Leu Ala Ala Gly Lys Lys Asp Ala Gly
                55                60                65

atc gac gac gcc gcc gcg tcc gtg agg cag ccc cgc gca ctc cgc ggt    475
Ile Asp Asp Ala Ala Ala Ser Val Arg Gln Pro Arg Ala Leu Arg Gly
        70                75                80

ggc gcc gcc acc aag gtc gcg gag cga agg gat ccc gtc aag acg ctc    523
Gly Ala Ala Thr Lys Val Ala Glu Arg Arg Asp Pro Val Lys Thr Leu
        85                90                95

gac cgc gac gcc gcg gaa ggc ggc ggg ccg tcc ccg ccg gca gcg agg    571
Asp Arg Asp Ala Ala Glu Gly Gly Gly Pro Ser Pro Pro Ala Ala Arg
100             105               110                   115

cag gac gcc gcc cgt ccg ccg agt atg aac ggc atg ccg gtg aac ggc    619
Gln Asp Ala Ala Arg Pro Pro Ser Met Asn Gly Met Pro Val Asn Gly
                120               125                   130

gag aac aaa tct acc ggc ggc ggc ggc gcg act aaa gac agc ggg ctg    667
Glu Asn Lys Ser Thr Gly Gly Gly Gly Ala Thr Lys Asp Ser Gly Leu
            135               140               145

ccc acg ccc gca cgc gcg ccc cat ccg tcg acc cag aac aga gca ccg    715
Pro Thr Pro Ala Arg Ala Pro His Pro Ser Thr Gln Asn Arg Ala Pro
        150               155               160

gtg aac ggt gaa aac aaa gct aac gtc gcc tcg ccg ccg acg agc ata    763
Val Asn Gly Glu Asn Lys Ala Asn Val Ala Ser Pro Pro Thr Ser Ile
        165               170               175

gcc gag gcc gcg gct tcg gat tcc gca gct acc att tcc atc agc gac    811
Ala Glu Ala Ala Ala Ser Asp Ser Ala Ala Thr Ile Ser Ile Ser Asp
180                 185               190                   195

aag gcg ccg gag tcc gtt gtc cca gct gag aag acg ccg ccg tcg tcc    859
Lys Ala Pro Glu Ser Val Val Pro Ala Glu Lys Thr Pro Pro Ser Ser
```

```
                    200                     205                     210

ggc tca aat ttc gag tcc tcg gcc tct gct ccc ggg tct gac act gtc        907
Gly Ser Asn Phe Glu Ser Ser Ala Ser Ala Pro Gly Ser Asp Thr Val
            215                     220                     225

agc gac gtg gaa caa gaa ctg aag aag ggt gcg gtc gtt gtc gaa gaa        955
Ser Asp Val Glu Gln Glu Leu Lys Lys Gly Ala Val Val Val Glu Glu
            230                     235                     240

gct cca aag cca aag gct ctt tcg ccg cct gca gcc ccc gct gta caa       1003
Ala Pro Lys Pro Lys Ala Leu Ser Pro Pro Ala Ala Pro Ala Val Gln
            245                     250                     255

gaa gac ctt tgg gat ttc aag aaa tac att ggt ttc gag gag ccc gtg       1051
Glu Asp Leu Trp Asp Phe Lys Lys Tyr Ile Gly Phe Glu Glu Pro Val
260                     265                     270                     275

gag gcc aag gat gat ggc cgg gct gtc gca gat gat gcg ggc tcc ttt       1099
Glu Ala Lys Asp Asp Gly Arg Ala Val Ala Asp Asp Ala Gly Ser Phe
                    280                     285                     290

gaa cac cac cag aat cac gac tcc gga cct ttg gca ggg gag aat gtc       1147
Glu His His Gln Asn His Asp Ser Gly Pro Leu Ala Gly Glu Asn Val
            295                     300                     305

atg aac gtg gtc gtc gtg gct gct gag tgt tct ccc tgg tgc aaa aca       1195
Met Asn Val Val Val Val Ala Ala Glu Cys Ser Pro Trp Cys Lys Thr
            310                     315                     320

ggt ggt ctg gga gat gtt gcg ggt gct ctg ccc aag gct ttg gca aag       1243
Gly Gly Leu Gly Asp Val Ala Gly Ala Leu Pro Lys Ala Leu Ala Lys
            325                     330                     335

aga gga cat cgt gtt atg gtt gtg gta cca agg tat ggg gac tat gaa       1291
Arg Gly His Arg Val Met Val Val Val Pro Arg Tyr Gly Asp Tyr Glu
340                     345                     350                     355

gaa gcc tac gat gtc gga gtc cga aaa tac tac aag gct gct gga cag       1339
Glu Ala Tyr Asp Val Gly Val Arg Lys Tyr Tyr Lys Ala Ala Gly Gln
                    360                     365                     370

gat atg gaa gtg aat tat ttc cat gct tat atc gat gga gtt gat ttt       1387
Asp Met Glu Val Asn Tyr Phe His Ala Tyr Ile Asp Gly Val Asp Phe
            375                     380                     385

gtg ttc att gac gct cct ctc ttc cga cac cgt cag gaa gac att tat       1435
Val Phe Ile Asp Ala Pro Leu Phe Arg His Arg Gln Glu Asp Ile Tyr
            390                     395                     400
```

56

```
ggg ggc agc aga cag gaa att atg aag cgc atg att ttg ttc tgc aag        1483
Gly Gly Ser Arg Gln Glu Ile Met Lys Arg Met Ile Leu Phe Cys Lys
    405             410             415

gcc gct gtt gag gtt cca tgg cac gtt cca tgc ggc ggt gtc cct tat        1531
Ala Ala Val Glu Val Pro Trp His Val Pro Cys Gly Gly Val Pro Tyr
420             425             430             435

ggg gat gga aat ctg gtg ttt att gca aat gat tgg cac acg gca ctc        1579
Gly Asp Gly Asn Leu Val Phe Ile Ala Asn Asp Trp His Thr Ala Leu
                440             445             450

ctg cct gtc tat ctg aaa gca tat tac agg gac cat ggt ttg atg cag        1627
Leu Pro Val Tyr Leu Lys Ala Tyr Tyr Arg Asp His Gly Leu Met Gln
            455             460             465

tac act cgg tcc att atg gtg ata cat aac atc gct cac cag ggc cgt        1675
Tyr Thr Arg Ser Ile Met Val Ile His Asn Ile Ala His Gln Gly Arg
            470             475             480

ggc cct gta gat gaa ttc ccg ttc acc gag ttg cct gag cac tac ctg        1723
Gly Pro Val Asp Glu Phe Pro Phe Thr Glu Leu Pro Glu His Tyr Leu
            485             490             495

gaa cac ttc aga ctg tac gac ccc gtg ggt ggt gaa cac gcc aac tac        1771
Glu His Phe Arg Leu Tyr Asp Pro Val Gly Gly Glu His Ala Asn Tyr
500             505             510             515

ttc gcc gcc ggc ctg aag atg gcg gac cag gtt gtc gtg gtg agc ccc        1819
Phe Ala Ala Gly Leu Lys Met Ala Asp Gln Val Val Val Val Ser Pro
            520             525             530

ggg tac ctg tgg gag ctg aag acg gtg gag ggc ggc tgg ggg ctt cac        1867
Gly Tyr Leu Trp Glu Leu Lys Thr Val Glu Gly Gly Trp Gly Leu His
            535             540             545

gac atc ata cgg cag aac gac tgg aag acc cgc ggc atc gtc aac ggc        1915
Asp Ile Ile Arg Gln Asn Asp Trp Lys Thr Arg Gly Ile Val Asn Gly
            550             555             560

atc gac aac atg gag tgg aac ccc gag gtg gac gcc cac ctc aag tcg        1963
Ile Asp Asn Met Glu Trp Asn Pro Glu Val Asp Ala His Leu Lys Ser
            565             570             575

gac ggc tac acc aac ttc tcc ctg agg acg ctg gac tcc ggc aag cgg        2011
Asp Gly Tyr Thr Asn Phe Ser Leu Arg Thr Leu Asp Ser Gly Lys Arg
580             585             590             595
```

```
cag tgc aag gag gcc ctg cag cgc gag ctg ggc ctg cag gtc cgc gcc      2059
Gln Cys Lys Glu Ala Leu Gln Arg Glu Leu Gly Leu Gln Val Arg Ala
                600                 605                 610

gac gtg ccg ctg ctc ggc ttc atc ggc cgc ctg gac ggg cag aag ggc      2107
Asp Val Pro Leu Leu Gly Phe Ile Gly Arg Leu Asp Gly Gln Lys Gly
                615                 620                 625

gtg gag atc atc gcg gac gcc atg ccc tgg atc gtg agc cag gac gtg      2155
Val Glu Ile Ile Ala Asp Ala Met Pro Trp Ile Val Ser Gln Asp Val
                630                 635                 640

cag ctg gtg atg ctg ggc acc ggg cgc cac gac ctg gag agc atg ctg      2203
Gln Leu Val Met Leu Gly Thr Gly Arg His Asp Leu Glu Ser Met Leu
                645                 650                 655

cag cac ttc gag cgg gag cac cac gac aag gtg cgc ggg tgg gtg ggg      2251
Gln His Phe Glu Arg Glu His His Asp Lys Val Arg Gly Trp Val Gly
660                 665                 670                 675

ttc tcc gtg cgc ctg gcg cac cgg atc acg gcg ggg gcg gac gcg ctc      2299
Phe Ser Val Arg Leu Ala His Arg Ile Thr Ala Gly Ala Asp Ala Leu
                680                 685                 690

ctc atg ccc tcc cgg ttc gag ccg tgc ggg ctg aac cag ctc tac gcc      2347
Leu Met Pro Ser Arg Phe Glu Pro Cys Gly Leu Asn Gln Leu Tyr Ala
                695                 700                 705

atg gcc tac ggc acc gtc ccc gtc gtg cac gcc gtc ggc ggc ctc agg      2395
Met Ala Tyr Gly Thr Val Pro Val Val His Ala Val Gly Gly Leu Arg
                710                 715                 720

gac acc gtg ccg ccg ttc gac ccc ttc aac cac tcc ggg ctc ggg tgg      2443
Asp Thr Val Pro Pro Phe Asp Pro Phe Asn His Ser Gly Leu Gly Trp
                725                 730                 735

acg ttc gac cgc gcc gag gcg cac aag ctg atc gag gcg ctc ggg cac      2491
Thr Phe Asp Arg Ala Glu Ala His Lys Leu Ile Glu Ala Leu Gly His
740                 745                 750                 755

tgc ctc cgc acc tac cga gac ttc aag gag agc tgg agg gcc ctc cag      2539
Cys Leu Arg Thr Tyr Arg Asp Phe Lys Glu Ser Trp Arg Ala Leu Gln
                760                 765                 770

gag cgc ggc atg tcg cag gac ttc agc tgg gag cac gcc gcc aag ctc      2587
Glu Arg Gly Met Ser Gln Asp Phe Ser Trp Glu His Ala Ala Lys Leu
                775                 780                 785

tac gag gac gtc ctc gtc aag gcc aag tac cag tgg tgaacgctag           2633
Tyr Glu Asp Val Leu Val Lys Ala Lys Tyr Gln Trp
```

```
Tyr Glu Asp Val Leu Val Lys Ala Lys Tyr Gln Trp
        790                795
```

```
ctgctagccg ctccagcccc gcatgcgtgc atgacaggat ggaactgcat tgcgcacgca     2693

ggaaagtgcc atggagcgcc ggcatccgcg aagtacagtg acatgaggtg tgtgtggttg     2753

agacgctgat tccaatccgg cccgtagcag agtagagcgg aggtatatgg gaatcttaac     2813

ttggtattgt aatttgttat gttgtgtgca ttattacaat gttgttactt attcttgtta     2873

agtcggaggc caagggcgaa agctagctca catgtctgat ggatgcacgt gccatggttg     2933

gtttggtagc gcagtgcaaa cggcaagaat gggaagtgaa ttcctccctg cttgaaaaaa     2993

aaaaaaa                                                              3000
```

```
<210>  4
<211>  799
<212>  PRT
<213>  Triticum aestivum
```

```
<400>  4
```

```
Met Ser Ser Ala Val Ala Ser Ala Ala Ser Phe Leu Ala Leu Ala Ser
1               5                  10                 15
```

```
Ala Ser Pro Gly Arg Ser Arg Arg Arg Ala Arg Val Ser Ala Gln Pro
            20                 25                 30
```

```
Pro His Ala Gly Ala Gly Arg Leu His Trp Pro Pro Trp Pro Pro Gln
            35                 40                 45
```

```
Arg Thr Ala Arg Asp Gly Ala Val Ala Ala Leu Ala Ala Gly Lys Lys
    50                 55                 60
```

```
Asp Ala Gly Ile Asp Asp Ala Ala Ala Ser Val Arg Gln Pro Arg Ala
65                 70                 75                 80
```

```
Leu Arg Gly Gly Ala Ala Thr Lys Val Ala Glu Arg Arg Asp Pro Val
                85                 90                 95
```

```
Lys Thr Leu Asp Arg Asp Ala Ala Glu Gly Gly Gly Pro Ser Pro Pro
            100                 105             110

Ala Ala Arg Gln Asp Ala Ala Arg Pro Pro Ser Met Asn Gly Met Pro
            115                 120             125

Val Asn Gly Glu Asn Lys Ser Thr Gly Gly Gly Gly Ala Thr Lys Asp
            130                 135             140

Ser Gly Leu Pro Thr Pro Ala Arg Ala Pro His Pro Ser Thr Gln Asn
145                 150                 155                 160

Arg Ala Pro Val Asn Gly Glu Asn Lys Ala Asn Val Ala Ser Pro Pro
                165                 170                 175

Thr Ser Ile Ala Glu Ala Ala Ala Ser Asp Ser Ala Ala Thr Ile Ser
                180                 185                 190

Ile Ser Asp Lys Ala Pro Glu Ser Val Val Pro Ala Glu Lys Thr Pro
            195                 200                 205

Pro Ser Ser Gly Ser Asn Phe Glu Ser Ser Ala Ser Ala Pro Gly Ser
            210                 215                 220

Asp Thr Val Ser Asp Val Glu Gln Glu Leu Lys Lys Gly Ala Val Val
225                 230                 235                 240

Val Glu Glu Ala Pro Lys Pro Lys Ala Leu Ser Pro Pro Ala Ala Pro
                245                 250                 255

Ala Val Gln Glu Asp Leu Trp Asp Phe Lys Lys Tyr Ile Gly Phe Glu
            260                 265                 270

Glu Pro Val Glu Ala Lys Asp Asp Gly Arg Ala Val Ala Asp Asp Ala
            275                 280                 285

Gly Ser Phe Glu His His Gln Asn His Asp Ser Gly Pro Leu Ala Gly
```

290                    295                    300

Glu Asn Val Met Asn Val Val Val Ala Ala Glu Cys Ser Pro Trp
305                    310                    315                    320

Cys Lys Thr Gly Gly Leu Gly Asp Val Ala Gly Ala Leu Pro Lys Ala
                    325                    330                    335

Leu Ala Lys Arg Gly His Arg Val Met Val Val Pro Arg Tyr Gly
                    340                    345                    350

Asp Tyr Glu Glu Ala Tyr Asp Val Gly Val Arg Lys Tyr Tyr Lys Ala
                    355                    360                    365

Ala Gly Gln Asp Met Glu Val Asn Tyr Phe His Ala Tyr Ile Asp Gly
                    370                    375                    380

Val Asp Phe Val Phe Ile Asp Ala Pro Leu Phe Arg His Arg Gln Glu
385                    390                    395                    400

Asp Ile Tyr Gly Gly Ser Arg Gln Glu Ile Met Lys Arg Met Ile Leu
                    405                    410                    415

Phe Cys Lys Ala Ala Val Glu Val Pro Trp His Val Pro Cys Gly Gly
                    420                    425                    430

Val Pro Tyr Gly Asp Gly Asn Leu Val Phe Ile Ala Asn Asp Trp His
                    435                    440                    445

Thr Ala Leu Leu Pro Val Tyr Leu Lys Ala Tyr Tyr Arg Asp His Gly
                    450                    455                    460

Leu Met Gln Tyr Thr Arg Ser Ile Met Val Ile His Asn Ile Ala His
465                    470                    475                    480

Gln Gly Arg Gly Pro Val Asp Glu Phe Pro Phe Thr Glu Leu Pro Glu
                    485                    490                    495

```
His Tyr Leu Glu His Phe Arg Leu Tyr Asp Pro Val Gly Gly Glu His
        500             505             510

Ala Asn Tyr Phe Ala Ala Gly Leu Lys Met Ala Asp Gln Val Val Val
        515             520             525

Val Ser Pro Gly Tyr Leu Trp Glu Leu Lys Thr Val Glu Gly Gly Trp
        530             535             540

Gly Leu His Asp Ile Ile Arg Gln Asn Asp Trp Lys Thr Arg Gly Ile
545             550             555             560

Val Asn Gly Ile Asp Asn Met Glu Trp Asn Pro Glu Val Asp Ala His
            565             570             575

Leu Lys Ser Asp Gly Tyr Thr Asn Phe Ser Leu Arg Thr Leu Asp Ser
        580             585             590

Gly Lys Arg Gln Cys Lys Glu Ala Leu Gln Arg Glu Leu Gly Leu Gln
        595             600             605

Val Arg Ala Asp Val Pro Leu Leu Gly Phe Ile Gly Arg Leu Asp Gly
        610             615             620

Gln Lys Gly Val Glu Ile Ile Ala Asp Ala Met Pro Trp Ile Val Ser
625             630             635             640

Gln Asp Val Gln Leu Val Met Leu Gly Thr Gly Arg His Asp Leu Glu
            645             650             655

Ser Met Leu Gln His Phe Glu Arg Glu His His Asp Lys Val Arg Gly
        660             665             670

Trp Val Gly Phe Ser Val Arg Leu Ala His Arg Ile Thr Ala Gly Ala
        675             680             685
```

```
Asp Ala Leu Leu Met Pro Ser Arg Phe Glu Pro Cys Gly Leu Asn Gln
    690             695             700

Leu Tyr Ala Met Ala Tyr Gly Thr Val Pro Val Val His Ala Val Gly
705             710             715             720

Gly Leu Arg Asp Thr Val Pro Pro Phe Asp Pro Phe Asn His Ser Gly
            725             730             735

Leu Gly Trp Thr Phe Asp Arg Ala Glu Ala His Lys Leu Ile Glu Ala
            740             745             750

Leu Gly His Cys Leu Arg Thr Tyr Arg Asp Phe Lys Glu Ser Trp Arg
            755             760             765

Ala Leu Gln Glu Arg Gly Met Ser Gln Asp Phe Ser Trp Glu His Ala
    770             775             780

Ala Lys Leu Tyr Glu Asp Val Leu Val Lys Ala Lys Tyr Gln Trp
785             790             795
```

```
<210>   5
<211>   2433
<212>   DNA
<213>   Oryza sativa


<220>
<221>   CDS
<222>   (1)..(2430)

<400>   5
atg tct agc gcg gtg gtt gcg tcc agc aca act ttt ctc gtc gca ctt      48
Met Ser Ser Ala Val Val Ala Ser Ser Thr Thr Phe Leu Val Ala Leu
1               5               10              15

gcc tct agc gcg agc cgg ggc ggg cca cgt agg ggg cgc gtc gtg ggc      96
Ala Ser Ser Ala Ser Arg Gly Gly Pro Arg Arg Gly Arg Val Val Gly
            20              25              30

gtg gcc gct ccc cca gcc ctc ctg tat gac ggg aga gct ggc agg cta     144
```

EP 1 950 303 A1

```
Val Ala Ala Pro Pro Ala Leu Leu Tyr Asp Gly Arg Ala Gly Arg Leu
        35              40              45

gcc ctg cgc gcc cct ccg cca ccc cgc cct aga cct agg cgc agg gat      192
Ala Leu Arg Ala Pro Pro Pro Pro Arg Pro Arg Pro Arg Arg Arg Asp
        50              55              60

gcg ggt gtt gtc agg cgg gct gat gac ggg gag aac gag gcc gca gtg      240
Ala Gly Val Val Arg Arg Ala Asp Asp Gly Glu Asn Glu Ala Ala Val
65              70              75              80

gag cgg gcc ggc gag gac gat gac gag gag gag gag ttc tcg tcc ggg      288
Glu Arg Ala Gly Glu Asp Asp Asp Glu Glu Glu Glu Phe Ser Ser Gly
                85              90              95

gcc tgg cag cca ccg cgt tca agg cgc ggt gga gtt ggc aag gtc ctc      336
Ala Trp Gln Pro Pro Arg Ser Arg Arg Gly Gly Val Gly Lys Val Leu
                100             105             110

aaa cgt cgc ggt acc gtg ccg cca gtc gga agg tac ggc tcc ggt gga      384
Lys Arg Arg Gly Thr Val Pro Pro Val Gly Arg Tyr Gly Ser Gly Gly
        115             120             125

gac gcc gct cgg gtg aga gga gcc gcg gca ccc gct cca gca ccg acg      432
Asp Ala Ala Arg Val Arg Gly Ala Ala Ala Pro Ala Pro Ala Pro Thr
    130             135             140

caa gac gca gcg tcg tct aag aat ggc gcg ctt ttg tca ggc agg gat      480
Gln Asp Ala Ala Ser Ser Lys Asn Gly Ala Leu Leu Ser Gly Arg Asp
145             150             155             160

gac gac aca cct gcc tca cgg aac gga tcg gtc gtt acc ggc gcc gac      528
Asp Asp Thr Pro Ala Ser Arg Asn Gly Ser Val Val Thr Gly Ala Asp
                165             170             175

aag cct gcc gcc gcc acg ccg ccg gtg acc ata acg aag ctc cca gcg      576
Lys Pro Ala Ala Ala Thr Pro Pro Val Thr Ile Thr Lys Leu Pro Ala
        180             185             190

ccg gac tcc ccc gtg atc ctt cca tcc gta gac aag ccg cag ccg gag      624
Pro Asp Ser Pro Val Ile Leu Pro Ser Val Asp Lys Pro Gln Pro Glu
        195             200             205

ttc gtc atc cca gac gcg acg gcg ccg gcg ccg cca ccg ccc ggt tca      672
Phe Val Ile Pro Asp Ala Thr Ala Pro Ala Pro Pro Pro Gly Ser
        210             215             220

aat ccc agg tcg tcc gct cct ctc ccc aag cct gac aat tcg gaa ttt      720
Asn Pro Arg Ser Ser Ala Pro Leu Pro Lys Pro Asp Asn Ser Glu Phe
```

64

```
        225                       230                       235                       240


        gca gag gat aag agc gca aaa gtt gtt gag agt gct ccg aag cca aag        768
        Ala Glu Asp Lys Ser Ala Lys Val Val Glu Ser Ala Pro Lys Pro Lys
                        245                       250                       255


        gcg act aga tct tcc cct att cct gcg gta gaa gag gag acg tgg gat        816
        Ala Thr Arg Ser Ser Pro Ile Pro Ala Val Glu Glu Glu Thr Trp Asp
                        260                       265                       270


        ttc aag aaa tat ttt gat ctg aac gaa ccg gac gcc gcg gag gat ggc        864
        Phe Lys Lys Tyr Phe Asp Leu Asn Glu Pro Asp Ala Ala Glu Asp Gly
                        275                       280                       285


        gat gac gat gat gac tgg gct gat tca gat gcg tca gat tct gag atc        912
        Asp Asp Asp Asp Asp Trp Ala Asp Ser Asp Ala Ser Asp Ser Glu Ile
                        290                       295                       300


        gac cag gat gac gat tcg ggt cct ttg gct ggg gag aat gtc atg aac        960
        Asp Gln Asp Asp Asp Ser Gly Pro Leu Ala Gly Glu Asn Val Met Asn
        305                       310                       315                       320


        gtg atc gtg gtg gct gct gaa tgt tct ccc tgg tgc aaa aca ggt ggg       1008
        Val Ile Val Val Ala Ala Glu Cys Ser Pro Trp Cys Lys Thr Gly Gly
                        325                       330                       335


        ctt gga gat gtt gca ggt gct tta ccc aag gct ttg gcg agg aga gga       1056
        Leu Gly Asp Val Ala Gly Ala Leu Pro Lys Ala Leu Ala Arg Arg Gly
                        340                       345                       350


        cat cgt gtt atg gtt gtc gta cca agg tac ggt gat tac gcg gaa gcc       1104
        His Arg Val Met Val Val Val Pro Arg Tyr Gly Asp Tyr Ala Glu Ala
                        355                       360                       365


        cag gat gta gga atc agg aaa tac tac aag gct gct gga cag gat ctg       1152
        Gln Asp Val Gly Ile Arg Lys Tyr Tyr Lys Ala Ala Gly Gln Asp Leu
                        370                       375                       380


        gaa gtg aaa tat ttc cat gca ttt atc gac gga gtt gat ttt gtg ttc       1200
        Glu Val Lys Tyr Phe His Ala Phe Ile Asp Gly Val Asp Phe Val Phe
        385                       390                       395                       400


        att gac gct cct ctc ttc cgt cac cgt cag gat gac atc tat ggg ggg       1248
        Ile Asp Ala Pro Leu Phe Arg His Arg Gln Asp Asp Ile Tyr Gly Gly
                        405                       410                       415


        aac aga cag gaa atc atg aag cgc atg att ctg ttt tgt aag gct gct       1296
        Asn Arg Gln Glu Ile Met Lys Arg Met Ile Leu Phe Cys Lys Ala Ala
                        420                       425                       430
```

```
gtt gag gtt cct tgg cac gtt cca tgc ggt ggt gtg ccc tat ggg gat    1344
Val Glu Val Pro Trp His Val Pro Cys Gly Gly Val Pro Tyr Gly Asp
        435             440             445

ggc aac ttg gtg ttc ctt gca aac gat tgg cac act gca ctc ctg cct    1392
Gly Asn Leu Val Phe Leu Ala Asn Asp Trp His Thr Ala Leu Leu Pro
        450             455             460

gtt tat ctg aag gca tat tac aga gac aat ggc atg atg cag tac act    1440
Val Tyr Leu Lys Ala Tyr Tyr Arg Asp Asn Gly Met Met Gln Tyr Thr
465             470             475             480

cgc tct gtc ctt gtg ata cat aat atc gct tac cag ggc cgt ggc cca    1488
Arg Ser Val Leu Val Ile His Asn Ile Ala Tyr Gln Gly Arg Gly Pro
            485             490             495

gta gat gaa ttc ccc tac atg gaa ttg ccg gag cac tac ctg gat cac    1536
Val Asp Glu Phe Pro Tyr Met Glu Leu Pro Glu His Tyr Leu Asp His
        500             505             510

ttc aag ctg tac gac ccc gtc ggc ggc gag cac gcc aac atc ttc ggc    1584
Phe Lys Leu Tyr Asp Pro Val Gly Gly Glu His Ala Asn Ile Phe Gly
        515             520             525

gcg ggc ctg aag atg gcg gac cgg gtg gtg acc gtg agc ccc ggc tac    1632
Ala Gly Leu Lys Met Ala Asp Arg Val Val Thr Val Ser Pro Gly Tyr
        530             535             540

ctc tgg gag ctg aag acg acg gag ggc ggc tgg ggc ctc cac gac atc    1680
Leu Trp Glu Leu Lys Thr Thr Glu Gly Gly Trp Gly Leu His Asp Ile
545             550             555             560

ata cgg gag aac gac tgg aag atg aac ggc atc gtg aac ggc atc gac    1728
Ile Arg Glu Asn Asp Trp Lys Met Asn Gly Ile Val Asn Gly Ile Asp
                565             570             575

tac cgg gag tgg aac ccg gag gtg gac gtg cac ctg cag tcc gac ggc    1776
Tyr Arg Glu Trp Asn Pro Glu Val Asp Val His Leu Gln Ser Asp Gly
            580             585             590

tac gcc aac tac acc gtg gcc tcg ctg gac tcc agc aag ccg cgg tgc    1824
Tyr Ala Asn Tyr Thr Val Ala Ser Leu Asp Ser Ser Lys Pro Arg Cys
            595             600             605

aag gcg gcg ctg cag cgc gag ctg ggg ctg gag gtg cgc gac gac gtg    1872
Lys Ala Ala Leu Gln Arg Glu Leu Gly Leu Glu Val Arg Asp Asp Val
        610             615             620
```

```
ccg ctg atc ggg ttc atc ggg cgg ctc gac ggg cag aaa ggt gtg gac        1920
Pro Leu Ile Gly Phe Ile Gly Arg Leu Asp Gly Gln Lys Gly Val Asp
625             630             635             640

atc atc ggc gac gcg atg ccg tgg atc gcc ggg cag gac gtg cag ctg        1968
Ile Ile Gly Asp Ala Met Pro Trp Ile Ala Gly Gln Asp Val Gln Leu
                645             650             655

gtg ctg ctg ggc tcc ggc cgc cgc gac ctg gag gtg atg ctg cag cgg        2016
Val Leu Leu Gly Ser Gly Arg Arg Asp Leu Glu Val Met Leu Gln Arg
            660             665             670

ttc gag gcg cag cac aac agc aag gtg cgc ggg tgg gtg ggg ttc tcg        2064
Phe Glu Ala Gln His Asn Ser Lys Val Arg Gly Trp Val Gly Phe Ser
            675             680             685

gtg aag atg gcg cac cgg atc acg gcg ggc gcc gac gtg ctg gtc atg        2112
Val Lys Met Ala His Arg Ile Thr Ala Gly Ala Asp Val Leu Val Met
            690             695             700

ccg tcg cgg ttc gag ccg tgc ggc ctc aac cag ctc tac gcc atg gcg        2160
Pro Ser Arg Phe Glu Pro Cys Gly Leu Asn Gln Leu Tyr Ala Met Ala
705             710             715             720

tac ggc acc gtc ccc gtc gtg cac gcc gtc ggc ggg ctg agg gac acc        2208
Tyr Gly Thr Val Pro Val Val His Ala Val Gly Gly Leu Arg Asp Thr
                725             730             735

gtg tcg gcg ttc gac ccg ttc gag gac acc ggc ctc ggg tgg acg ttc        2256
Val Ser Ala Phe Asp Pro Phe Glu Asp Thr Gly Leu Gly Trp Thr Phe
                740             745             750

gac cgc gcc gag ccg cac aag ctc atc gag gcg ctc ggc cac tgc ctg        2304
Asp Arg Ala Glu Pro His Lys Leu Ile Glu Ala Leu Gly His Cys Leu
            755             760             765

gag acg tac cgc aag tac aag gag agc tgg agg ggg ctc cag gtg cgc        2352
Glu Thr Tyr Arg Lys Tyr Lys Glu Ser Trp Arg Gly Leu Gln Val Arg
            770             775             780

ggc atg tcg cag gac ctc agc tgg gac cac gcc gcc gag ctc tac gag        2400
Gly Met Ser Gln Asp Leu Ser Trp Asp His Ala Ala Glu Leu Tyr Glu
785             790             795             800

gag gtc ctt gtc aag gcc aag tac caa tgg tga                            2433
Glu Val Leu Val Lys Ala Lys Tyr Gln Trp
                805             810
```

67

```
<210>    6
<211>    810
<212>    PRT
<213>    Oryza sativa

<400>    6
```

Met Ser Ser Ala Val Val Ala Ser Ser Thr Thr Phe Leu Val Ala Leu
1               5                   10                  15


Ala Ser Ser Ala Ser Arg Gly Gly Pro Arg Arg Gly Arg Val Val Gly
                20                  25                  30


Val Ala Ala Pro Pro Ala Leu Leu Tyr Asp Gly Arg Ala Gly Arg Leu
                35                  40                  45


Ala Leu Arg Ala Pro Pro Pro Pro Arg Pro Arg Pro Arg Arg Arg Asp
        50                  55                  60


Ala Gly Val Val Arg Arg Ala Asp Asp Gly Glu Asn Glu Ala Ala Val
65                  70                  75                  80


Glu Arg Ala Gly Glu Asp Asp Asp Glu Glu Glu Glu Phe Ser Ser Gly
                85                  90                  95


Ala Trp Gln Pro Pro Arg Ser Arg Arg Gly Gly Val Gly Lys Val Leu
                100                 105                 110


Lys Arg Arg Gly Thr Val Pro Pro Val Gly Arg Tyr Gly Ser Gly Gly
        115                 120                 125


Asp Ala Ala Arg Val Arg Gly Ala Ala Ala Pro Ala Pro Ala Pro Thr
        130                 135                 140


Gln Asp Ala Ala Ser Ser Lys Asn Gly Ala Leu Leu Ser Gly Arg Asp
145                 150                 155                 160


Asp Asp Thr Pro Ala Ser Arg Asn Gly Ser Val Val Thr Gly Ala Asp
                165                 170                 175

```
Lys Pro Ala Ala Ala Thr Pro Pro Val Thr Ile Thr Lys Leu Pro Ala
            180             185             190

Pro Asp Ser Pro Val Ile Leu Pro Ser Val Asp Lys Pro Gln Pro Glu
            195             200             205

Phe Val Ile Pro Asp Ala Thr Ala Pro Ala Pro Pro Pro Pro Gly Ser
    210             215             220

Asn Pro Arg Ser Ser Ala Pro Leu Pro Lys Pro Asp Asn Ser Glu Phe
225             230             235             240

Ala Glu Asp Lys Ser Ala Lys Val Val Glu Ser Ala Pro Lys Pro Lys
            245             250             255

Ala Thr Arg Ser Ser Pro Ile Pro Ala Val Glu Glu Glu Thr Trp Asp
            260             265             270

Phe Lys Lys Tyr Phe Asp Leu Asn Glu Pro Asp Ala Ala Glu Asp Gly
            275             280             285

Asp Asp Asp Asp Asp Trp Ala Asp Ser Asp Ala Ser Asp Ser Glu Ile
            290             295             300

Asp Gln Asp Asp Asp Ser Gly Pro Leu Ala Gly Glu Asn Val Met Asn
305             310             315             320

Val Ile Val Val Ala Ala Glu Cys Ser Pro Trp Cys Lys Thr Gly Gly
            325             330             335

Leu Gly Asp Val Ala Gly Ala Leu Pro Lys Ala Leu Ala Arg Arg Gly
            340             345             350

His Arg Val Met Val Val Val Pro Arg Tyr Gly Asp Tyr Ala Glu Ala
            355             360             365
```

```
Gln Asp Val Gly Ile Arg Lys Tyr Tyr Lys Ala Ala Gly Gln Asp Leu
    370             375             380

Glu Val Lys Tyr Phe His Ala Phe Ile Asp Gly Val Asp Phe Val Phe
385             390             395             400

Ile Asp Ala Pro Leu Phe Arg His Arg Gln Asp Asp Ile Tyr Gly Gly
                405             410             415

Asn Arg Gln Glu Ile Met Lys Arg Met Ile Leu Phe Cys Lys Ala Ala
            420             425             430

Val Glu Val Pro Trp His Val Pro Cys Gly Gly Val Pro Tyr Gly Asp
        435             440             445

Gly Asn Leu Val Phe Leu Ala Asn Asp Trp His Thr Ala Leu Leu Pro
    450             455             460

Val Tyr Leu Lys Ala Tyr Tyr Arg Asp Asn Gly Met Met Gln Tyr Thr
465             470             475             480

Arg Ser Val Leu Val Ile His Asn Ile Ala Tyr Gln Gly Arg Gly Pro
            485             490             495

Val Asp Glu Phe Pro Tyr Met Glu Leu Pro Glu His Tyr Leu Asp His
            500             505             510

Phe Lys Leu Tyr Asp Pro Val Gly Gly Glu His Ala Asn Ile Phe Gly
        515             520             525

Ala Gly Leu Lys Met Ala Asp Arg Val Val Thr Val Ser Pro Gly Tyr
    530             535             540

Leu Trp Glu Leu Lys Thr Thr Glu Gly Gly Trp Gly Leu His Asp Ile
545             550             555             560
```

```
Ile Arg Glu Asn Asp Trp Lys Met Asn Gly Ile Val Asn Gly Ile Asp
            565             570             575

Tyr Arg Glu Trp Asn Pro Glu Val Asp Val His Leu Gln Ser Asp Gly
            580             585             590

Tyr Ala Asn Tyr Thr Val Ala Ser Leu Asp Ser Ser Lys Pro Arg Cys
            595             600             605

Lys Ala Ala Leu Gln Arg Glu Leu Gly Leu Glu Val Arg Asp Asp Val
        610             615             620

Pro Leu Ile Gly Phe Ile Gly Arg Leu Asp Gly Gln Lys Gly Val Asp
625             630             635             640

Ile Ile Gly Asp Ala Met Pro Trp Ile Ala Gly Gln Asp Val Gln Leu
            645             650             655

Val Leu Leu Gly Ser Gly Arg Arg Asp Leu Glu Val Met Leu Gln Arg
            660             665             670

Phe Glu Ala Gln His Asn Ser Lys Val Arg Gly Trp Val Gly Phe Ser
            675             680             685

Val Lys Met Ala His Arg Ile Thr Ala Gly Ala Asp Val Leu Val Met
        690             695             700

Pro Ser Arg Phe Glu Pro Cys Gly Leu Asn Gln Leu Tyr Ala Met Ala
705             710             715             720

Tyr Gly Thr Val Pro Val Val His Ala Val Gly Gly Leu Arg Asp Thr
            725             730             735

Val Ser Ala Phe Asp Pro Phe Glu Asp Thr Gly Leu Gly Trp Thr Phe
            740             745             750

Asp Arg Ala Glu Pro His Lys Leu Ile Glu Ala Leu Gly His Cys Leu
```

755      760      765

Glu Thr Tyr Arg Lys Tyr Lys Glu Ser Trp Arg Gly Leu Gln Val Arg
770      775      780

Gly Met Ser Gln Asp Leu Ser Trp Asp His Ala Ala Glu Leu Tyr Glu
785      790      795      800

Glu Val Leu Val Lys Ala Lys Tyr Gln Trp
805      810

<210> 7
<211> 1830
<212> DNA
<213> Oryza sativa

<220>
<221> CDS
<222> (1)..(1827)

<400> 7
```
atg tcg gct ctc acc acg tcc cag ctc gcc acc tcg gcc acc ggc ttc     48
Met Ser Ala Leu Thr Thr Ser Gln Leu Ala Thr Ser Ala Thr Gly Phe
1               5                   10                  15

ggc atc gcc gac agg tcg gcg ccg tcg tcg ctg ctc cgc cac ggg ttc     96
Gly Ile Ala Asp Arg Ser Ala Pro Ser Ser Leu Leu Arg His Gly Phe
                20                  25                  30

cag ggc ctc aag ccc cgc agc ccc gcc ggc ggc gac gcg acg tcg ctc    144
Gln Gly Leu Lys Pro Arg Ser Pro Ala Gly Gly Asp Ala Thr Ser Leu
            35                  40                  45

agc gtg acg acc agc gcg cgc gcg acg ccc aag cag cag cgg tcg gtg    192
Ser Val Thr Thr Ser Ala Arg Ala Thr Pro Lys Gln Gln Arg Ser Val
        50                  55                  60

cag cgt ggc agc cgg agg ttc ccc tcc gtc gtc gtg tac gcc acc ggc    240
Gln Arg Gly Ser Arg Arg Phe Pro Ser Val Val Val Tyr Ala Thr Gly
65                  70                  75                  80

gcc ggc atg aac gtc gtg ttc gtc ggc gcc gag atg gcc ccc tgg agc    288
Ala Gly Met Asn Val Val Phe Val Gly Ala Glu Met Ala Pro Trp Ser
                85                  90                  95
```

```
aag acc ggc ggc ctc ggt gac gtc ctc ggt ggc ctc ccc cct gcc atg      336
Lys Thr Gly Gly Leu Gly Asp Val Leu Gly Gly Leu Pro Pro Ala Met
        100             105             110

gct gcg aat ggc cac agg gtc atg gtg atc tct cct cgg tac gac cag      384
Ala Ala Asn Gly His Arg Val Met Val Ile Ser Pro Arg Tyr Asp Gln
        115             120             125

tac aag gac gct tgg gat acc agc gtt gtg gct gag atc aag gtt gca      432
Tyr Lys Asp Ala Trp Asp Thr Ser Val Val Ala Glu Ile Lys Val Ala
        130             135             140

gac agg tac gag agg gtg agg ttt ttc cat tgc tac aag cgt gga gtc      480
Asp Arg Tyr Glu Arg Val Arg Phe Phe His Cys Tyr Lys Arg Gly Val
145             150             155             160

gac cgt gtg ttc atc gac cat ccg tca ttc ctg gag aag gtt tgg gga      528
Asp Arg Val Phe Ile Asp His Pro Ser Phe Leu Glu Lys Val Trp Gly
            165             170             175

aag acc ggt gag aag atc tac gga cct gac act gga gtt gat tac aaa      576
Lys Thr Gly Glu Lys Ile Tyr Gly Pro Asp Thr Gly Val Asp Tyr Lys
        180             185             190

gac aac cag atg cgt ttc agc ctt ctt tgc cag gca gca ctc gag gct      624
Asp Asn Gln Met Arg Phe Ser Leu Leu Cys Gln Ala Ala Leu Glu Ala
        195             200             205

cct agg atc cta aac ctc aac aac aac cca tac ttc aaa gga act tat      672
Pro Arg Ile Leu Asn Leu Asn Asn Asn Pro Tyr Phe Lys Gly Thr Tyr
        210             215             220

ggt gag gat gtt gtg ttc gtc tgc aac gac tgg cac act ggc cca ctg      720
Gly Glu Asp Val Val Phe Val Cys Asn Asp Trp His Thr Gly Pro Leu
225             230             235             240

gcg agc tac ctg aag aac aac tac cag ccc aat ggc atc tac agg aat      768
Ala Ser Tyr Leu Lys Asn Asn Tyr Gln Pro Asn Gly Ile Tyr Arg Asn
        245             250             255

gca aag gtt gct ttc tgc atc cac aac atc tcc tac cag ggc cgt ttc      816
Ala Lys Val Ala Phe Cys Ile His Asn Ile Ser Tyr Gln Gly Arg Phe
        260             265             270

gct ttc gag gat tac cct gag ctg aac ctc tcc gag agg ttc agg tca      864
Ala Phe Glu Asp Tyr Pro Glu Leu Asn Leu Ser Glu Arg Phe Arg Ser
        275             280             285
```

73

```
tcc ttc gat ttc atc gac ggg tat gac acg ccg gtg gag ggc agg aag      912
Ser Phe Asp Phe Ile Asp Gly Tyr Asp Thr Pro Val Glu Gly Arg Lys
    290             295             300

atc aac tgg atg aag gcc gga atc ctg gaa gcc gac agg gtg ctc acc      960
Ile Asn Trp Met Lys Ala Gly Ile Leu Glu Ala Asp Arg Val Leu Thr
305             310             315             320

gtg agc ccg tac tac gcc gag gag ctc atc tcc ggc atc gcc agg gga     1008
Val Ser Pro Tyr Tyr Ala Glu Glu Leu Ile Ser Gly Ile Ala Arg Gly
                325             330             335

tgc gag ctc gac aac atc atg cgg ctc acc ggc atc acc ggc atc gtc     1056
Cys Glu Leu Asp Asn Ile Met Arg Leu Thr Gly Ile Thr Gly Ile Val
            340             345             350

aac ggc atg gac gtc agc gag tgg gat ccc agc aag gac aag tac atc     1104
Asn Gly Met Asp Val Ser Glu Trp Asp Pro Ser Lys Asp Lys Tyr Ile
            355             360             365

acc gcc aag tac gac gca acc acg gca atc gag gcg aag gcg ctg aac     1152
Thr Ala Lys Tyr Asp Ala Thr Thr Ala Ile Glu Ala Lys Ala Leu Asn
    370             375             380

aag gag gcg ttg cag gcg gag gcg ggt ctt ccg gtc gac agg aaa atc     1200
Lys Glu Ala Leu Gln Ala Glu Ala Gly Leu Pro Val Asp Arg Lys Ile
385             390             395             400

cca ctg atc gcg ttc atc ggc agg ctg gag gaa cag aag ggc tct gac     1248
Pro Leu Ile Ala Phe Ile Gly Arg Leu Glu Glu Gln Lys Gly Ser Asp
                405             410             415

gtc atg gcc gcc gcc atc ccg gag ctc atg cag gag gac gtc cag atc     1296
Val Met Ala Ala Ala Ile Pro Glu Leu Met Gln Glu Asp Val Gln Ile
            420             425             430

gtt ctt ctg ggt act gga aag aag aag ttc gag aag ctg ctc aag agc     1344
Val Leu Leu Gly Thr Gly Lys Lys Lys Phe Glu Lys Leu Leu Lys Ser
            435             440             445

atg gag gag aag tat ccg ggc aag gtg agg gcc gtg gtg aag ttc aac     1392
Met Glu Glu Lys Tyr Pro Gly Lys Val Arg Ala Val Val Lys Phe Asn
    450             455             460

gcg ccg ctt gct cat ctc atc atg gcc gga gcc gac gtg ctc gcc gtc     1440
Ala Pro Leu Ala His Leu Ile Met Ala Gly Ala Asp Val Leu Ala Val
465             470             475             480

ccc agc cgc ttc gag ccc tgt gga ctc atc cag ctg cag ggg atg aga     1488
```

74

```
Pro Ser Arg Phe Glu Pro Cys Gly Leu Ile Gln Leu Gln Gly Met Arg
            485             490             495
```

```
tac gga acg ccc tgt gct tgc gcg tcc acc ggt ggg ctc gtg gac acg      1536
Tyr Gly Thr Pro Cys Ala Cys Ala Ser Thr Gly Gly Leu Val Asp Thr
            500             505             510
```

```
gtc atc gaa ggc aag act ggt ttc cac atg ggc cgt ctc agc gtc gac      1584
Val Ile Glu Gly Lys Thr Gly Phe His Met Gly Arg Leu Ser Val Asp
            515             520             525
```

```
tgc aag gtg gtg gag cca agc gac gtg aag aag gtg gcg gcc acc ctg      1632
Cys Lys Val Val Glu Pro Ser Asp Val Lys Lys Val Ala Ala Thr Leu
            530             535             540
```

```
aag cgc gcc atc aag gtc gtc ggc acg ccg gcg tac gag gag atg gtc      1680
Lys Arg Ala Ile Lys Val Val Gly Thr Pro Ala Tyr Glu Glu Met Val
545             550             555             560
```

```
agg aac tgc atg aac cag gac ctc tcc tgg aag ggg cct gcg aag aac      1728
Arg Asn Cys Met Asn Gln Asp Leu Ser Trp Lys Gly Pro Ala Lys Asn
            565             570             575
```

```
tgg gag aat gtg ctc ctg ggc ctg ggc gtc gcc ggc agc gcg ccg ggg      1776
Trp Glu Asn Val Leu Leu Gly Leu Gly Val Ala Gly Ser Ala Pro Gly
            580             585             590
```

```
atc gaa ggc gac gag atc gcg ccg ctc gcc aag gag aac gtg gct gct      1824
Ile Glu Gly Asp Glu Ile Ala Pro Leu Ala Lys Glu Asn Val Ala Ala
            595             600             605
```

```
cct tga                                                               1830
Pro
```

```
<210>  8
<211>  609
<212>  PRT
<213>  Oryza sativa

<400>  8
```

```
Met Ser Ala Leu Thr Thr Ser Gln Leu Ala Thr Ser Ala Thr Gly Phe
1               5               10              15
```

```
Gly Ile Ala Asp Arg Ser Ala Pro Ser Ser Leu Leu Arg His Gly Phe
            20              25              30
```

```
Gln Gly Leu Lys Pro Arg Ser Pro Ala Gly Gly Asp Ala Thr Ser Leu
         35              40              45


Ser Val Thr Thr Ser Ala Arg Ala Thr Pro Lys Gln Gln Arg Ser Val
         50              55              60


Gln Arg Gly Ser Arg Arg Phe Pro Ser Val Val Val Tyr Ala Thr Gly
65              70              75              80


Ala Gly Met Asn Val Val Phe Val Gly Ala Glu Met Ala Pro Trp Ser
                 85              90              95


Lys Thr Gly Gly Leu Gly Asp Val Leu Gly Gly Leu Pro Pro Ala Met
             100             105             110


Ala Ala Asn Gly His Arg Val Met Val Ile Ser Pro Arg Tyr Asp Gln
         115             120             125


Tyr Lys Asp Ala Trp Asp Thr Ser Val Val Ala Glu Ile Lys Val Ala
         130             135             140


Asp Arg Tyr Glu Arg Val Arg Phe Phe His Cys Tyr Lys Arg Gly Val
145             150             155             160


Asp Arg Val Phe Ile Asp His Pro Ser Phe Leu Glu Lys Val Trp Gly
                 165             170             175


Lys Thr Gly Glu Lys Ile Tyr Gly Pro Asp Thr Gly Val Asp Tyr Lys
             180             185             190


Asp Asn Gln Met Arg Phe Ser Leu Leu Cys Gln Ala Ala Leu Glu Ala
         195             200             205


Pro Arg Ile Leu Asn Leu Asn Asn Asn Pro Tyr Phe Lys Gly Thr Tyr
         210             215             220
```

76

Gly Glu Asp Val Val Phe Val Cys Asn Asp Trp His Thr Gly Pro Leu
225             230             235             240

Ala Ser Tyr Leu Lys Asn Asn Tyr Gln Pro Asn Gly Ile Tyr Arg Asn
                245             250             255

Ala Lys Val Ala Phe Cys Ile His Asn Ile Ser Tyr Gln Gly Arg Phe
            260             265             270

Ala Phe Glu Asp Tyr Pro Glu Leu Asn Leu Ser Glu Arg Phe Arg Ser
            275             280             285

Ser Phe Asp Phe Ile Asp Gly Tyr Asp Thr Pro Val Glu Gly Arg Lys
        290             295             300

Ile Asn Trp Met Lys Ala Gly Ile Leu Glu Ala Asp Arg Val Leu Thr
305             310             315             320

Val Ser Pro Tyr Tyr Ala Glu Glu Leu Ile Ser Gly Ile Ala Arg Gly
            325             330             335

Cys Glu Leu Asp Asn Ile Met Arg Leu Thr Gly Ile Thr Gly Ile Val
            340             345             350

Asn Gly Met Asp Val Ser Glu Trp Asp Pro Ser Lys Asp Lys Tyr Ile
            355             360             365

Thr Ala Lys Tyr Asp Ala Thr Thr Ala Ile Glu Ala Lys Ala Leu Asn
            370             375             380

Lys Glu Ala Leu Gln Ala Glu Ala Gly Leu Pro Val Asp Arg Lys Ile
385             390             395             400

Pro Leu Ile Ala Phe Ile Gly Arg Leu Glu Glu Gln Lys Gly Ser Asp
                405             410             415

```
Val Met Ala Ala Ala Ile Pro Glu Leu Met Gln Glu Asp Val Gln Ile
        420                 425                 430

Val Leu Leu Gly Thr Gly Lys Lys Lys Phe Glu Lys Leu Leu Lys Ser
        435                 440                 445

Met Glu Glu Lys Tyr Pro Gly Lys Val Arg Ala Val Val Lys Phe Asn
    450                 455                 460

Ala Pro Leu Ala His Leu Ile Met Ala Gly Ala Asp Val Leu Ala Val
465                 470                 475                 480

Pro Ser Arg Phe Glu Pro Cys Gly Leu Ile Gln Leu Gln Gly Met Arg
            485                 490                 495

Tyr Gly Thr Pro Cys Ala Cys Ala Ser Thr Gly Gly Leu Val Asp Thr
            500                 505                 510

Val Ile Glu Gly Lys Thr Gly Phe His Met Gly Arg Leu Ser Val Asp
        515                 520                 525

Cys Lys Val Val Glu Pro Ser Asp Val Lys Lys Val Ala Ala Thr Leu
    530                 535                 540

Lys Arg Ala Ile Lys Val Val Gly Thr Pro Ala Tyr Glu Glu Met Val
545                 550                 555                 560

Arg Asn Cys Met Asn Gln Asp Leu Ser Trp Lys Gly Pro Ala Lys Asn
            565                 570                 575

Trp Glu Asn Val Leu Leu Gly Leu Gly Val Ala Gly Ser Ala Pro Gly
            580                 585                 590

Ile Glu Gly Asp Glu Ile Ala Pro Leu Ala Lys Glu Asn Val Ala Ala
        595                 600                 605

Pro
```

```
<210>   9
<211>   1818
<212>   DNA
<213>   Triticum aestivum


<220>
<221>   CDS
<222>   (1)..(1815)


<400>   9
atg gcg gct ctg gtc acg tcg cag ctc gcc acc tcc ggc acc gtc ctc        48
Met Ala Ala Leu Val Thr Ser Gln Leu Ala Thr Ser Gly Thr Val Leu
1               5                   10                  15


ggc atc acc gac agg ttc cgg cgt gca ggt ttt cag ggt gtg agg ccc        96
Gly Ile Thr Asp Arg Phe Arg Arg Ala Gly Phe Gln Gly Val Arg Pro
                20                  25                  30


cgg agc ccg gca gat gcg ccg ctc ggc atg agg act acc gga gcg agc       144
Arg Ser Pro Ala Asp Ala Pro Leu Gly Met Arg Thr Thr Gly Ala Ser
            35                  40                  45


gcc gcc ccg aag caa caa agc cgg aaa gcg cac cgc ggg acc cgg cgg       192
Ala Ala Pro Lys Gln Gln Ser Arg Lys Ala His Arg Gly Thr Arg Arg
        50                  55                  60


tgc ctc tcc atg gtg gtg cgc gcc acg ggc agc gcc ggc atg aac ctc       240
Cys Leu Ser Met Val Val Arg Ala Thr Gly Ser Ala Gly Met Asn Leu
65                  70                  75                  80


gtg ttc gtc ggc gcc gag atg gcg ccc tgg agc aag acc ggc ggc ctc       288
Val Phe Val Gly Ala Glu Met Ala Pro Trp Ser Lys Thr Gly Gly Leu
                85                  90                  95


ggc gac gtc ctc ggg ggc ctc ccc cca gcc atg gcc gcc aac ggt cac       336
Gly Asp Val Leu Gly Gly Leu Pro Pro Ala Met Ala Ala Asn Gly His
            100                 105                 110


cgg gtc atg gtc atc tcc ccg cgc tac gac cag tac aag gac gcc tgg       384
Arg Val Met Val Ile Ser Pro Arg Tyr Asp Gln Tyr Lys Asp Ala Trp
            115                 120                 125


gac acc agc gtc gtc tcc gag atc aag gtc gcg gac gag tac gag agg       432
Asp Thr Ser Val Val Ser Glu Ile Lys Val Ala Asp Glu Tyr Glu Arg
        130                 135                 140
```

```
gtg agg tac ttc cac tgc tac aag cgc ggg gtg gac cgc gtg ttc gtc          480
Val Arg Tyr Phe His Cys Tyr Lys Arg Gly Val Asp Arg Val Phe Val
145             150             155             160

gac cac ccg tgc ttc ctg gag aag gtc cgg ggc aag acc aag gag aag          528
Asp His Pro Cys Phe Leu Glu Lys Val Arg Gly Lys Thr Lys Glu Lys
                165             170             175

atc tac ggg ccc gat gcc ggc acg gac tac gag gac aac cag cta cgc          576
Ile Tyr Gly Pro Asp Ala Gly Thr Asp Tyr Glu Asp Asn Gln Leu Arg
            180             185             190

ttc agc ctg ctc tgc cag gca gcg ctt gag gca ccc agg atc ctc gac          624
Phe Ser Leu Leu Cys Gln Ala Ala Leu Glu Ala Pro Arg Ile Leu Asp
            195             200             205

ctc aac aac aac cca tac ttc tcc gga ccc tac ggg gaa gac gtg gtg          672
Leu Asn Asn Asn Pro Tyr Phe Ser Gly Pro Tyr Gly Glu Asp Val Val
        210             215             220

ttc gtg tgc aac gac tgg cac acg ggc ctt ctg gcc tgc tac ctc aag          720
Phe Val Cys Asn Asp Trp His Thr Gly Leu Leu Ala Cys Tyr Leu Lys
225             230             235             240

agc aac tac cag tcc agt ggc atc tat agg acg gcc aag gta gcg ttc          768
Ser Asn Tyr Gln Ser Ser Gly Ile Tyr Arg Thr Ala Lys Val Ala Phe
                245             250             255

tgc atc cac aac atc tcg tat cag ggc cgc ttc tcc ttc gac gac ttc          816
Cys Ile His Asn Ile Ser Tyr Gln Gly Arg Phe Ser Phe Asp Asp Phe
            260             265             270

gcg cag ctc aac ctg ccc gac agg ttc aag tcg tcc ttc gac ttc atc          864
Ala Gln Leu Asn Leu Pro Asp Arg Phe Lys Ser Ser Phe Asp Phe Ile
            275             280             285

gac ggc tac gac aag ccg gtg gag ggg cgc aag atc aac tgg atg aag          912
Asp Gly Tyr Asp Lys Pro Val Glu Gly Arg Lys Ile Asn Trp Met Lys
        290             295             300

gcc ggg atc ctg cag gcc gac aag gtg ctc acg gtg agc ccc tac tac          960
Ala Gly Ile Leu Gln Ala Asp Lys Val Leu Thr Val Ser Pro Tyr Tyr
305             310             315             320

gcg gag gag ctc atc tcc ggc gaa gcc agg ggc tgc gag ctc gac aac         1008
Ala Glu Glu Leu Ile Ser Gly Glu Ala Arg Gly Cys Glu Leu Asp Asn
                325             330             335
```

```
atc atg cgc ctc acg ggc atc acc ggc atc gtc aac ggc atg gac gtc    1056
Ile Met Arg Leu Thr Gly Ile Thr Gly Ile Val Asn Gly Met Asp Val
            340             345             350

agc gag tgg gac ccc gcc aag gac aag ttc ctc gcc gcc aac tac gac    1104
Ser Glu Trp Asp Pro Ala Lys Asp Lys Phe Leu Ala Ala Asn Tyr Asp
            355             360             365

gtc acc acc gcg ttg gag ggg aag gcg ctg aac aag gag gcg ctg cag    1152
Val Thr Thr Ala Leu Glu Gly Lys Ala Leu Asn Lys Glu Ala Leu Gln
            370             375             380

gcc gag gtg ggg ctg ccg gtg gac cgg aag gtg ccc ctg gtg gcc ttc    1200
Ala Glu Val Gly Leu Pro Val Asp Arg Lys Val Pro Leu Val Ala Phe
385             390             395             400

atc ggc agg ctg gag gag cag aag ggc ccc gac gtg atg atc gcc gcc    1248
Ile Gly Arg Leu Glu Glu Gln Lys Gly Pro Asp Val Met Ile Ala Ala
            405             410             415

atc ccg gag atc ttg aag gag gag gac gtc cag atc gtt ctc ctg ggc    1296
Ile Pro Glu Ile Leu Lys Glu Glu Asp Val Gln Ile Val Leu Leu Gly
            420             425             430

acc ggg aag aag aag ttt gag cgg ctg ctc aag agc gtg gag gag aag    1344
Thr Gly Lys Lys Lys Phe Glu Arg Leu Leu Lys Ser Val Glu Glu Lys
            435             440             445

ttc ccg agc aag gtg agg gcc gtg gtc agg ttc aac gcg ccg ctg gct    1392
Phe Pro Ser Lys Val Arg Ala Val Val Arg Phe Asn Ala Pro Leu Ala
            450             455             460

cac cag atg atg gcc ggc gcc gac gtg ctc gcc gtc acc agc cgc ttc    1440
His Gln Met Met Ala Gly Ala Asp Val Leu Ala Val Thr Ser Arg Phe
465             470             475             480

gag ccc tgc ggc ctc atc cag ctc cag ggg atg cgc tac gga acg ccg    1488
Glu Pro Cys Gly Leu Ile Gln Leu Gln Gly Met Arg Tyr Gly Thr Pro
            485             490             495

tgc gcg tgc gcg tcc acc ggc ggg ctc gtc gac acg atc atg gag ggc    1536
Cys Ala Cys Ala Ser Thr Gly Gly Leu Val Asp Thr Ile Met Glu Gly
            500             505             510

aag acc ggg ttc cac atg ggc cgc ctc agc gtc gac tgc aac gtg gtg    1584
Lys Thr Gly Phe His Met Gly Arg Leu Ser Val Asp Cys Asn Val Val
            515             520             525

gag ccg gcc gac gtg aag aag gtg gtg acc acc ctg aag cgc gcc gtc    1632
```

```
Glu Pro Ala Asp Val Lys Lys Val Val Thr Thr Leu Lys Arg Ala Val
    530             535             540

aag gtc gtc ggc acg cca gcc tac cat gag atg gtc aag aac tgc atg    1680
Lys Val Val Gly Thr Pro Ala Tyr His Glu Met Val Lys Asn Cys Met
545             550             555             560

atc cag gat ctc tcc tgg aag ggg cca gcc aag aac tgg gag gac gtg    1728
Ile Gln Asp Leu Ser Trp Lys Gly Pro Ala Lys Asn Trp Glu Asp Val
                565             570             575

ctt ctg gaa ctg ggg gtc gag ggg agc gag cca ggg gtc atc ggc gag    1776
Leu Leu Glu Leu Gly Val Glu Gly Ser Glu Pro Gly Val Ile Gly Glu
            580             585             590

gag att gcg ccg ctc gcc atg gag aac gtc gcc gct ccc tga            1818
Glu Ile Ala Pro Leu Ala Met Glu Asn Val Ala Ala Pro
        595             600             605
```

```
<210>  10
<211>  605
<212>  PRT
<213>  Triticum aestivum

<400>  10
```

```
Met Ala Ala Leu Val Thr Ser Gln Leu Ala Thr Ser Gly Thr Val Leu
1               5               10              15

Gly Ile Thr Asp Arg Phe Arg Arg Ala Gly Phe Gln Gly Val Arg Pro
            20              25              30

Arg Ser Pro Ala Asp Ala Pro Leu Gly Met Arg Thr Thr Gly Ala Ser
            35              40              45

Ala Ala Pro Lys Gln Gln Ser Arg Lys Ala His Arg Gly Thr Arg Arg
        50              55              60

Cys Leu Ser Met Val Val Arg Ala Thr Gly Ser Ala Gly Met Asn Leu
65              70              75              80

Val Phe Val Gly Ala Glu Met Ala Pro Trp Ser Lys Thr Gly Gly Leu
                85              90              95
```

Gly Asp Val Leu Gly Gly Leu Pro Pro Ala Met Ala Ala Asn Gly His
            100             105                 110

Arg Val Met Val Ile Ser Pro Arg Tyr Asp Gln Tyr Lys Asp Ala Trp
            115             120                 125

Asp Thr Ser Val Val Ser Glu Ile Lys Val Ala Asp Glu Tyr Glu Arg
            130             135                 140

Val Arg Tyr Phe His Cys Tyr Lys Arg Gly Val Asp Arg Val Phe Val
145                 150             155                 160

Asp His Pro Cys Phe Leu Glu Lys Val Arg Gly Lys Thr Lys Glu Lys
                165             170                 175

Ile Tyr Gly Pro Asp Ala Gly Thr Asp Tyr Glu Asp Asn Gln Leu Arg
                180             185                 190

Phe Ser Leu Leu Cys Gln Ala Ala Leu Glu Ala Pro Arg Ile Leu Asp
            195             200                 205

Leu Asn Asn Asn Pro Tyr Phe Ser Gly Pro Tyr Gly Glu Asp Val Val
            210             215                 220

Phe Val Cys Asn Asp Trp His Thr Gly Leu Leu Ala Cys Tyr Leu Lys
225                 230             235                 240

Ser Asn Tyr Gln Ser Ser Gly Ile Tyr Arg Thr Ala Lys Val Ala Phe
                245             250                 255

Cys Ile His Asn Ile Ser Tyr Gln Gly Arg Phe Ser Phe Asp Asp Phe
                260             265                 270

Ala Gln Leu Asn Leu Pro Asp Arg Phe Lys Ser Ser Phe Asp Phe Ile
            275             280                 285

83

```
Asp Gly Tyr Asp Lys Pro Val Glu Gly Arg Lys Ile Asn Trp Met Lys
    290             295             300

Ala Gly Ile Leu Gln Ala Asp Lys Val Leu Thr Val Ser Pro Tyr Tyr
305             310             315             320

Ala Glu Glu Leu Ile Ser Gly Glu Ala Arg Gly Cys Glu Leu Asp Asn
                325             330             335

Ile Met Arg Leu Thr Gly Ile Thr Gly Ile Val Asn Gly Met Asp Val
            340             345             350

Ser Glu Trp Asp Pro Ala Lys Asp Lys Phe Leu Ala Ala Asn Tyr Asp
        355             360             365

Val Thr Thr Ala Leu Glu Gly Lys Ala Leu Asn Lys Glu Ala Leu Gln
    370             375             380

Ala Glu Val Gly Leu Pro Val Asp Arg Lys Val Pro Leu Val Ala Phe
385             390             395             400

Ile Gly Arg Leu Glu Glu Gln Lys Gly Pro Asp Val Met Ile Ala Ala
            405             410             415

Ile Pro Glu Ile Leu Lys Glu Glu Asp Val Gln Ile Val Leu Leu Gly
        420             425             430

Thr Gly Lys Lys Lys Phe Glu Arg Leu Leu Lys Ser Val Glu Glu Lys
        435             440             445

Phe Pro Ser Lys Val Arg Ala Val Val Arg Phe Asn Ala Pro Leu Ala
    450             455             460

His Gln Met Met Ala Gly Ala Asp Val Leu Ala Val Thr Ser Arg Phe
465             470             475             480
```

84

```
Glu Pro Cys Gly Leu Ile Gln Leu Gln Gly Met Arg Tyr Gly Thr Pro
            485             490             495

Cys Ala Cys Ala Ser Thr Gly Gly Leu Val Asp Thr Ile Met Glu Gly
            500             505             510

Lys Thr Gly Phe His Met Gly Arg Leu Ser Val Asp Cys Asn Val Val
            515             520             525

Glu Pro Ala Asp Val Lys Lys Val Val Thr Thr Leu Lys Arg Ala Val
        530             535             540

Lys Val Val Gly Thr Pro Ala Tyr His Glu Met Val Lys Asn Cys Met
545             550             555             560

Ile Gln Asp Leu Ser Trp Lys Gly Pro Ala Lys Asn Trp Glu Asp Val
            565             570             575

Leu Leu Glu Leu Gly Val Glu Gly Ser Glu Pro Gly Val Ile Gly Glu
            580             585             590

Glu Ile Ala Pro Leu Ala Met Glu Asn Val Ala Ala Pro
        595             600             605
```

<210>  11
<211>  1818
<212>  DNA
<213>  Zea mays

<220>
<221>  CDS
<222>  (1)..(1815)

<400>  11

```
atg gcg gct ctg gcc acg tcg cag ctc gtc gca acg cgc gcc ggc ctg      48
Met Ala Ala Leu Ala Thr Ser Gln Leu Val Ala Thr Arg Ala Gly Leu
1               5               10              15

ggc gtc ccg gac gcg tcc acg ttc cgc cgc ggc gcc gcg cag ggc ctg      96
Gly Val Pro Asp Ala Ser Thr Phe Arg Arg Gly Ala Ala Gln Gly Leu
```

<pre>
                    20                      25                      30

agg ggg gcc cgg gcg tcg gcg gcg gcg gac acg ctc agc atg cgg acc    144
Arg Gly Ala Arg Ala Ser Ala Ala Ala Asp Thr Leu Ser Met Arg Thr
        35                      40                      45

agc gcg cgc gcg gcg ccc agg cac cag cag cag gcg cgc cgc ggg ggc    192
Ser Ala Arg Ala Ala Pro Arg His Gln Gln Gln Ala Arg Arg Gly Gly
        50                      55                      60

agg ttc ccg tcg ctc gtc gtg tgc gcc agc gcc ggc atg aac gtc gtc    240
Arg Phe Pro Ser Leu Val Val Cys Ala Ser Ala Gly Met Asn Val Val
65                      70                      75                  80

ttc gtc ggc gcc gag atg gcg ccg tgg agc aag acc ggc ggc ctc ggc    288
Phe Val Gly Ala Glu Met Ala Pro Trp Ser Lys Thr Gly Gly Leu Gly
                    85                      90                      95

gac gtc ctc ggc ggc ctg ccg ccg gcc atg gcc gcg aac ggg cac cgt    336
Asp Val Leu Gly Gly Leu Pro Pro Ala Met Ala Ala Asn Gly His Arg
                100                     105                     110

gtc atg gtc gtc tct ccc cgc tac gac cag tac aag gac gcc tgg gac    384
Val Met Val Val Ser Pro Arg Tyr Asp Gln Tyr Lys Asp Ala Trp Asp
                115                     120                     125

acc agc gtc gtg tcc gag atc aag atg gga gac ggg tac gag acg gtc    432
Thr Ser Val Val Ser Glu Ile Lys Met Gly Asp Gly Tyr Glu Thr Val
                130                     135                     140

agg ttc ttc cac tgc tac aag cgc gga gtg gac cgc gtg ttc gtt gac    480
Arg Phe Phe His Cys Tyr Lys Arg Gly Val Asp Arg Val Phe Val Asp
145                     150                     155                 160

cac cca ctg ttc ctg gag agg gtt tgg gga aag acc gag gag aag atc    528
His Pro Leu Phe Leu Glu Arg Val Trp Gly Lys Thr Glu Glu Lys Ile
                165                     170                     175

tac ggg cct gtc gct gga acg gac tac agg gac aac cag ctg cgg ttc    576
Tyr Gly Pro Val Ala Gly Thr Asp Tyr Arg Asp Asn Gln Leu Arg Phe
                180                     185                     190

agc ctg cta tgc cag gca gca ctt gaa gct cca agg atc ctg agc ctc    624
Ser Leu Leu Cys Gln Ala Ala Leu Glu Ala Pro Arg Ile Leu Ser Leu
                195                     200                     205

aac aac aac cca tac ttc tcc gga cca tac ggg gag gac gtc gtg ttc    672
Asn Asn Asn Pro Tyr Phe Ser Gly Pro Tyr Gly Glu Asp Val Val Phe
                210                     215                     220
</pre>

```
gtc tgc aac gac tgg cac acc ggc cct ctc tcg tgc tac ctc aag agc        720
Val Cys Asn Asp Trp His Thr Gly Pro Leu Ser Cys Tyr Leu Lys Ser
225             230             235             240

aac tac cag tcc cac ggc atc tac agg gac gca aag acc gct ttc tgc        768
Asn Tyr Gln Ser His Gly Ile Tyr Arg Asp Ala Lys Thr Ala Phe Cys
            245             250             255

atc cac aac atc tcc tac cag ggc cgg ttc gcc ttc tcc gac tac ccg        816
Ile His Asn Ile Ser Tyr Gln Gly Arg Phe Ala Phe Ser Asp Tyr Pro
            260             265             270

gag ctg aac ctc ccg gag aga ttc aag tcg tcc ttc gat ttc atc gac        864
Glu Leu Asn Leu Pro Glu Arg Phe Lys Ser Ser Phe Asp Phe Ile Asp
            275             280             285

ggc tac gag aag ccc gtg gaa ggc cgg aag atc aac tgg atg aag gcc        912
Gly Tyr Glu Lys Pro Val Glu Gly Arg Lys Ile Asn Trp Met Lys Ala
            290             295             300

ggg atc ctc gag gcc gac agg gtc ctc acc gtc agc ccc tac tac gcc        960
Gly Ile Leu Glu Ala Asp Arg Val Leu Thr Val Ser Pro Tyr Tyr Ala
305             310             315             320

gag gag ctc atc tcc ggc atc gcc agg ggc tgc gag ctc gac aac atc        1008
Glu Glu Leu Ile Ser Gly Ile Ala Arg Gly Cys Glu Leu Asp Asn Ile
            325             330             335

atg cgc ctc acc ggc atc acc ggc atc gtc aac ggc atg gac gtc agc        1056
Met Arg Leu Thr Gly Ile Thr Gly Ile Val Asn Gly Met Asp Val Ser
            340             345             350

gag tgg gac ccc agc agg gac aag tac atc gcc gtg aag tac gac gtg        1104
Glu Trp Asp Pro Ser Arg Asp Lys Tyr Ile Ala Val Lys Tyr Asp Val
            355             360             365

tcg acg gcc gtg gag gcc aag gcg ctg aac aag gag gcg ctg cag gcg        1152
Ser Thr Ala Val Glu Ala Lys Ala Leu Asn Lys Glu Ala Leu Gln Ala
            370             375             380

gag gtc ggg ctc ccg gtg gac cgg aac atc ccg ctg gtg gcg ttc atc        1200
Glu Val Gly Leu Pro Val Asp Arg Asn Ile Pro Leu Val Ala Phe Ile
385             390             395             400

ggc agg ctg gaa gag cag aag ggc ccc gac gtc atg gcg gcc gcc atc        1248
Gly Arg Leu Glu Glu Gln Lys Gly Pro Asp Val Met Ala Ala Ala Ile
            405             410             415
```

```
ccg cag ctc atg gag atg gtg gag gac gtg cag atc gtt ctg ctg ggc        1296
Pro Gln Leu Met Glu Met Val Glu Asp Val Gln Ile Val Leu Leu Gly
            420             425             430

acg ggc aag aag aag ttc gag cgc atg ctc atg agc gcc gag gag aag        1344
Thr Gly Lys Lys Lys Phe Glu Arg Met Leu Met Ser Ala Glu Glu Lys
            435             440             445

ttc cca ggc aag gtg cgc gcc gtg gtc aag ttc aac gcg gcg ctg gcg        1392
Phe Pro Gly Lys Val Arg Ala Val Val Lys Phe Asn Ala Ala Leu Ala
        450             455             460

cac cac atc atg gcc ggc gcc gac gtg ctc gcc gtc acc agc cgc ttc        1440
His His Ile Met Ala Gly Ala Asp Val Leu Ala Val Thr Ser Arg Phe
465             470             475             480

gag ccc tgc ggc ctc atc cag ctg cag ggg atg cga tac gga acg ccc        1488
Glu Pro Cys Gly Leu Ile Gln Leu Gln Gly Met Arg Tyr Gly Thr Pro
            485             490             495

tgc gcc tgc gcg tcc acc ggt gga ctc gtc gac acc atc atc gaa ggc        1536
Cys Ala Cys Ala Ser Thr Gly Gly Leu Val Asp Thr Ile Ile Glu Gly
            500             505             510

aag acc ggg ttc cac atg ggc cgc ctc agc gtc gac tgt aac gtc gtg        1584
Lys Thr Gly Phe His Met Gly Arg Leu Ser Val Asp Cys Asn Val Val
            515             520             525

gag ccg gcg gac gtc aag aag gtg gcc acc aca ttg cag cgc gcc atc        1632
Glu Pro Ala Asp Val Lys Lys Val Ala Thr Thr Leu Gln Arg Ala Ile
        530             535             540

aag gtg gtc ggc acg ccg gcg tac gag gag atg gtg agg aac tgc atg        1680
Lys Val Val Gly Thr Pro Ala Tyr Glu Glu Met Val Arg Asn Cys Met
545             550             555             560

atc cag gat ctc tcc tgg aag ggc cct gcc aag aac tgg gag aac gtg        1728
Ile Gln Asp Leu Ser Trp Lys Gly Pro Ala Lys Asn Trp Glu Asn Val
            565             570             575

ctg ctc agc ctc ggg gtc gcc ggc ggc gag cca ggg gtc gaa ggc gag        1776
Leu Leu Ser Leu Gly Val Ala Gly Gly Glu Pro Gly Val Glu Gly Glu
            580             585             590

gag atc gcg ccg ctc gcc aag gag aac gtg gcc gcg ccc tga            1818
Glu Ile Ala Pro Leu Ala Lys Glu Asn Val Ala Ala Pro
        595             600             605
```

```
<210>   12
<211>   605
<212>   PRT
<213>   Zea mays

<400>   12

Met Ala Ala Leu Ala Thr Ser Gln Leu Val Ala Thr Arg Ala Gly Leu
1               5                   10                  15


Gly Val Pro Asp Ala Ser Thr Phe Arg Arg Gly Ala Ala Gln Gly Leu
            20                  25                  30


Arg Gly Ala Arg Ala Ser Ala Ala Ala Asp Thr Leu Ser Met Arg Thr
        35                  40                  45


Ser Ala Arg Ala Ala Pro Arg His Gln Gln Gln Ala Arg Arg Gly Gly
    50                  55                  60


Arg Phe Pro Ser Leu Val Val Cys Ala Ser Ala Gly Met Asn Val Val
65                  70                  75                  80


Phe Val Gly Ala Glu Met Ala Pro Trp Ser Lys Thr Gly Gly Leu Gly
            85                  90                  95


Asp Val Leu Gly Gly Leu Pro Pro Ala Met Ala Ala Asn Gly His Arg
            100                 105                 110


Val Met Val Val Ser Pro Arg Tyr Asp Gln Tyr Lys Asp Ala Trp Asp
        115                 120                 125


Thr Ser Val Val Ser Glu Ile Lys Met Gly Asp Gly Tyr Glu Thr Val
        130                 135                 140


Arg Phe Phe His Cys Tyr Lys Arg Gly Val Asp Arg Val Phe Val Asp
145                 150                 155                 160


His Pro Leu Phe Leu Glu Arg Val Trp Gly Lys Thr Glu Glu Lys Ile
                165                 170                 175
```

Tyr Gly Pro Val Ala Gly Thr Asp Tyr Arg Asp Asn Gln Leu Arg Phe
            180             185             190

Ser Leu Leu Cys Gln Ala Ala Leu Glu Ala Pro Arg Ile Leu Ser Leu
            195             200             205

Asn Asn Asn Pro Tyr Phe Ser Gly Pro Tyr Gly Glu Asp Val Val Phe
            210             215             220

Val Cys Asn Asp Trp His Thr Gly Pro Leu Ser Cys Tyr Leu Lys Ser
225             230             235             240

Asn Tyr Gln Ser His Gly Ile Tyr Arg Asp Ala Lys Thr Ala Phe Cys
            245             250             255

Ile His Asn Ile Ser Tyr Gln Gly Arg Phe Ala Phe Ser Asp Tyr Pro
            260             265             270

Glu Leu Asn Leu Pro Glu Arg Phe Lys Ser Ser Phe Asp Phe Ile Asp
            275             280             285

Gly Tyr Glu Lys Pro Val Glu Gly Arg Lys Ile Asn Trp Met Lys Ala
            290             295             300

Gly Ile Leu Glu Ala Asp Arg Val Leu Thr Val Ser Pro Tyr Tyr Ala
305             310             315             320

Glu Glu Leu Ile Ser Gly Ile Ala Arg Gly Cys Glu Leu Asp Asn Ile
            325             330             335

Met Arg Leu Thr Gly Ile Thr Gly Ile Val Asn Gly Met Asp Val Ser
            340             345             350

Glu Trp Asp Pro Ser Arg Asp Lys Tyr Ile Ala Val Lys Tyr Asp Val
            355             360             365

90

Ser Thr Ala Val Glu Ala Lys Ala Leu Asn Lys Glu Ala Leu Gln Ala
    370                 375                 380

Glu Val Gly Leu Pro Val Asp Arg Asn Ile Pro Leu Val Ala Phe Ile
385                 390                 395                 400

Gly Arg Leu Glu Glu Gln Lys Gly Pro Asp Val Met Ala Ala Ala Ile
                405                 410                 415

Pro Gln Leu Met Glu Met Val Glu Asp Val Gln Ile Val Leu Leu Gly
            420                 425                 430

Thr Gly Lys Lys Lys Phe Glu Arg Met Leu Met Ser Ala Glu Glu Lys
        435                 440                 445

Phe Pro Gly Lys Val Arg Ala Val Val Lys Phe Asn Ala Ala Leu Ala
    450                 455                 460

His His Ile Met Ala Gly Ala Asp Val Leu Ala Val Thr Ser Arg Phe
465                 470                 475                 480

Glu Pro Cys Gly Leu Ile Gln Leu Gln Gly Met Arg Tyr Gly Thr Pro
                485                 490                 495

Cys Ala Cys Ala Ser Thr Gly Gly Leu Val Asp Thr Ile Ile Glu Gly
                500                 505                 510

Lys Thr Gly Phe His Met Gly Arg Leu Ser Val Asp Cys Asn Val Val
        515                 520                 525

Glu Pro Ala Asp Val Lys Lys Val Ala Thr Thr Leu Gln Arg Ala Ile
    530                 535                 540

Lys Val Val Gly Thr Pro Ala Tyr Glu Glu Met Val Arg Asn Cys Met
545                 550                 555                 560

91

```
Ile Gln Asp Leu Ser Trp Lys Gly Pro Ala Lys Asn Trp Glu Asn Val
                565                 570                 575


Leu Leu Ser Leu Gly Val Ala Gly Gly Glu Pro Gly Val Glu Gly Glu
            580                 585                 590


Glu Ile Ala Pro Leu Ala Lys Glu Asn Val Ala Ala Pro
        595                 600                 605
```